(19) Europäisches Patentamt
European Patent Office
Office européen des brevets

(11) Veröffentlichungsnummer : **0 027 258**
**B1**

(12) **EUROPÄISCHE PATENTSCHRIFT**

(45) Veröffentlichungstag der Patentschrift : **15.05.85**

(51) Int. Cl.[4] : **C 07 H 15/04**, A 61 K 31/70

(21) Anmeldenummer : **80106134.2**

(22) Anmeldetag : **09.10.80**

(54) **Phosphorylmuramylpeptide, Verfahren zu deren Herstellung und deren Verwendung in Arzneimitteln.**

(30) Priorität : **12.10.79 CH 9219/79**

(43) Veröffentlichungstag der Anmeldung : **22.04.81 Patentblatt 81/16**

(45) Bekanntmachung des Hinweises auf die Patenterteilung : **15.05.85 Patentblatt 85/20**

(84) Benannte Vertragsstaaten : **AT BE CH DE FR GB IT LI LU NL SE**

(56) Entgegenhaltungen :
**EP-A- 0 025 495**
**FR-A- 2 361 902**

(73) Patentinhaber : **CIBA-GEIGY AG**
**Postfach**
**CH-4002 Basel (CH)**

(72) Erfinder : **Baschang, Gerhard, Dr.**
**Bückenweg 7**
**CH-4126 Bettingen (CH)**
Erfinder : **Tarcsay, Lajos, Dr.**
**Muttenzerstrasse 25**
**D-7889 Grenzach-Wyhlen (DE)**
Erfinder : **Hartmann, Albert, Dr.**
**Steingasse 21A**
**D-7889 Grenzach (DE)**
Erfinder : **Stanek, Jaroslav, Dr.**
**Florastrasse 6**
**CH-4127 Birsfelden (CH)**

(74) Vertreter : **Zumstein, Fritz sen., Dr. et al**
**Bräuhausstrasse 4**
**D-8000 München 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 027 258 B1

**Beschreibung**

Gegenstand der Erfindung sind neue Phosphorylmuramylpeptide, ferner Verfahren zu deren Herstellung, sowie pharmazeutische Präparate, die diese Phosphorylmuramylpeptide enthalten, wie auch deren Verwendung zur Stimulierung der Immunität.

Die Erfindung betrifft insbesondere Verbindungen der Formel

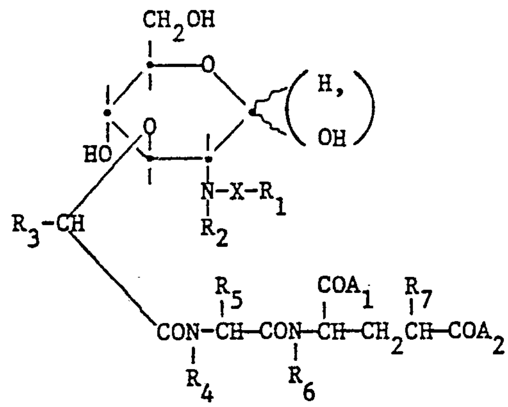

(I)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkylniederalkyl, gegebenenfalls substituiertes Aryl oder Aralkyl, stickstoffhaltiges Heterocyclyl oder Heterocyclylnierderalkyl, oder $R_4$ und $R_5$ zusammen auch Alkylen mit 3-4 Kohlenstoffatomen, $R_7$ Wasserstoff oder gegebenenfalls verestertes oder amidiertes Carboxyl und einer der Reste $A_1$ und $A_2$ einen Rest der Formel

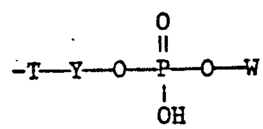

(II)

bedeutet, worin T für NH oder O steht, Y eine gegebenenfalls substituierte Alkylengruppe, die auch durch ein oder zwei Oxycarbonyl und/oder Iminocarbonyl unterbrochen sein kann und W einen aliphatischen Rest oder einen Cycloalkyl- oder Cycloalkenylrest mit jeweils mehr als 6 Kohlenstoffatomen darstellen, und der andere der Reste $A_1$ und $A_2$ freies oder veräthertes Hydroxy, Amino, Niederalkylamino oder im Niederalkylrest gegebenenfalls substituiertes Aminocarbonylniederalkyl-amino ist, und ihre Salze mit Ausnahme der in der europäischen Patentanmeldung mit der Veröffentlichungsnummer 0 025 495 offenbarten Verbindungen, nämlich von Muramylpeptiden der Formel

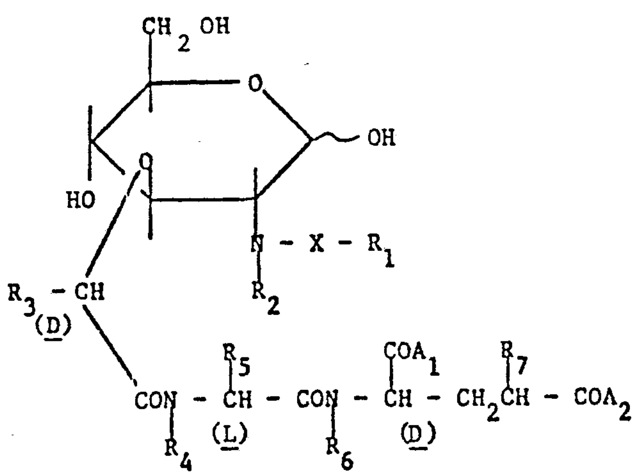

(Ia)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptioniederalkyl, gegebenenfalls substituiertes Aminoniederalkyl, Cycloalkyl, Cycloalkyl-niederalkyl, gegebenenfalls

substituiertes Aryl oder Aralkyl, stickstoffhaltiges Heterocyclyl oder Heterocyclylniederalkyl, oder $R_4$ und $R_5$ zusammen auch Alkylen mit 3-4 Kohlenstoffatomen, $R_2$ Wasserstoff oder gegebenenfalls verestertes oder amidiertes Carbonyl und einer der reste $A_1$ und $A_2$ einen Rest der Formel

$$\begin{array}{ccccccccccc} & & & & & O & & & & W_a \\ & & & & & \| & & & & | \\ -T & - & Y & - & O & - P & - & O & - & CH \\ & & & & & | & & & & | \\ & & & & & OH & & & & Z_a \end{array} \qquad \text{(IIa)}$$

bedeuten, worin T für HN oder O steht, Y eine gegebenenfalls substituierte Alkylengruppe, die auch durch ein oder zwei Oxycarbonyl und/oder Iminocarbonyl unterbrochen sein kann, $W_a$ Wasserstoff und $Z_a$ eine 1,2-Dihydroxyäthyl- oder 2-Hydroxyäthylgruppe, in denen mindestens eine Hydroxygruppe mit einer gegebenenfalls ungesättigten aliphatischen Carbonsäure mit 12-90 C-Atomen verestert oder mit einem gegebenenfalls ungesättigten aliphatischen Alkohol mit 10-22 C-Atomen veräthert ist, oder $W_a$ und $Z_a$ je eine mit einer gegebenenfalls ungesättigten aliuphatischen Carbonsäure mit 12-90 C-Atomen veresterte oder mit einem gegebenenfalls ungesättigten aliphatischen Alkohol mit 10-22 C-Atomen verätherte Hydroxymethylgruppe darstellen, und der andere der Reste $A_1$ und $A_2$ freies oder veräthertes Hydroxy, Amino, Niederalkylamino oder Carboxamidoniederalkyl-amino ist, und ihrer Salze, wobei das Präfix « Nieder » Reste bis und mit 7 C-Atomen bezeichnet, sowie von Muramylpeptiden der oben dargestellten Formel Ia, worin X Carbonyl oder Carbonyloxy, $R_1$ Niederalkyl mit 1-3 Kohlenstoffatomen oder Phenyl, $R_2$, $R_4$ und $R_6$ Wasserstoff, $R^3$ Wasserstoff oder Niederalkyl mit 1-3 Kohlenstoffatomen, $R^5$ Wasserstoff oder Niederalkyl, $R^7$ Wasserstoff, $A_1$ Amino, Niederalkylamino, Hydroxy oder Niederalkoxy und $A_2$ einen Rest der oben dargestellen Formel IIa, worin T für NH oder O steht, Y Niederalkylen mit 2-3 Kohlenstoffatomen oder einen Rest der Formel

$$—CH_2—CO—NH—CH_2—CH_2—$$

bedeuten, $W_a$ für Wasserstoff und $Z_a$ für eine 1,2-Dihydroxyäthyl- oder 2-Hydroxyäthyl-Gruppe stehen, in der eine oder zwei Hydroxygruppen mit gleichen oder verschiedenen gegebenenfalls ein- oder zweifach ungesättigten Alkancarbonsäuren mit 16-22 Kohlenstoffatomen verestert oder mit einem gegebenenfalls ein- oder zweifach ungesättigten Alkanol mit 12-18 Kohlenstoffatomen veräthert sind oder worin zwei vicinale Hydroxygruppen unter Bildung eines Dioxolanringes formal mit einem Bisniederalkylketon ketalisiert oder mit einem unsubstituierten aliphatischen Aldehyd mit bis zu 20 C-Atomen acetalisiert sind, oder worin $W_a$ und $Z_a$ je eine Hydroxymethylgruppe darstellen, die mit einer gegebenenfalls ein- oder zweifach ungesättigten Alkancarbonsäure mit 16-22 Kohlenstoffatomen verestert oder mit einem gegebenenfalls ein- oder zweifach ungesättigten Alkanol mit 12-18 Kohlenstoffatomen veräthert ist, und ihrer Salze, wobei das Präfix « Nieder » Reste bis und mit 7 C-Atomen bezeichnet, sowie von N-Acetyl-muramyl-L-alanyl-D-isoglutaminyl-L-alanin-2-(1',2'-hexadecyliden-sn-glycero-3'-hydroxyphosphoryloxy)-äthylamid und N-Acetyl-desmethylmuramyl-L-alanyl-D-isoglutaminyl-L-alanin-2-(1',2'-hexadecyliden-sn-glycero-3'-hydroxyphosphoryloxy)-äthylamid und deren Salzen.

Alkyl ist geradkettiges oder verzweigtes, in beliebiger Stellung gebundenes Alkyl mit bis zu 18 Kohlenstoffatomen, in erster Linie jedoch Niederalkyl.

Als Substituenten der gegebenenfalls substituierten Alkylgruppen kommen in erster Linie freie oder funktionell abgewandelte Hydroxy- oder Mercaptogruppen, wie verätherte oder veresterte Hydroxy- oder Mercaptogruppen, z. B. Niederalkoxy oder Niederalkylmercaptogruppen, oder Halogenatome oder freie oder funktionell abgewandelte Carboxyl-, wie Niederalkoxycarbonyl- oder Carbamoylgruppen in Frage. Dabei kann der substituierte Alkylrest, wie Niederalkylrest, einen, zwei oder mehrere gleiche oder verschiedene Substituenten, insbesondere freie Hydroxylgruppen oder Halogenatome tragen.

Der aliphatische Rest W ist ein Alkyl- oder Alkenyl-Rest, der bis zu 30 Kohlenstoffatome enthält und als Substituenten vorzugsweise freie oder funktionell abgewandelte Hydroxygruppen, wie verätherte oder veresterte Hydroxygruppen, z. B. Niederalkoxy- oder Niederalkanoyloxygruppen, Halogenatome oder freie oder acylierte Aminogruppen, z. B. Alkanoyl-, wie Niederalkanoylaminogruppen, oder Ketogruppen tragen kann. Diese Substituenten sitzen in erster Linie in 2-Stellung, d. h. in β-Stellung zur Phosphoryloxygruppe. W kann auch für einen Cycloalkyl- oder einen Cycloalkenylrest, z. B. Cholesteryl, mit bis zu 30 Kohlenstoffatomen stehen.

Arylreste sind insbesondere monocyclische, sowie bicyclische Arylreste, in erster Linie Phenyl, aber auch Naphthyl. Sie können gegebenenfalls, z. B. durch Niederalkylgruppen, freies, verestertes oder veräthertes Hydroxy, z. B. Niederalkoxy oder Niederalkylendioxy oder Halogenatome, und/oder Trifluoromethylgruppen mono-, di- oder polysubstituiert sein.

Aralkyl ist insbesondere Arylniederalkyl, worin Aryl die oben gegebenen Bedeutung hat. In erster Linie steht Arylniederalkyl für Benzyl oder Phenyläthyl, worin der Phenylkern mono-, di- oder polysubstituiert sein kann.

Gegebenenfalls substituierte Aralkylreste sind insbesondere solche Reste, die im aromatischen Kern

gegebenenfalls, z. B. durch Niederalkyl, freie, verätherte oder veresterte Hydroxy- oder Mercaptogruppen, z. B. Niederalkoxy oder Niederalkylendioxy, sowie Niederalkylmercapto- oder Trifluormethylgruppen und/oder Halogenatome, mono-, di- oder polysubstituiert sind.

Cycloalkyl, ausser als Substituent W, ist insbesondere Cycloalkyl mit 5 bis 6 Kohlenstoffatomen, wie Cyclopentyl oder Cyclohexyl, und Cycloalkylniederalkyl insbesondere ein solcher Rest, worin der Cycloalkylrest 5 bis 6 Kohlenstoffatome besitzt und der Niederalkylrest in erster Linie Methyl oder Aethyl darstellt.

Stickstoffhaltiges Heterocyclyl ist insbesondere der Rest einer 5- oder 6-gliedrigen, ein oder zwei Stickstoffatome im Ring enthaltenden heterocyclischen Verbindung. Er kann ungesättigt oder auch gesättigt sein und z. B. einen ankondensierten Phenylrest enthalten. Als solche seien z. B. die Pyrrolyl-, Indolyl-, Pyridyl- oder Imidazolylreste genannt.

Im stickstoffhaltigen Heterocyclylniederalkyl hat der Heterocyclylrest die oben genannte Bedeutung und der Niederalkylrest ist in erster Linie Methyl oder Aethyl.

Der Alkylenrest, der von den Resten $R_4$ und $R_5$ gebildet sein kann, ist vorzugsweise unsubstituiert und in erster Linie der Trimethylenrest.

Eine gegebenenfalls veresterte oder amidierte Carboxylgruppe ist in erster Linie die Carboxylgruppe selbst oder eine mit einem Niederalkanol veresterte Carboxylgruppe oder auch eine Carbamoylgruppe, die am Stickstoffatom unsubstituiert, oder mit gegebenenfalls substituiertem Alkyl, insbesondere Niederalkyl, Aryl, in erster Linie Phenyl, oder Aralkyl, wie Benzyl, mono- oder disubstituiert ist. Die Carbamoylgruppe kann aber auch einen Alkylen-, wie den Tetra- oder Pentamethylenrest, tragen.

Als gegebenenfalls funktionell abgewandelte Hydroxy- oder Mercaptogruppen sind insbesondere verätherte oder veresterte Hydroxy- oder Mercaptogruppen zu nennen, wie Niederalkoxy, Niederacyloxy, z. B. Niederalkanoyloxy, oder Halogenatome, Niederalkylmercapto oder Niederacylmercapto, z. B. Niederalkanoylmercapto.

Als gegebenenfalls substituierte Aminoniederalkyl sind insbesondere Mono- oder Diniederalkylaminoniederalkyl, wie Methylamino-, Aethylamino-, Dimethylamino-, Diäthylamino, oder acyliertes Aminoniederalkyl, wie Alkanoylaminoniederalkyl, z. B. Niederalkanoylaminoniederalkyl, zu nennen.

Eine am Niederalkylrest gegebenenfalls substituierte Aminocarbonylniederalkylaminogruppe ist in erster Linie eine Niederalkylaminogruppe, die den Aminocarbonylrest in 1-Stellung trägt, z. B. Aminocarbonylmethylamino, 1-Aminocarbonyl-äthylamino, 1-Aminocarbonylisobutylamino oder 1-Aminocarbonyl-3-methyl-butylamino, oder auch eine 1-Aminocarbonyl-niederalkylaminogruppe, deren Niederalkylgruppe Hydroxy, Carboxy oder Aminogruppen trägt, z. B. 1-Aminocarbonyl-2-hydroxy-äthylamino, 1-Aminocarbonyl-2-hydroxy-propylamino, 1,2-bis-Aminocarbonyl-äthylamino oder 1-Aminocarbonyl-5-amino-1-pentylamino.

Der Alkylenrest Y ist insbesondere ein durch eine oder zwei Oxycarbonyl- oder N-$R_8$-Carbonyliminogruppen unterbrochener Niederalkylenrest und stellt dann insbesondere einen Rest einer der Formeln

$$Y_1\text{-COO-}Y_2 \quad \text{(IIIa)}$$

$$Y_1\text{-OOC-}Y_2 \quad \text{(IIIb)}$$

$$Y_1\text{-}\underset{\displaystyle R_8}{\underset{|}{\text{CON}}}\text{-}Y_2 \quad \text{(IIIc)}$$

bzw.

$$Y_1\text{-}\underset{\displaystyle R_8}{\underset{|}{\text{N}}}\text{-OC-}Y_2 \quad \text{(IIId)}$$

dar, worin einer der Rester $Y_1$ und $Y_2$ ein gegebenenfalls substituierter Niederalkylenrest und der andere ein gegebenenfalls substituierter Niederalkylenrest ist, der auch durch Oxycarbonyl oder N-$R_8$-Carbonylimino unterbrochen sein kann, und worin $Y_1$ und $Y_2$ zusammen mehr als zwei Kohlenstoffatome aufweisen und $R_8$ Wasserstoff oder Niederalkyl ist. Als Substituenten der Reste $Y_1$ und $Y_2$ sollen insbesondere freies oder funktionell abgewandeltes Hydroxy oder Hydroxyniederalkyl, freies oder funktionell abgfewandeltes Mercapto oder Mercaptoniederalkyl, freies oder mono- oder di-niederalkyliertes oder acyliertes Aminoniederalkyl, Aminocarbonyl, Alkyl, Cycloalkyl mit 5 bis 6 Kohlenstoffatomen, Aryl der Aralkyl genannt werden, wobei die Allgemeinbegriffe die oben angegebene Bedeutung haben können. Der Alkylenrest Y kann aber auch ein Niederalkylenrest sein, vorzugsweise mit 2 oder 3 Kohlenstoffatomen.

Die im Zusammenhang mit der vorliegenden Beschreibung und den Patentansprüchen mit « Nieder » bezeichneten Reste und Verbindungen enthalten bis und mit 7 und in erster Linie bis und mit 4 Kohlenstoffatome.

Vorstehend wie nachfolgend können die Allgemeinbegriffe folgende Bedeutung haben :

Niederalkyl ist z. B. n-Propyl, n-Butyl, Isobutyl, sek.-Butyl oder tert.-Butyl, ferner n-Pentyl, n-Hexyl, Isohexyl oder n-Heptyl und in erster Linie Methyl oder Aethyl. In Aryl-, Cycloalkyl- oder Heterocyclylniederalkyl ist der Niederalkylrest insbesondere Methyl oder Aethyl, wobei der Aryl-, Cycloalkyl- oder Heterocyclylrest die obgenannte Bedeutung besitzt.

Niederalkoxy ist z. B. n-Propoxy, n-Butoxy, Isobutoxy, sek.-Butoxy oder tert.-Butoxy und in erster Linie Methoxy ode Aethoxy.

Niederalkylmercapto ist z. B. n-Propyl-, n-Butyl, Isobutyl-, sek.-Butyl- oder tert.-Butylmercapto und in erster Linie Methylmercapto oder Aethylmercapto.

Niederalkylendioxy ist insbesondere Methylendioxy, Aethylen- oder Propylendioxy.

Halogen steht für Fluor oder Brom, vorzugsweise jedoch für Chlor.

Niederalkanoyl ist insbesondere Propionyl oder Butyryl, in erster Linie jedoch Acetyl.

Die Verbindungen der Formel I können in Form von Isomerengemischen oder reinen Isomeren vorliegen. Vorzugsweise liegt der mit dem Sauerstoffatom verknüpfte Rest der Formel $—CH(R_3)—C(=O)—$, falls $R_3$ für Niederalkyl steht, in optisch aktiver Form vor und hat insbesondere die D-Form, während der Rest der Aminosäure der Formel $—N(R_4)—CH(R_5)—C(=O)—$ falls $R_5$ von Wasserstoff verschieden ist, ebenfalls vorzugsweise in optisch aktiver, in erster Linie in der L-Form vorliegt, und der terminale α-Amino-glutarsäurerest vorzugsweise in optisch aktiver, insbesondere in der D-Form vorliegt. Ferner kann die gegebenenfalls substituierte 1-Hydroxygruppe die α- oder die β-Konfiguration haben ; die neuen Verbindungen der Formel I können jedoch auch als Gemisch der 1α- und 1β-Isomeren vorliegen.

In den Verbindungen der Formel I kann das über ein Sauertoffatom an Phosphor gebundene Proton leicht mit Basen abgespalten werden. Ueblicherweise liegen die Verbindungen der Formel I in Form eines Gemisches de freien Verbindungen und ihrer Salze vor. So liegen die in den Beispielen beschriebenen Muramylpeptide der Formel I zu etwa 40 bis 55 % als Salze vor. Diese Salze gehören zum Gegenstand der Erfindung.

Die Erfindung betrifft generell auch die Salze de Verbindungen der Formel I mit irgendwelchen anderen salzbildenden Gruppen. Als solche salzbildenden Gruppen kommen z. B. Carboxylgruppen in Frage, die z. B. durch die Reste $COA_1$, $COA_2$ oder $R_7$ repräsentiert werden können, oder Aminogruppen im Rest $R_5$. Insbesondere betrifft die Erfindung pharmazeutisch verwendbare nicht toxische Salze der Verbindungen der Formel I. Als Gegenionen von Carboxylatonionen sind in erster Linie Metall- oder Ammoniumionen zu nennen, wie Alkalimetall- und Erdalkalimetall-, z. B. Natrium-, Kalium-, Magnesium- oder Calciumionen, sowie Ammoniumionen aus Ammoniak oder geeigneten organischen Aminen, wie Niederalkylaminen, z. B. Triäthylamin. Die Verbindungen der Formel I mit basischen Gruppen, z. B. Aminogruppen, können Säureadditionssalze bilden. Die Verbindungen können bevorzugterweise auch in Form von inneren Salzen, d. h. als Zwitterionen, vorleigen. z. B. kann das über ein Sauerstoffatom an Phosphor gebundene Proton eine Aminogruppe im Rest $R_5$ protonieren. Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt werden.

In der FR-A-2 361 902 sind Muramylpeptide beschrieben, die sich in struktureller Hinsicht von den Verbindungen der vorliegenden Anmeldung durch den fehlenden Phosphorylsubstituenten der Formel II unterscheiden, und die als Ausgangsstoffe zu Herstellung der Verbindungen der vorliegenden Anmeldung verwendet werden können.

In der EP-A-0025 495, die zum Stand de Technick gemäss Art. 54(3) EPU gehört, sind Muramylpeptide beschrieben, die anstelle des obengenannten Restes der Formel II den obengenannten Rest der Formel IIa enthalten, worin der Rest W durch den Rest

$W_a$
|
CH
|
$Z_a$

ersetzt ist, und die durch Disclaimer vom Gegenstand der vorliegenden Anmeldung ausgenommen sind.

Die neuen Phosphorylmuramylpeptide der vorliegenden Erfindung weisen eine Reihe wertvoller pharmakologischer Eigenschaften, insbesondere eine ausgeprägte immunpotenzierende Wirkung auf.

So wird durch diese Verbindung in vivo die Antikörperbildungsfähigkeit von Mäusen erheblich gesteigert :

NMRI Mäusen werden durch intraperitoneale Injektion von 10 μg präzipitatfreiem Bovin-Serum-Albumin (BSA) am Tag 0 immunisiert. 9, 15 und 29 Tage später werden Serumproben entnommen und auf ihren Gehalt an anti-BSA-Antikörpern mit einer passiven Haemagglutinationstechnik untersucht. In der verwendeten Dosis ist lösliches BSA für die Empfägertiere subimmunogen, d. h. es vermag keine oder nur eine ganz geringfügige Produktion von Antikörpern auszulösen. Zusätzliche Behandlung der Mäuse mit gewissen immunpotenzierenden Stoffen vor oder nach der Antigenbage führt zi einem Anstieg der Antikörpertiter im Serum. Der Effekt der Behandlung wird durch den erreichten Scorewert, d. h. durch die Summe der $log_2$ Titerdifferenzen an den drei Blutungstagen ausgedrückt.

In diesem Test sind die Verbindungen der Formel (I) in der Lage, bei intraperitonealer oder subkutaner Applikation von 0,5-5 mg/kg Tier an fünf aufeinander folgenden Tagen nach Immunisierung mit BSA die Antikörperproduktion gegen BSA signifikant zu steigern. Sie sind diesbezüglich den herkömmlichen hydrophilen Muramylpeptiden hoch überlegen.

Auch Manifestationen der zellvermittelten Immunität können durch die genannten Verbindungen in vivo potenziert werden :

Während Sensibilisierung von Meerschweinchen mit BSA in inkomplettem Freund'schem Adjuvans nur zu humoraler Antikörperbildung führt, induziert die Beimischung der erfindungsgemässen Phosphorylmuramylpeptide in einem Dosisbereich von 5 bis 50 µg zur Antigen-Oelemulsion Spättyp-Ueberempfindlichkeit gegenüber BSA : 3 Wochen nach Immunisierung führt die intrakutane Injektion von BSA bei diesen Tieren zu einer lokalen Entzündungsreaktion mit Erythem und Verdickung der Haut, die innert 24 bis 48 Stunden ihr Maximum erreicht. Diese Spättyp-Reaktionen entsprechen quantitativ und qualitativ denjenigen, die üblicherweise durch Immunisierung mit BSA in komplettem Freund'schem Adjuvans (d. h. mit Zusatz von Mykobakterien) erhalten werden. Die $ED_{50}$-Werte (benötigte µg/Tier zur Induktion einer Differenz des Reaktionsvolumens (Erythemfläche x Hautdickenzunahme) bei behandelten und unbehandelten Tieren von 200 µl, 25 Stunden nach Auslösung) betragen 10-20 µg.

Besonders hervorzuheben ist auch die Fähigkeit solcher Phosphorylmuramylpeptide, durch Applikation zusammen mit BSA in Liposomen (Eilecithin : Cholesterin 4 : 1 ; 4mg/Tier) und ohne die toxische Mineralöl-Komponente in Meerschweinchen eine Spättypüberempfindlichkeit gegen BSA zu induzieren. Quantitativ und qualitativ sind diese Spättyp-Reaktionen ebenfalls indentisch mit denjenigen, die durch Immusierung mit BSA is komplettem Freund'schen Adjuvans erhalten werden. Die $ED_{50}$-Werte betragen 100-300 µg pro Tier.

Die neuen Verbindungen der Formel (I) zeigen im Vergleich zu hydrophilen Muramyldipeptiden weitere zusätzliche Qualitätsverbesserungen :

Balb/c Mäuse werden durch intraperitoneale Injektion von $2 \times 10^4$ P815 Mastocytomzellen am Tag 0 immunisiert. Am Tag 15 werden die Milzzellen der so immunisierten Tiere in vitro auf das Vorhandensein zytotoxischer, gegen P815 Mastocytom-Zellen gerichteter T-Lymphocyten untersucht. Dazu weden die P815 Zielzellen mit $^{51}Cr$ markiert und das Ausmass der zytotoxischen Reaktion durch Messen der Radioaktivität im Kulturüberstand ermittelt. In der verwendeten Dosis sind die P815 Mastozytomzellen für die Empfänger-Mäuse subimmunogen, d. h. sie induzieren keine oder nur ganz geringe Bildung zytotoxischer T-Zellen. Gleichzeitige intraperitoneale Applikation von 1 bis 50 µg der genannten Muramylpeptide der Formel I führen zu einer signifikanten Steigerung der Bildung zytotoxischer T-Zellen (Faktor 10 bis 30 gegenüber unbehandelten Mäusen).

Die immunpotenzierenden Eigenschaften der neuen Verfindungen der Formel (I) lassen sich auch im Falle der Induktion spezifischer Immuntoleranz gegen Transplantationsantigene durch Immunisierung mit adjuvierten Autoblasten bei der Maus darstellen :

In einer gemischten Lymphocytenkultur werden Milzlymphocyten des zukünftigen Transplantat-Empfängers (C57B1/6J Mäuse) mit bestrahlten Milzzellen des zukünftigen Transplantat-Spenders (CBA/J Mäuse) inkubiert. T-Lymphocyten mit spezifischen Rezeptoren für die Histokompatibilitätsantigene des Spenders proliferieren und werden zu Blasten ; diese können durch Sedimentation von den andern Zellen abgetrennt werden. Die spezifischen Blasten exprimieren die relevanten idiotypischen Spezifitäten der Membranrezeptoren und werden adjuviert mit komplettem Freund'chen Adjuvans (CFA) als Autoimmunogene zur Induktion spezifischer Toleranz gegen die betreffenden Transplantationsantigene in die prospektiven Transplantat-Empfänger (C 57 B1/6J) injiziert. Die Immunisierung erfolgt viermal in vierwöchigen Abständen mit autologen anti-CBA/J T Lymphoblasten. Adsorbate von T-Autoblasten mit den neuen Verbindungen der Formel (I) ($10^9$ Blasten werden in einer Lösung von 20 mg Substanz in 20 ml PBS suspendiert. Nach zweistündiger Inkubation werden die Zellen zentrifugiert und zweimal mit PBS gewaschen.) sind in der Lage, spezifische Immuntoleranz in Abwesenheit von CFA zu induzieren, wobei die Absorbate gleich wirksam sind wie Lymphoblasten in CFA.

Die neuen Verbindungen der Formel (I) sind ausserdem in der Lage, in Konzentrationen von 0,5 bis 100 µg/ml in Milzzellkulturen von normalen Mäusen die Bildung antikörperproduzierender Zellen zu induzieren (Vermehrung der 19S-plaquebildenden Zellen um einen Faktor 10 bis 30 über den Kontrollwert [in Abwesenheit der stimulierenden Substanzen]) : So werden in Anwesenheit der genannten Verbindungen z. B. spezifische Antikörper gegen Schaferythrocyten gebildet, ohne dass den Kulturen Schaferythrocyten zur Immunisierung zugesetzt werden. Andererseits vermögen die genannten Substanzen im selben Konzentrationsbereich auch die immunologische Reaktivität von T-zellverarmten Milzzellkulturen (von kongenital athymischen nu/nu Mäusen) gegenüber einem normalerweise thymusabhängigen Antigen (Schaferythrocyten) zu steigern (Faktor 10 bis 30 gegenüber unbehandelten Kontrollkulturen). Durch die genannten Verbindungen werden aber in vitro direkt oder indirekt nicht nur Proliferations- und Syntheseleistungen von B-Lymphocyten (d. h. von potentiell antikörperbildenden Zellen) induziert, sondern auch Effekte auf T-Lymphocyten (zu denen regulatorisch aktive Helfer- und Suppressorzellen sowie cytotoxische Effektorzellen gehören) vermittelt. So vermögen z. B. die erwähnten Verbindungen in einem Konzentrationsbereich von 1 bis 20 µg/ml die Reaktivität von cortisonresistenten Thymuszellen gegenüber allogenen bestrahlten Stimulatorlymphocyten erheblich (bis zu 10-fach) zu potenzieren.

Die oben erwähnten Wirkungen kommen wahrscheinlich indirekt dadurch zustande, dass solche Phosphorylmuramylpeptide Makrophagen aktivieren, die ihrerseits die Reaktivität von T- und B-Lymphocyten fördern. Tatsächlich kann man zeigen, dass die genannten Verbindungen bereits in geringen Konzentrationen (0,5-10 μg/ml) grosse Mengen « colony stimulating activity » (CSA) aus Maus-Makrophagen freisetzen (Induktion von bis zu 150 bis 200 Kolonien innert 7 Tagen aus $10^5$ Mäuse-Knochenmarkzellen, nach Zugabe von 20 % Ueberstand aus während 24 Stunden mit Substanz inkubierten Makrophagenkulturen, im Vergleich zu 0 bis 5 Kolonien bei Zugabe von Ueberständen unbehandelter Makrophagenkulturen). CSA ist ein biologischer Mediator, der für die Differenzierung von Knochenmark-Stammzellen zu Makrophagen und polymorphkernigen Leucocyten notwendig ist. Damit bewirken die genannten Verbindungen einen erhöhten Nachschub von Zellen, die für die unspezifische Resistenz und für die Induktion, Amplifikation und Expression spezifischer (lymphocytenvermittelter) Immunreaktionen von zentraler Bedeutung sind.

Die immunpotenzierende Wirkung der neuen verbindungen kann in vivo nachgewiesen werden : So führt die Injektion eines Phospholipid-Derivates eines Muramylpeptids gemäss der Erfindung innert 3 bis 9 Stunden zu einem hohen Anstieg der CSA-Konzentration im Serum (bis zu 120 Kolonien pro $10^5$ Mäuseknochenmarkzellen nach Zugabe von Chloroform extrahiertem Serum [5 % Endkonzentration] im Vergleich zu 0 bis 5 Kolonien bei unbehandelten Tieren). Dementsprechend wird durch Verabreichung derselben Verbindungen in vivo die Antikörperbildungsfähigkeit von Mäusen erheblich potenziert.

Die immunpotenzierenden Eigenschaften der neuen Verbindungen der Formel I lassen sich auch in Tumormodellen demonstrieren, so z. B. beim Ehrlich Ascites Tumor in der Maus.

Eine intraperitoneale Injektion von $10^6$ syngenen Ehrlich Ascites Tumorzellen führt bei Balb/c Mäusen durchschnittlich in 18 Tagen zum Absterben der Tiere. Injiziert man den Mäusen intraperitoneal $10^7$ (Gruppe 1), $10^6$ (Gruppen 2) und $10^5$ (Gruppen 3) Ascites Tumorzellen, die man in vitro mit den neuen Verbindungen der Formel I beladen hat ($10^9$ Ascites Tumorzellen werden in einer Lösung von 40 mg der Prüfsubstanz in 20 ml phosphat-gepufferter physiologischer Kochsalzlösung (PBS) suspendiert und nach zweistündiger Inkubation bei 37 °C werden die Zellen zentrifugiert und zweimal mit PBS gewaschen ; die Zellen inkorporieren bei dieser Behandlung die Prüfverbindung in ihre Membran), so kommt es in 18 Tagen zu keinem Tumorwachstum. Am 19. Tag werden die Tiere jeweils mit $10^6$ nativen Ehrlich Ascites Tumorzellen intraperitoneal belastet. Folgende Effekte werden beobachtet :

Gruppe 1 : 8 von 10 Tieren überleben den 80. Tag
Gruppe 2 : 6 von 10 Tieren überleben den 80. Tag
Gruppe 3 : die Tiere sterben, wie die Kontrolltiere, nach 18 Tagen.

Die Verbindungen gemäss der vorliegenden Erfindung sind zudem wenig toxisch : Auch 5-malige intraperitoneale Applikation in einer Dosis von 100 mg/kg/Tag an fünf aufeinanderfolgenden Tagen wurde von Mäusen anscheinend symptomlos vertragen. Da die für die Immunstimulation benötigten Dosen sehr gering sind, ist die therapeutische Breite der neuen Verbindungen sehr gross.

Die neuen Verbindungen gemäss der vorliegenden Erfindung können somit die zelluläre und besonders die humorale Immunität erheblich steigern, und zwar sowohl in Mischung mit dem Antigen selber (Adjuvanseffekt im engeren Sinne) als auch bei zeitlich und örtlich von der Antigeninjektion getrennter Zufuhr (systemische Immunpotenzierung).

Die neuen Verbindungen gemäss der vorliegenden Erfindung können somit als Adjuvantien in Mischung mit Impfstoffen dazu benützt werden, den Impferfolg zu verbessern und den durch humorale Antikörper und/oder zelluläre Immunität vermittelten Infektionsschutz gegenüber bakteriellen, viralen oder parasitären Erregern zu verbessern.

Schliesslich eignen sich die beschriebenen Verbindungen in Mischung mit verschiedenen Antigen als Adjuvantien bei der experimentellen und industriellen Herstellung von Antiseren für Therapie und Diagnostik und bei der Induktion von immunologisch aktivierten Lymphocytenpopulationen für Zelltransferverfahren.

Darüberhinaus können die neuen Verbindungen auch ohne gleichzeitige Antigenzufuhr dazu benützt werden, bereits unterschwellig ablaufende Immunreaktionen bei Mensch und Tier zu fördern. Die Verbindungen eignen sich demnach besonders für die Stimulation der körperlichen Abwehr, z. B. bei chronischen und akuten Infektionen oder bei selektiven (antigenspezifischen) immunologischen Defekten, sowie bei angeborenen, aber auch bei erworbenen allgemeinen (d. h. nicht antigenspezifischen) immunologischen Defektzuständen, wie sie im Alter, im Verlauf schwerer Primärerkrankungen und vor allem nach Therapie mit ionisierenden Strahlen oder mit immunosuppressiv wirkenden Hormonen auftreten. Die genannten Stoffe können somit vorzugsweise auch in Kombination mit Antibiotika, Chemotherapeutika oder anderen Heilmitteln verabreicht werden. Schliesslich sind die beschriebenen Stoffe auch zur allgemeinen Prophylaxe von Infektionskrankheiten bei Mensch und Tier geeignet.

Die Kombination der erfindungsgemässen Muramylpeptide mit Antibiotika gehört zum Gegenstand der Erfindung und bewirkt eine Steigerung der antibiotischen Aktivität. Zu diesem Zwecke verwendet man eine wirksame oder unter-wirksame Dosis des Antibiotikums, je nach Art des letzteren z. B. von etwa 20 bis etwa 750 mg pro Einzeldosis.

Die Muramylpeptide der Formel I werden in Einzeldosen von etwa 5 mg bis ungefähr zur halben Antibiotikamenge verwendet. Dabei kann das Muramylpeptid-Derivat bis zu 24 Stunden vor oder nach, vorzugsweise jedoch etwa gleichzeitig mit dem Antibiotikum verabreicht werden.

Die Verabreichung der Antibiotika erfolgt auf dem üblichen Wege, wie subkutan, intravenös oder oral, während man die Muramylpeptide, insbesondere wenn sie getrennt von den Antibiotika verabreicht werden, meist subkutan verabfolgt.

Bei diesem Verfahren kann man einzelne Antibiotika, wie auch Antibiotikagemische verwenden. Antibiotikapräparate, welche dadurch gekennzeichnet sind, dass sie eines oder mehrere der vorgenannten Antibiotika und mindestens ein Muramylpeptid der Formel I enthalten, weisen die üblichen Mengen an Antibiotika, z. B. zwischen 20 und 1 000 mg, bevorzugt zwischen etwa 200 und 500 mg auf, sowie 5 mg bis zur Hälfte der Antibiotikamenge an Muramylpeptid der Formel I. Diese Präparate können, insbesondere wenn sie für orale Gabe zu verwenden sind, ausserdem noch die üblichen Mengen an pharmakologischen Trägerstoffen, Streck- und/oder Verdünnungsmitteln enthalten.

Die hohe antibiotische Wirkung des neuen Verfahrens und der neuen Präparate kann durch « in vivo »-Versuche nachgewiesen werden, die an verschiedenen Tierarten, insbesondere Säugetieren, wie Mäusen, durchgeführt werden. Dazu infiziert man sie mit einer letalen oder sub-letalen Dosis eines pathogenen Microorganismus und gibt dann das genannte neue Präparat bzw. die einzelnen Dosen an Muramylpeptid und Antibiotikum. Die Wirkung wird als $ED_{50}$ bestimmt, das ist diejenige Dosis bei denen 50 % der Tiere überleben.

Es wurde nun überraschenderweise gefunden, dass die Infektion mit pathogenen Keimen, insbesondere der weniger beeinflussbaren gramnegativen Bakterien, wie z. B. Stämmen von Aerobakter, Brucella, Escherichia, Klebsiella, Malleomyces, Neisseria, Pasteurella, Proteus, Pseudomonas, Shigella und Vibro, aber auch von gram-positiven Bakterien, wie von Aktinomycetes, Clostridia, Corynebakterien, Diplokokken, Mycobakterien oder Staphylococcen, oder von Pilzen, wie Candida Albicans, Cryptokokkus neoformans, Plastomyces dermatitides oder Hystoplasma capsulatum, in erhöhtem Masse gehemmt und bekämpft wird.

Von den zur Kombination mit den erfindungsgemässen Muramylpeptiden in Frage kommenden Antibiotika sind besonders solche aus den Gruppen der β-Lactamantibiotika, Aminoglycoside, Tetracycline, Macrolide, Lincomycine, Polyenantibiotika, Polypeptidantibiotika, Anthracycline, Chlor- oder Thiamphenicole, Cycloserine, Fusidinsäuren oder Rifamycine zu nennen.

Als bevorzugte Antibiotika seien unter den β-Lactamen die Penicilline, Cephalosporine, Peneme, Nocardicine, Thienamycine und Clavulansäuren genannt.

Penicillin-Antibiotika sind in erster Linie Amoxycillin, Ampicillin, Carbenicillin, Cloxacillin, Cyclacillin, Dicloxacillin, Mecillinam, Methicillin, Penicillin G, Penicillin V, Pivampicillin, Sulbenicillin, Azlocillin, Ticarcillin, Mezlocillin, Pivmecillinam oder 6-(4-endo-Azatricyclo $[5.2.2.0^{2,6}]$ undec-8-enyl)-methylenamino-penicillansäure.

Aus der Gruppe der Cephalosporine können z. B. Cefaclor, Cefazaflur, Cefazolin, Cefadroxil, Cefoxitin, Cefuroxim, Cephacetril, Cephalexin, Cephaloglycin, Cephaloridine, Cephalotin, Cefamandol, Cephanon, Cephapirin, Cefatrizin, Cephradin, Cefroxadin (7β-[D-2-amino-2-(1,4-cyclohexadienyl)-acetamido]-3-methoxy-3-cephem-4-carbonsäure = CGP 9 000), Cefsulodin, Cefotaxim, Cefotiam, Ceftezol oder Cefazedon genannt werden.

Unter den Nocardicinen ist z. B. Nocardicin A und unter den Thienamycinen und Clavulansäuren z. B. Thienamycin bzw. Clavulansäure zu erwähnen.

Unter den Aminoglycosiden sind insbedondere Streptomycine, z. B. Streptomycin und Streptomycin A, Neomycine, z. B. Neomycin B, Tobramycine, z. B. Tobramycin oder Dibekacin, Kanamycine (z. B. Gemische von Kanamycin A, B und C), sowie Amicacine, Gentamycine (z. B. Gemische von Gentamycin A, $C_1$, $C_2$ oder $C_{1a}$), oder Sisomicine, wie Sisomicin oder Netilmicin, ferner Lividomycin, Ribocamycin und Paromomycin zu erwähnen.

Als Tetracycline sind insbesondere Tetracyclin, Doxycyclin, Chlortetracyclin, Oxytetracyclin oder Methacyclin zu nennen.

Als Macrolide sind z. B. Maridomycin, Spiramycine, wie Spiramycin I, II und III, Erythromycine, z. B. Erythromycin, Oleandomycine, z. B. Oleandomycin und Tetraacetylodeandomycin, und als Lincomycine z. B. Lincomycin und Clindamycin zu erwähnen.

Als Polyen-Antibiotika sind besonders Amphothericin B und dessen Methylester oder Nystalin zu nennen.

Als Polypeptid-Antibiotika können z. B. Colistin, Gramicidin S, Polymixin B, Virginamycin, Tyrothricin, Viomycin oder Vancomycin besonders genannt werden.

Als Rifamycine kommen in erster Linie das Rifamycin S, Rifamycin SV oder Rifamycin B oder deren halbsynthetische Derivate, insbesondere das Rifampicin, in Frage.

Die vorliegende Anmeldung betrifft besonders Verbindungen der Formel I, worin X Carbonyl, $R_1$ gegebenenfalls substituiertes Alkyl oder Aryl, $R_2$, $R_3$, $R_4$ und $R_6$ Wasserstoff oder Niederalkyl, $R_5$ Wasserstoff, gegebenenfalls durch Hydroxy, Niederalkoxy, Mercapto oder Niederalkylmercapto oder Halogen substituiertes Niederalkyl, Cycloalkyl oder Cycloalkylniederalkyl, worin der Cycloalkylrest 4 bis 6 Kohlenstoffatome enthält, gegebenenfalls substituiertes Phenyl oder Phenylniederalkyl, ein oder zwei Azaatome enthaltendes Heterocyclyl oder Heterocyclylniederalkyl oder $R_4$ und $R_5$ zusammen auch

Alkylen mit 3 bis 4 Kohlenstoffatomen, $R_7$ Wasserstoff und einer der Reste $A_1$ und $A_2$ einen Rest der Formel

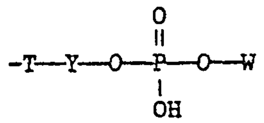

(II)

bedeuten, worin T für NH oder O steht, worin Y gegebenenfalls substituiertes Alkylen, das auch durch Oxycarbonyl oder Iminocarbonyl unterbrochen sein kann und W eine gegebenenfalls durch Hydroxy, Niederalkanoyloxy, Amino, Alkanoylamino oder Oxo substituierte Alkylgruppe mit mehr als 6 Kohlenstoffatomen bedeuten, und der andere der Reste $A_1$ und $A_2$ Hydroxy, Niederalkoxy, Amino, Niederalkylamino oder im Niederalkylrest gegebenenfalls durch Hydroxy, Carboxy und/oder Amino substituiertes Aminocarbonylniederalkylamino ist, und ihre Salze.

Die Erfindung betrifft insbesondere Verbindungen der Formel I, worin X Carbonyl, $R_1$ gegebenenfalls durch Hydroxy, Niederalkoxy oder Halogen substituiertes Niederalkyl oder gegebenenfalls durch Hydroxy, Niederalkoxy, Niederalkyl oder Halogen substituiertes Phenyl, $R_2$, $R_4$ und $R_6$ Wasserstoff, $R_3$ Wasserstoff oder Niederalkyl, $R_5$ Wasserstoff, gegebenenfalls durch Hydroxy, Niederalkoxy, Mercapto, Niederalkylmercapto oder Halogen substituiertes Niederalkyl mit 1 bis 3 Kohlenstoffatomen, Cycloalkyl oder Cycloalkylniederalkyl, worin der Cycloalkylrest 4 bis 6 Kohlenstoffatome und der Niederalkylrest 1 bis 3 Kohlenstoffatome enthält, gegebenenfalls durch Hydroxy, Niederalkoxy oder Halogen substituiertes Phenyl oder Phenylniederalkyl mit 1 bis 3 Kohlenstoffatomen im Niederalkylrest, ein oder zwei Azaatome enthaltendes Heterocyclyl oder Heterocyclylniederalkyl mit 5 bis 6 Ringgliedern und 1 bis 3 Kohlenstoffatomen im Niederalkylrest oder $R_4$ und $R_5$ zusammen auch Alkylen mit 3-4 Kohlenstoffatomen, $R_7$ Wasserstoff und einer der Reste $A_1$ und $A_2$ einen Rest der Formel

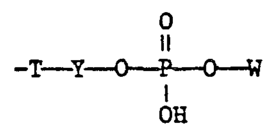

(II)

bedeuten, worin T für HN oder O steht, Y gegebenenfalls substituiertes Niederalkylen oder einen Rest einer der Formeln

$$Y_1—COO—Y_2 \quad \text{(IIIa)}$$

$$Y_1—OOC—Y_2 \quad \text{(IIIb)}$$

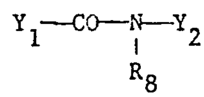

(IIIc)

oder

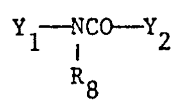

(IIId),

bedeutet, worin $Y_1$ und $Y_2$ je für gegebenenfalls substituiertes Niederalkylen und $R_8$ für Wasserstoff stehen, W eine Alkylgruppe mit 10 bis 25 Kohlenstoffatomen, die in 2-Stellung eine Hydroxygruppe, Alkanoylxygruppe, Amino- oder Alkanoylaminogruppe trägt, darstellt und der andere der Reste $A_1$ und $A_2$ Hydroxy, Niederalkoxy, Amino, Niederalkylamino oder im Niederalkylrest gegebenenfalls durch Hydroxy, Carboxy oder Aminogruppen substituiertes Aminocarbonylniederalkylamino ist, und ihre Salze.

Die Erfindung betrifft in erster Linie Verbindungen der Formel I, worin X Carbonyl, $R_1$ Niederalkyl mit 1 bis 3 Kohlenstoffatomen oder Phenyl, $R_2$, $R_4$ und $R_6$ Wasserstoff, $R_3$ Wasserstoff oder Niederalkyl mit 1 bis 3 Kohlenstoffatomen, $R_5$ Wasserstoff, gegebenenfalls durch Hydroxy, Methoxy, Mercapto, Methylmercapto oder Halogen substituiertes Niederalkyl mit 1 bis 3 Kohlenstoffatomen, gegebenenfalls durch Hydroxy, Methoxy oder Halogen substituiertes Phenyl oder Phenylmethyl, ein oder zwei Azaatome enthaltendes Heterocyclyl oder Heterocyclylmethyl mit 5 Ringgliedern oder $R_4$ und $R_5$ zusammen auch Trimethylen, $R_7$ Wasserstoff und einer der Reste $A_1$ und $A_2$ einen Rest der Formel

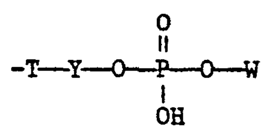

(II)

bedeuten, worin T für HN oder O steht, Y Niederalkylen mit 2 bis 3 Kohlenstoffatomen oder einen Rest der Formel (IIIa) oder (IIIc)

$$Y_1 - COO - Y_2 \qquad \text{(IIIa)}$$

$$Y_1 - CO - \underset{\underset{R_8}{|}}{N} - Y_2 \qquad \text{(IIIc)}$$

bedeutet, worin $R_8$ für Wasserstoff steht und $Y_1$ und $Y_2$ unabhängig voneinander je gegebenenfalls durch Hydroxy, Niederalkoxy, Mercapto oder Niederalkylmercapto substituiertes Niederalkylen mit 1 bis 3 Kohlenstoffatomen oder Niederalkylen mit 1 bis 3 Kohlenstoffatomen oder Niederalkylen mit 1 bis 3 Kohlenstoffatomen bedeuten, das durch gegebenenfalls durch Hydroxy, Methoxy oder Halogen substituiertes Phenyl oder Phenylniederalkyl oder ein oder zwei Azaatome enthaltendes Heterocyclyl oder Heterocyclylniederalkyl mit 5 bis 6 Ringgliedern und 1 bis 3 Kohlenstoffatomen im Niederalkylrest substituiert ist, W eine Alkylgruppe mit 10 bis 25 Kohlenstoffatomen, die in 2-Stellung durch Hydroxy, Niederalkanoyloxy, Amino oder Alkanoylamino substituiert ist, und der andere der Reste $A_1$ und $A_2$ Hydroxy, Niederalkoxy, Amino, Niederalkylamino oder Aminocarbonylniederalkylamino ist, und ihre Salze.

Hauptsächlich betrifft die Erfindung Verbindungen der Formel I, worin X Carbonyl, $R_1$ Niederalkyl mit 1 bis 3 Kohlenstoffatomen oder Phenyl, $R_2$, $R_4$ und $R_6$ Wasserstoff, $R_3$ Wasserstoff oder Niederalkyl mit 1 bis 3 Kohlenstoffatomen, $R_5$ Wasserstoff, gegebenenfalls durch Hydroxy, Methoxy, Mercapto, Methylmercapto oder Halogen substituiertes Niederalkyl mit 1 bis 3 Kohlenstoffatomen, gegebenenfalls durch Hydroxy, Methoxy oder Halogen substituiertes Phenyl oder Phenylmethyl, ein oder zwei Azaatome enthaltendes Heterocyclyl oder Heterocyclylmethyl mit 5 Ringgliedern oder $R_4$ und $R_5$ zusammen auch Trimethylen, $R_7$ Wasserstoff und einer der Reste $A_1$ und $A_2$ einen Rest der Formel

$$-T-Y-O-\overset{\overset{O}{\|}}{\underset{\underset{OH}{|}}{P}}-O-W \qquad \text{(II)}$$

bedeuten, worin T für HN oder O steht, Y Niederalkylen mit 2 bis 3 Kohlenstoffatomen oder einen Rest der Formeln (IIIa) oder (IIIc)

$$Y_1 - COO - Y_2 \qquad \text{(IIIa)}$$

$$Y_1 - CO - \underset{\underset{R_8}{|}}{N} - Y_2 \qquad \text{(IIIc)}$$

bedeutet, worin $R_8$ für Wasserstoff steht und $Y_1$ und $Y_2$ unabhängig voneinader je gegebenenfalls durch Hydroxy, Niederalkoxy, Mercapto oder Niederalkylmercapto substituiertes Niederalkylen mit 1 bis 3 Kohlenstoffatomen oder Niederalkylen mit 1 bis 3 Kohlenstoffatomen bedeuten, das durch gegebenfalls durch Hydroxy, Methoxy oder Halogen substituiertes Phenyl oder Phenylniederalkyl oder ein oder zwei Azaatome enthaltendes Heterocyclyl oder Heterocyclylniederalkyl mit 5 bis 6 Ringgliedern und 1 bis 3 Kohlenstoffatomen im Niederalkylrest substituiert ist, W eine Alkylgruppe mit 10 bis 25 Kohlenstoffatomen, die in 2-Stellung durch Hydroxy, Niederalkanoyloxy, Amino oder Alkanoylamino substituiert ist, und der andere der Reste $A_1$ und $A_2$ Hydroxy, Niederalkoxy, Amino, Niederalkylamino oder Aminocarbonylniederalkylamino ist, und ihre Salze.

Vornehmlich betrifft die Erfindung Verbindungen der Formel I, worin X Carbonyl, $R_1$ Niederalkyl mit 1 bis 7 Kohlenstoffatomen oder Phenyl, $R_2$, $R_4$, $R_6$ und $R_7$ Wasserstoff, $R_3$ Wasserstoff oder Niederalkyl, $R_5$ Wasserstoff, Niederalkyl, Hydroxymethyl, Mercaptomethyl, 1-Hydroxy-äthyl, 2-Methylthio-äthyl, Phenylmethyl, p-Hydroxy-phenyl-methyl, 4-Amino-butyl, 4-Imidazolyl-methyl, 3-Indolyl-methyl oder $R_4$ und $R_5$ zusammen auch Trimethylen bedeuten, worin $A_1$ für Hydroxy, Niederalkoxy, Amino, Niederalkylamino oder Aminocarbonylniederalkylamino steht und $A_2$ einen Rest der Formel

$$-T-Y-O-\overset{\overset{O}{\|}}{\underset{\underset{OH}{|}}{P}}-O-W \qquad \text{(II)}$$

bedeutet, worin T für NH oder O und W für eine Alkyl- oder Alkenylgruppe, die unsubstituiert oder durch Hydroxy, Niederalkoxy, Niederalkanoyloxy, Amino, Niederalkylamino oder Alkanoylamino mit bis zu 25 Kohlenstoffatomen substituiert ist, oder für einen Cycloalkyloder Cycloalkenylrest mit 10 bis 30 Kohlenstoffatomen stehen, Y Aethylen oder einen Rest der Formeln

$$Y_1 - COO - Y_2 \qquad \text{(IIIa)}$$

oder

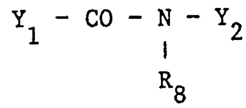

(IIIc)

bedeutet, worin $R_8$ für Wasserstoff steht und $Y_1$ und $Y_2$ unabhängig voneinander je für Niederalkylen mit 1 bis 7 Kohlenstoffatomen stehen, das gegebenenfalls durch Hydroxy, Niederalkoxy, Mercapto, Methylthio, Phenyl, 4-Imidazolyl oder 3-Indolyl substituiert ist, und ihre Salze, sowie derartige Verbindungen, worin die Bedeutungen für $A_1$ und $A_2$ vertauscht sind, und ihre Salze.

Ganz besonders betrifft die Erfindung Verbindungen der Formel I, worin X Carbonyl, $R_1$ Niederalkyl mit 1 bis 3 Kohlenstoffatomen, $R_2$, $R_4$, $R_6$ und $R_7$ Wasserstoff, $R_3$ Wasserstoff oder Methyl, $R_5$ Wasserstoff oder Niederalkyl, $A_1$ Amino und $A_2$ einen Rest der Formel

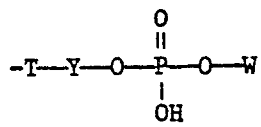

(II)

bedeuten, worin T für NH, W für eine Alkyl- oder Alkenylgruppe mit 10 bis 25 Kohlenstoffatomen, die unsbustituiert oder durch Hydroxy, Niederalkoxy, Niederalkanoyloxy, Amino, Niederalkylamino oder Alkanoylamino mit bis zu 25 Kohlenstoffatomen substituiert ist, oder für einen Cholesterylrest und Y für Aethylen oder einen Rest der Formel

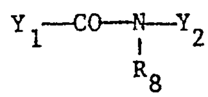

(IIIc)

stehen, worin $R_8$ Wasserstoff und $Y_1$ und $Y_2$ unabhängig voneinander je Niederalkylen bedeuten, und ihre Salze, sowie derartige Verbindungen, worin die Bedeutungen für $A_1$ und $A_2$ vertauscht sind, und ihre Salze.

Insbesondere betrifft die Erfindung die neuen, in den Beispielen beschriebenen Muramylpeptide.

Die neuen Verbindungen der Formel I können nach an sich bekannten Methoden erhalten werden. So lassen sie sich erhalten, wenn man eine Verbindung der Formel

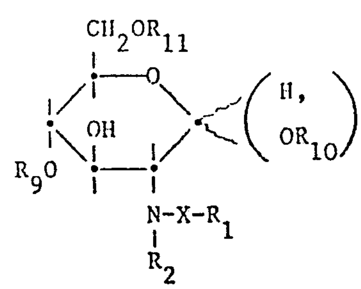

(V)

worin X, $R_1$ und $R_2$ die obgenannte Bedeutung haben und gegebenenfalls darin vorhandene Hydroxygruppen mit einer leicht abspaltbaren Schutzgruppe geschützt sind, $R_9$, $R_{10}$ und $R_{11}$ je eine leicht abspaltbare Scghutzgruppe bedeuten, oder eine Metallverbindung davon mit einer Verbindung der Formel

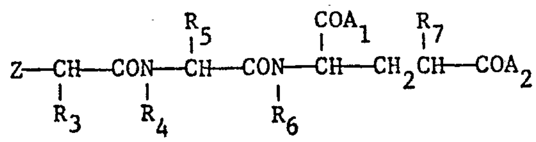

(VI)

umsetzt, worin Z eine reaktionsfähige veresterte Hydroxygruppe darstellt, $R_3$, $R_4$, $R_5$, $R_6$, $R_7$, $A_1$ und $A_2$

die oben angegebene Bedeutung haben und gegebenenfalls darin vorhandene Hydroxygruppen durch eine leicht abspaltbare Schutzgruppe geschützt sind, und vorhandene Schutzgruppen abspaltet.

Eine reaktionsfähige veresterte Hydroxygruppe ist insbesondere eine mit einer starken anorganischen oder organischen Säure veresterte Hydroxygruppe, in erster Linie eine solche, die mit einer Halogenwasserstoffsäure, wie Chlor-, Brom- oder insbesondere Jodwasserstoffsäure, verestert ist.

Eine Metallverbindung ist insbesondere ein entsprechendes Alkalimetall-, z. B. Natrium- oder Kaliumderivat. Sie kann z. B. durch Behandeln einer Verbindung der Formel V mit einer geeigneten Base, wie einer entsprechenden Alkalimetallverbindung, wie Natriumhydrid, Natriumamid oder Butyllithium, hergestellt werden.

Leicht abspaltbare Schutzgruppen sind solche die aus der Peptid- bzw. Zuckerchemie bekannt sind. Für Hydroxygruppen sollen insbesondere Acylreste, z. B. Niederalkanoylreste wie Acetyl, Aroylreste, wie Benzoyl und vor allem von Kohlensäurederivaten sich ableitende Reste, wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl, gegebenenfalls durch Halogen oder Niederalkoxy, wie Methoxy, substituiertes Triphenylmethyl, oder Tretrahydropyranyl oder gegebenenfalls substituierte Alkylidenreste, die die Sauerstoffatome in 4- und 6-Stellung des Glucoseteils verbinden, genannt werden. Solche Alkylidenreste sind insbesondere ein Niederalkyliden-, in erster Linie der Methyliden-, Isopropyliden- oder Propylidenrest, oder auch ein gegebenenfalls substituierter Benzylidenrest.

Diese Schutzgruppen können in an sich bekannter Weise abgespalten werden. So kann man sie durch saure Hydrolyse, Benzyl- oder Benzylidenreste auch hydrogenolytisch, z. B. mit Wasserstoff in Gegenwart eines Edelmetall-, wie Palladium- oder Platinkatakysators, entfernen.

Die verwendeten Ausgangsstoffe sind bekannt oder lassen sich in an sich bekannter Weise herstellen.

Die neuen Verbindungen können auch erhalten werden, wenn man in an sich bekannter Weise eine Verbindung der Formel

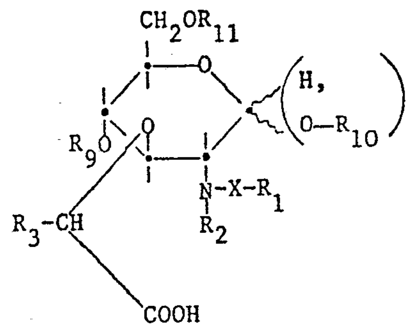

(VII)

worin X, $R_1$, $R_2$ und $R_3$ die obengenannte Bedeutung besitzen und $R_9$, $R_{10}$ und $R_{11}$ für Wasserstoff oder eine leicht abspaltbare Schutzgruppe stehen, oder ein Derivat davon mit einer Verbindung der Formel

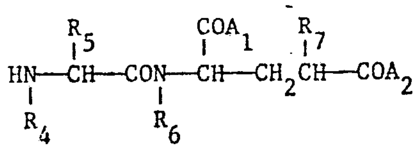

(VIII)

worin $R_4$, $R_5$, $R_6$, $R_7$, $—COA_1$ und $—COA_2$ die obgenannte Bedeutung besitzen, mit der Massgabe, dass in diesen Resten vorhandene Carboxy- und, wenn erwünscht, freie Hydroxygruppen durch leicht abspaltbare Schutzgruppen geschützt sind, oder einem Derivat davon kondensiert und vorhandene Schutzgruppen abspaltet.

Die Kondensation erfolgt dabei z. B. in der Weise, dass man die Säure (VII) in aktivierter Form mit der Aminoverbindung (VIII) umsetzt oder dass man die Säure (VII) mit der Verbindung (VIII), deren Aminogruppe in aktivierter Form vorliegt, umsetzt. Die aktivierte Carboxylgruppe kann beispielsweise ein Säureanhydrid, vorzugsweise ein gemischtes Säureanhydrid wie z. B. mit Kohlensäureniederalkylester, wie Kohlensäureäthyl- oder isobutylester, ein Säureazid, ein Säureamid, wie ein Imidazolid, oder ein aktivierter Ester sein. Als aktivierte Ester seien insbesondere genannt : Cyanmethylester, Carboxymethylester, p-Nitrophenylthioester, p-Nitrophenylester, 2,4,5-Trichlorphenylester, Pentachlorphenylester, N-Hydroxy-succinimidester, N-Hydroxy-phthalimidester, 8-Hydroxychinolinester, N-Hydroxy-1,2-dihydro-1-carboäthoxychinolinester, N-Hydroxypiperidinester oder Enolester, die mit N-Aethyl-5-phenyl-isoxazolium-3'-sulfonat gebildet werden. Aktivierte Ester können auch gegebenenfalls mit einem Carbodiimid unter Zusatz von N-Hydroxysuccinimid oder einem unsubstituierten oder z. B. durch Halogen, Methyl oder Methoxy substituierten 1-Hydroxybenzotriazol, 3-Hydroxy-4-oxo-3,4-dihydro-benzo[d]-1,2,3-triazin erhalten werden.

Die Aminogruppe ist beispielsweise durch Reaktion mit einem Phosphit aktiviert.

Unter den Methoden der Reaktion mit aktivierten Säuren sind insbesondere diejenigen mit N-Aethyl-5-phenyl-isoxazolium-3'-sulfonat (Woodward Reagens K) oder 2-Aethoxy-1,2-dihydro-1-carboäthoxy-chinolin oder Carbodiimid zu erwähnen.

Leicht abspaltbare Schutzgruppen sind solche die aus der Peptid- bzw. Zuckerchemie bekannt sind. Für Carboxygruppen sollen insbesondere tertiär-Butyl, Benzyl oder Benzhydryl und für Hydroxygruppen insbesondere Acylreste, z. B. Niederalkanoylreste, wie Acetyl, Aroylreste, wie Benzoyl, und vor allem von Kohlensäurederivaten sich ableitende Reste wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl, gegebenenfalls durch Halogen oder Niederalkoxy, wie Methoxy, substituiertes Triphenylmethyl oder Tetrahydropyranyl oder gegebenenfalls substituierte Alkylidenreste, die die Sauerstoffatome in 4- und 6-Stellung des Glucoseteils verbinden, genannt werden. Solche Alkylidenreste sind insbesondere ein Niederalkyliden-, in erster Linie der Methyliden-, Isopropyliden- oder Propylidenrest oder auch ein gegebenenfalls substituierter, vorzugsweise in p-Stellung substituierter Benzylidenrest.

Diese Schutzgruppen können in an sicht bekannter Weise abgespalten werden. So kann man sie durch saure Hydrolyse, Benzyl- oder Benzylidenreste auch hydrogenolytisch, z. B. mit Wasserstoff in Gegenwart eines Edelmetall-, wie Palladium- oder Platinkatalysators, entfernen.

Die verwendeten Ausgangsstoffe sind bekannt oder lassen sich in an sich bekannter Weise herstellen.

Eine andere Verfahrensweise zur Herstellung dieser neuen Verbindungen besteht darin, dass man eine verbindung der Formel

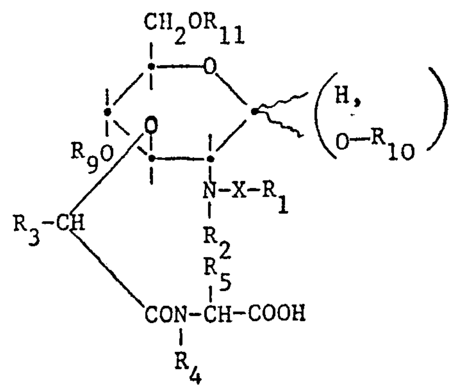

(IX)

worin X, $R_1$, $R_2$, $R_3$, $R_4$ und $R_5$ die obenganannte Bedeutung haben mit der Massgabe, dass darin enthaltene freie Hydroxygruppen gegebenenfalls mit einer leicht abspaltbaren Schutzgruppe geschützt sind, und $R_9$, $R_{10}$ und $R_{11}$ Wasserstoff oder leicht abspaltbare Schutzgruppen darstellen, oder Derivate davon mit einer Verbindung der Formel

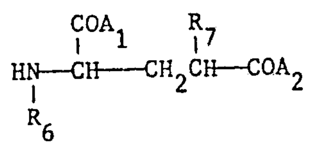

(X)

worin $R_6$, $R_7$, $A_1$ und $A_2$ die obgenannte Bedeutung haben, mit der Massgabe, dass in den Resten $R_7$, $COA_1$ und $COA_2$ vorhandene freie Caboxylgruppen durch leicht abspaltbare Schutzgruppen geschützt sind, kondensiert und vorhandene Schutzgruppen abspaltet.

Die Kondensation erfolgt dabei z. B. in der Weise, dass man die Säure IX in aktivierter Form mit der Aminoverbindung X umsetzt, oder dass man die Säure IX mit der Verbindung X, deren Aminogruppe in aktivierter Form vorliegt, umsetzt. Die aktivierte Carboxylgruppe kann beispielsweise ein Säureanhydrid, vorzugsweise ein gemischtes Säureanhydrid, ein Säureamid oder ein aktivierter Ester sein. Als solche kommen insbesondere die obengenannten Säureanhydride, Amide oder Ester in Frage. Die Aminogruppe ist beispielsweise durch Reaktion mit einem Phosphit aktiviert.

Auch die leicht abspaltbaren Schutzgruppen entsprechen den bereits oben genannten. Sie können in an sich bekannter Weise abgespalten werden ; durch saure Hydrolyse, Benzyl- oder Benzylidenreste auch hydrogenolytisch, beispielsweise mit Wasserstoff in Gegenwart eines Edelmetall-, wie Palladium- oder Platinkatalysators.

Die Ausgangsstoffe lassen sich in an sich bekannter Weise erhalten. So kann man z. B. entsprechende in 3-Stellung unsubstituierte Zucker mit einem Halogen-$R_3$-essigsäure-$R_4$-amid umsetzen, oder eine Verbindung der Formel VII mit einer $R_4$-Amino-$R_5$-essigsäure, deren Carboxylgruppe geschützt ist, in der oben gezeigten Weise umsetzen und die Schutzgruppen abspalten.

Eine weitere Verfahrensweise zur Herstellung dieser neuen Verbindungen der Formel I, worin T für NH steht, besteht darin, dass man in an sich bekannter Weise eine Verbindung der Formel

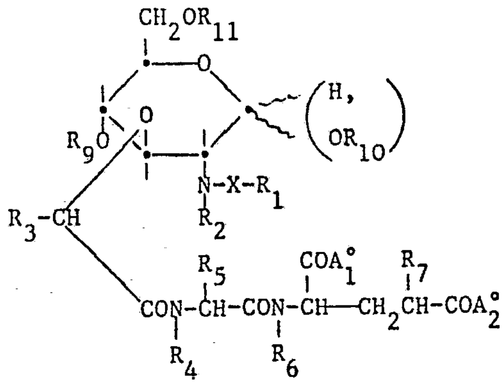

(XI)

worin X, $R_1$, $R_2$, $R_3$, $R_4$, $R_5$, $R_6$ und $R_7$ die obgenannte Bedeutung haben, $R_9$, $R_{10}$ und Wasserstoff oder eine leicht abspaltbare Schutzgruppe darstellen und einer der Reste $A_1°$ und $A_2°$ eine aktivierte Hydroxygruppe darstellt und der andere veräthertes Hydroxy, Amino, Niederalkylamino oder Aminocarbonylniederalkylamino ist, mit einer Verbindung der Formel

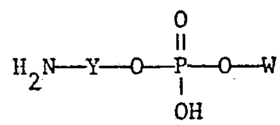

(XII)

worin Y und W die obgenannte Bedeutung besitzen, kondenziert, und gegebenenfalls vorhandene Schutzgruppen abspaltet.

Die aktivierte Carbonsäuregruppe $COA_1°$ bzw. $COA_2°$ kann beispielsweise ein Säureanhydrid z. B. mit Kohlensäureniederalkylester, wie Kohlensäureäthyl- oder Isobutylester, ein Säureazid, ein Säureamid, wie ein Imidazolid, Isoxazolid oder ein aktivierter Ester sein. Als aktivierte Ester seien insbesondere genannt: Cyanmethylester, Carboxymethylester, p-Nitrophenylthioester, Methoxyäthylthioester, Acetylaminoäthylthioester, p-Nitrophenylester, 2, 4, 5-trichlorphenylester, N-Hydroxy-succinimidester, N-Hydroxy-phthalimidester, 8-Hydroxy-chinolinester, N-Hydroxy-piperidinester. Aktive Ester können auch gegebenenfalls mit einem Carbodiimid unter Zusatz von N-Hydroxysuccinimid oder einem unsubstituierten oder z. B. durch Halogen, Methyl oder Methoxy substituierten 1-hydroxybenzotriazol oder 3-Hydroxy-4-oxo-3,4-dihydro-benzo[d]-1,2,3-triazin erhalten werden.

Man bevorzugt als aktive Ester solche mit N-Hydroxysuccinimid oder deren C-Substitutionsprodukten, wie N-Hydroxy-methyl- oder -dimethyl-succinimid, oder die Umsetzung mit Carbodiimid, wie Carbodiimid selbst oder 1-Aethyl-3-(3-dimethylaminopropyl)-carbodiimid.

Die dazu verwendeten Ausgangstoffe sind bekannt oder lassen sich in an sich bekannter Weise herstellen.

Steht in den neuen Verbindungen der Formel I T für O, kann man sie auch herstellen, wenn man in an sich bekannter Weise eine Verbindung der Formel

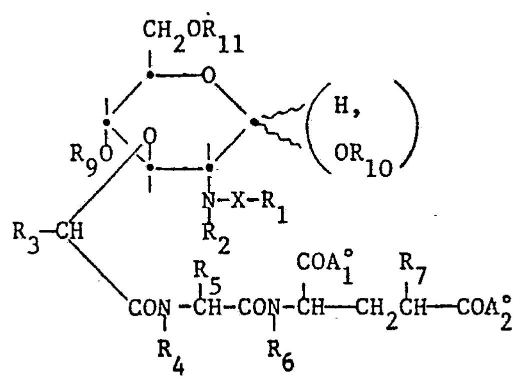

(XIa)

worin X, $R_1$, $R_2$, $R_3$, $R_4$, $R_5$, $R_6$ und $R_7$ die obengenannte Bedeutung haben, $R_9$, $R_{10}$ und $R_{11}$ Wasserstoff oder eine leicht abspaltbare Schutzgruppe darstellen und einer der Reste $A_1°$ und $A_2°$ eine Hydroxygruppe darstellt und der andere veräthertes Hydroxy, Amino, Niederalkylamino oder Aminocarbonylniederalkylamino ist, mit einer Verbindung der Formel

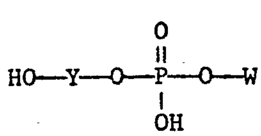

(XIIa)

worin Y und W die obgenannte Bedeutung besitzen, wobei die Säure XIa oder der Alkohol XIIa in reaktionsfähiger Form vorliegt, in an sich bekannter Weise verestert und gegebenenfalls vorhandene Schutzgruppen abspaltet.

Diese Reaktion lässt sich so durchführen, dass man die freie Säure mit dem Alkohol in Gegenwart eines wasserabspaltenden Mittels, wie einem carbodiimid, z. B. Dicyclohexylcarbodiimid und einem Amin, wie Pyridin oder Dimethylaminopyridin oder einem Trialkylamin, z. B. Trimethylamin, verestert. Man kann aber auch die Carbonsäure, z. B. in Form eines Salzes, wie Natrium- ode Kaliumsalzes, mit einem reaktionsfähigen Ester des Alkohols, z. B. einem Ester mit einer starken anorgasnischen oder organischen Säure, wie einer Halogen-wasserstoffsäure, z. B. Chlor-, Brom- oder Jodwasserstoffsäure oder einer organischer Sulfonsäure, wie p-Toluolsaulfonsäure oder Methanoder Aethansulfonsäure, umsetzen.

Ferner ist es auch möglich, den Alkohol, gegebenenfalls als Salz, z. B. Natrium- oder Kaliumsalz, mit einer aktivierten Carbonsäure umsetzen. Als aktivierte Carbonsäure sind insbesondere Anhydride, in erster Linie gemischte Säureanhydride, Säureazide, Halogenide oder aktivierte Ester zu nennen, wie Cyanmethylester, Carboxymethylester, p-Nitrophenylthioester, p-Nitrophenylester, 2,4,5-Trichlorphenylester, Pentachlorphenylester, N-Hydroxysuccinimidester, N-Hydroxy-phthalimidester, 8-Hydroxychinolinester, 2-Hydroxy-1,2-dihydro-1-carboäthoxy-chinolinester, N-hydroxy-piperidinester oder Enolester, die mit N-Aethyl-5-phenylisoxazolium-3'-sulfonat gewonnen werden. Aktivierte Ester können auch gegebenenfalls mit einem Carbodiimid unter Zusatz von N-Hydroxysuccinimid oder einem unsubstituierten oder z. B. durch Halogen, Methyl oder Methoxy substituierten 1-Hydroxybenzotriazol oder 3-Hydroxy-4-oxo-3,4-dihydrobenzo[d]-1,2,3-triazin erhalten werden.

Leicht abspaltbare Schutzgruppen sind solche die aus der Peptid- bzw. Zuckerchemie bekannt sind. Für Carboxygruppen sollen insbesondere tertiär-Butyl, Benzyl oder benzhydryl und für Hydroxygruppen insbesondere Acylreste, z. B. Niederalkanoylreste, wie Acetyl, Aroylreste, wie Benzoyl, und vor allem von der Kohlensäure sich ableitende Rest wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl, gegebenenfalls durch Halogen oder Niederalkoxy, wie Methoxy substituiertes Triphenylmethyl oder Tetrahydropyranyl oder gegebenenfalls substituierte Alkylidenreste, die die Sauerstoffatome in 4- und 6-Stellung des Glucoseteils verbinden, genannt werden. Solche Alkylidenreste sind insbesondere ein Niederalkyliden-, in erster Linie der Aethyliden-, Isopropyliden- oder propylidenrest, oder auch ein gegebenenfalls substituierter, vorzugsweise in p-Stellung substitutierter Benzylidenrest.

Diese Schutzgruppen können in an sich bekannter Weise abgespalten werden. So kann man sie durch saure Hydrolyse, den Benzyl- oder Benzylidenrest auch hydrogenolytisch z. B. mit Wasserstoff in Gegenwart eines Edelmetall-, wie Palladium oder Platinkatalysators entfernen.

Ferner ist es auch möglich die neuen Verbindungen der Formel I, worin X eine Carbonylgruppe und $R_2$ Wasserstoff sind, zu erhalten, wenn man einer Verbindung der Formel

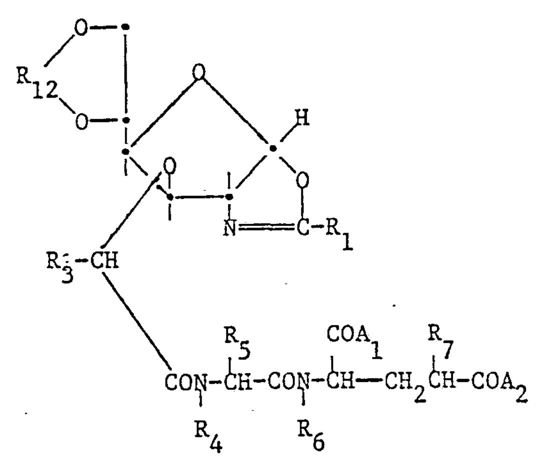

(XIII)

worin $R_1$, $R_3$, $R_4$, $R_5$, $R_6$, $R_7$ $A_1$ und $A_2$ die obengenannte Bedeutung haben und $R_{12}$ eine Alkyliden- oder Cycloalkylidengruppe ist, den Oxazolin- und den Dioxolanring sauer aufspaltet und gegebenenfalls vorhandene Schutzgruppen abspaltet.

Alkyliden ist darin insbesondere Niederalkyliden, wie Isopropyliden, und Cycloalkyliden in erster Linie Cyclopentyliden oder Cyclohexyliden.

Diese Spaltung erfolgt ebenfalls in an sicht bekannter Weise, z. B. mit einem sauren Ionenaustauscher, insbesondere solchen mit Sulfonsäuregruppen, wie Amberlite® IR-120 (ein Styrolharz mit stark sauren Sulfogruppen) oder Dowex® 50 (Polystyrolsulfonsäuren) oder einer starken anorganischen oder organischen Säure, wie Salzsäure, Bromwasserstoffsäure, Schwefelsäure oder einer Sulfonsäure, z. B. Methansulfonsäure, oder einer gegebenfalls im aromatischen Ring substituierten Phenylsulfonsäure, wie p-Toluolsulfonsäure, oder Trifluoressigsäure. Arbeitet man dabei in Gegenwart von Wasser, erhält man in 1-Stellung eine freie Hydroxygruppe. Ist auch eine der Carboxylgruppen —$COA_1$ bzw. —$COA_2$ und/oder

$R_7$ mit einem Alkohol, insbesondere einem Niederalkanol, verestert, lässt sie sich, insbesondere bei höherer Temperatur mit wässriger Saüre verseifen.

In den erhaltenen Verbindungen lassen sich Schutzgruppen am Peptidrest nachträglich, z. B. durch Hydrogenolyse, wie z. B. mit katalytisch angeregtem Wasserstoff, oder Hydrolyse, abspalten.

Die dabei verwendeten Ausgangsstoffe lassen sich z. B. erhalten, wenn man in ein entsprechendes Oxazolin, mit einer freien Hydroxygruppe in 3-Stellung des Zuckerrestes den $R_3$-Acetylaminopeptidrest in einer oder mehreren Stufen einführt.

Verbindungen der Formel I, worin Y einen Rest der Formel IIIc oder IIId darstellt, kann man auch dadurch gewinnen, das man eine Verbindung der Formel

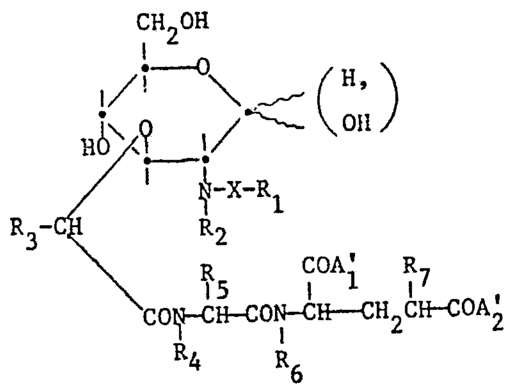

(XIV)

worin einer der Reste $A_1'$ und $A_2'$ einen Reste der Formel

$$—T—Y_1—M_1 \quad (XV)$$

und der andere der Reste $A_1'$ und $A_2'$ veräthertes Hydroxy oder Amino, Niederalkylamino der Aminocarbonylniederalkymamino ist, und einer Verbindung der Formel

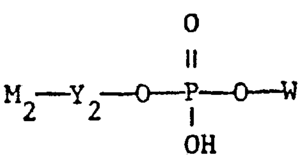

(XVI)

worin X, $R_1$, $R_2$, $R_3$, $R_4$, $R_5$, $R_6$, T, $Y_1$, $Y_2$ und W die obengenannten Bedeutungen haben und darin vorhandene Hydroxygruppen gegebenenfalls durch leicht abspaltbare Schutzgruppen geschützt sind und einer der Reste $M_1$ und $M_2$ eine freie Aminogruppe oder ein aktiviertes Derivat davon und der andere eine Carbonsäuregruppe oder ein aktiviertes Derivat davon bedeutet, kondensiert und gegebenenfalls vorhandene Schutzgruppen abspaltet.

Leicht abspaltbare Schutzgruppen sind solche die aus der Peptid- bzw. Zuckerchemie bekannt sind. Für Carboxygruppen sollen insbesondere tertiär-Butyl, Benzyl, gegebenenfalls durch Halogen oder Niederalkoxy, wie Methoxy, substituiertes Triphenylmethyl oder Benzhydryl und für Hydroxygruppen insbesondere Acylreste, z. B. Niederalkanoylreste wie Acetyl, Aroylreste, wie Benzoyl, und vor allem von der Kohlensäure sich ableitende Rest wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl, oder Tetrahydropyranyl oder gegebenenfalls substituierte Alkylidenreste, die die Sauerstoffatome in 4- und 6-Stellung des Glucoseteils verbinden, genannt werden. Solche Alkylidenreste sind insbesondere ein Niederalkyliden-, in erster Linie der Aethyliden-, Isopropyliden- oder Propylidenrest oder auch ein gegebenenfalls substituierter, vorzugsweise in p-Stellung substituierter Benzylidenrest.

Diese Schutzgruppen können in an sich bekannter Weise abgespalten werden. So kann man sie durch saure Hydrolyse, Benzyl- oder Benzylidenreste auch hydrogenolytisch, z. B. mit Wasserstoff in Gegenwart eines Edelmetall-, wie Palladium- oder Platinkatalysators, entfernen.

Die verwendeten Ausgangsstoffe sind bekannt oder lassen sich in an sich bekannter Weise herstellen.

Die Kondensation erfolgt dabei z. B. in der Weise, dass man die Verbindung (XIV) in Form der aktivierten Carbonsäure mit der Aminoverbindung (XVI) umsetzt oder dass man die Säure (XIV) mit der Verbindung (XVI), deren Aminogruppe in aktivierter Form vorliegt, umsetzt. Die aktivierte Carboxylgruppe kann beispielsweise ein Säureanhydrid, vorzugsweise ein gemischtes Säureanhydrid wie z. B. mit Kohlensäureniederalkylester, wie Kohlensäureäthyl oder Isobutylester, ein Säureazid, ein Säureamid, wie ein Imidiazolid, Isoxazolid, oder ein aktivierter Ester sein. Als aktivierte Ester seien insbesondere genannt: Cyanmethylester, Carboxymethylester, p-Nitrophenylthioester, p-Nitrophenylester, 2,4,5-Trichlorphenylester, Pentachlorphenylester, N-Hydroxy-succinimidester, N-Hydroxy-phthalimidester, 8-Hydroxy-chinolinester, 2-Hydroxy-1,2-dihydro-1-carboäthoxy-chinolinester, N-Hydroxy-piperidinester oder Enolester, die mit N-Aethyl-5-phenylisoxazolium-3'-sulfonat gewonnen werden. Aktivierte Ester können auch gegebenenfalls mit einem carbodiimid unter Zusatz von N-Hydroxysuccinimid oder einem unsubstituierten oder z. B. durch Halogen, Methyl oder Methoxy substituierten 1-Hydroxybenzotriazol oder 3-Hydroxy-4-oxo-3,4-dihydro-benzo[d]-1,2,3-triazin erhalten werden.

Die Aminogruppe ist besipielsweise durch Reaktion mit einem Phosphit aktiviert.

Unter den Methoden der Reaktion mit aktivierten Säuren sind insbesondere diejenigen mit N-Aethyl-5-phenyl-isoxazolium-3'-sulfonat (Woodward Reagens K) oder 2-Aethoxy-1,2-dihydro-1-carboäthoxy-chinolin oder Carbodiimid zu erwähnen.

Verbindungen der Formel I, worin Y einen Rest der Formel IIIa oder IIIb darstellt, lassen sich auch erhalten, wenn man eine Verbindung der Formel

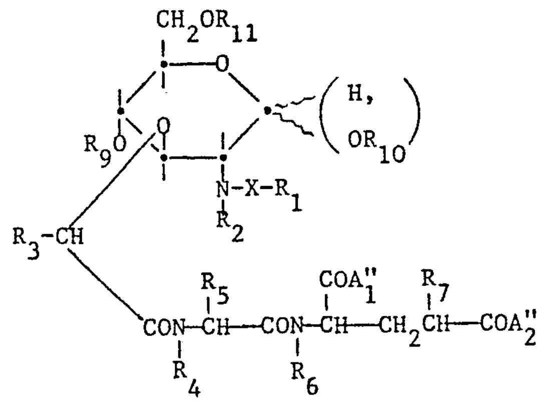

(XVII)

worin eriner der Reste $A_1''$ und $A_2''$ einen Reste der Formel

$$—T—Y_1—M_3 \quad (XVIII)$$

darstellt, mit einer Verbindung der Formel

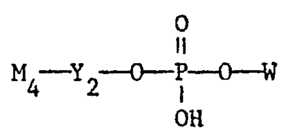

(XIX)

worin X, $R_1$, $R_2$, $R_3$, $R_4$, $R_5$, $R_6$, $R_7$, T, $Y_1$, $Y_2$ und W die obengenannte Bedeutung haben und gegebenenfalls darin vorhandene Hydroxygruppen durch leicht abspaltbare Schutzgruppen geschützt sind, $R_9$, $R_{10}$ und $R_{11}$ leicht abspaltbare Schutzgruppen darstellen, und der andere der Rest $A_1''$ und $A_2''$ veräthertes Hydroxy, Amino, Niederalkylamino oder Aminocarbonylniederalkylamino ist, und einer der Reste $M_3$ und $M_4$ eine freie Hydroxygruppe und der andere eine freie Carboxylgruppe darstellt, wobei gegebenenfalls einer der beiden Reste $M_3$ und $M_4$ in reaktionsfähiger Form vorliegt, in an sich bekannter Weise verestert und gegebenenfalls vorhandene Schutzgruppen abspaltet.

Diese Reaktion lässt sich so durchführen, dass man die freie Säure mit Alkohol in Gegenwart eines wasserabspaltenden Mittels, wie einem Carbodiimid, z. B. Dicyclohexylcarbodiimid, und einem Amin, wie Pyridin, Dimethylaminopyridin, einem Trialkylamin, z. B. Trimethylamin, verestert. Man kann aber auch die Carbonsäure, z. B. in Form eines Salzes, mit einem reaktionsfähigen Ester des Alkohols, z. B. einem Ester mit einer starken anorganischen oder organischen Säure, wie einer Halogenwasserstoffsäure, z. B. Chlor-, Brom- oder Jodwasserstoffsäure, oder eine organischen Sulfonsäure, wie p-Toluolsulfonsäure oder Methan- oder Aethansulfonsäure, umsetzen.

Ferner ist es auch möglich den Alkohol, gegebenenfalls als Salz, z. B. Natrium- oder Kaliumsalz, mit einer aktivierten Carbonsäuren umzusetzen. Als aktivierte Carbonsäuren sind insbesondere Anhydride, in erster Linie gemischte Säureanhydride, wie ein Säureazid oder Halogenid oder ein aktivierter Ester zu nennen, wie Cyanmethylester, Carboxymethylester, p-Nitrophenylthioester, p-Nitrophenylester, 2,4,5-Trichlorphenylester, Pentachlorphenylester, N-Hydroxysuccinimidester, N-Hydroxyphthalimidester, 8-Hydroxychinolinester, 2-Hydroxy-1,2-dihydro-1-carboäthoxy-chinolin-ester, N-Hydroxypiperdinester oder Enolester, die mit N-Aethyl-5-phenyl-isoxazolium-3'-sulfonat gewonnen werden. Aktivierte Ester können auch gegebenenfalls mit einem unsubstituirten oder z. B. durch Halogen, Methyl oder Methoxy substituierten 1-Hydroxybenzotriazol, 3-Hydroxy-4-oxo-3,4-dihydrobenzo[d]-1,2,3-triazin erhalten werden.

Leicht abspaltbare Schutzgruppen sind solche die aus der Peptid-bzw. Zuckerchemie bekannt sind.

Für Carboxygruppen sollen insbesondere tertiär-Butyl, Benzyl, gegebenenfalls durch Halogen oder Niederalkoxy, wie Methoxy, substituiertes Triphenylmethyl oder Benzhydryl und für Hydroxygruppen insbesondere Acylreste, z. B. Niederalkanoylreste, wie Acetyl, Aroylreste, wie Benzoyl, und vor allem von der Kohlensäure sich ableitende Rest, wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl oder Tetrahydropyranyl oder gegebenenfalls substituierte Alkylidenreste, die die Sauerstoffatome in 4- und 6-Stellung des Glucoseteils verbinden, genannt werden. Solche Alkylidenreste sind insbesondere ein Niederalkyliden-, in erster Linie der Aethyliden-, Isopropyliden- oder Propylidenrest, oder auch ein gegebenenfalls substituierter, vorzugsweise in p-Stellung substituierter Benzylidenrest.

Diese Schutzgruppen können in an sich bekannter Weise abgespalten werden. So kann man sie durch saure Hydrolyse, Benzyl- oder Benzylidenreste auch hydrogenolytisch, z. B. mit Wasserstoff in Gegenwart eines Edelmetall-, wie Palladium- oder Platinkatalysators, entfernen.

Die verwendeten Ausgangsstoffe sind bekannt und lassen sich in an sich bekannter Weise herstellen.

Eine weitere Verfahrensmethode zur Herstellung der neuen Verbindung der Formel I besteht darin, dass man eine Verbindung der Formel

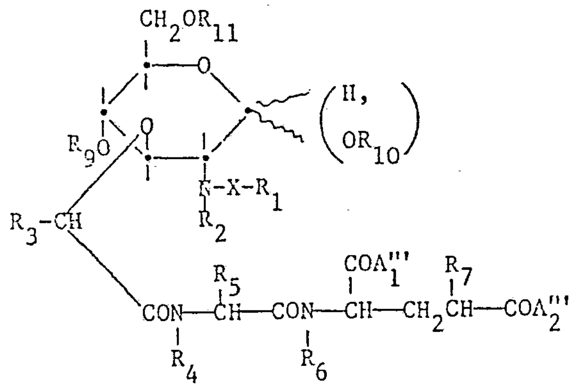

(XX)

worin X, $R_1$, $R_2$, $R_3$, $R_4$, $R_5$, $R_6$ und $R_7$ die obgenannte Bedeutung haben und gegebenenfalls darin vorhandene Hydroxygruppen mit einer leicht abspaltbaren Schutzgruppe geschützt sind, $R_9$, $R_{10}$ und $R_{11}$ leicht abspaltbare Schutzgruppen bedeuten und einer der Reste $A_1'''$ und $A_2'''$ für —T—Y—OH steht, worin Y und T die obengenannten Bedeutungen besitzen, und der andere der Reste $A_1'''$ und $A_2'''$ für freies oder veräthertes Hydroxy, Amino, Niederalkylamino oder im Niederalkylrest gegebenenfalls substituiertes Aminocarbonylniederalkylamino steht, mit einer den Rest der Formel

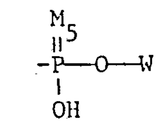

(XXI)

worin $\underset{\|}{M_5}$ ein Elektronenpaar oder Oxo bedeutet, abgebenden Verbindung umsetzt, falls $\underset{\|}{M_5}$ ein Elektronenpaar ist, mit einem schwachen Oxydationsmittel oxydiert, und vorhandene Schutzgruppen abspaltet.

Als Rest der Formel XXI abgebende Verbindungen sollen insbesondere Verbindungen der Formel

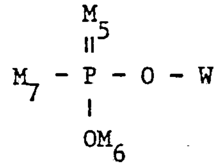

genannt werden, worin W und $\underset{\|}{M_5}$ die obgenannte Bedeutung besitzen, $M_6$ Wasserstoff oder eine leicht abspaltbare Schutzgruppe und $M_7$ gegebenenfalls in reaktionsfähiger Form vorliegendes Hydroxy ist. Falls $M_6$ Wasserstoff ist, leigen die den Rest der Formel XXI abgebenden Verbindungen überwiegend in der tautomeren Form vor, worin $M_6$ direkt an das Phosphoratom gebunden ist.

Eine leicht abspaltbare Schutzgruppe $M_6$ ist insbesondere Niederalkyl, wie Methyl oder Aethyl, Niederalkenyl, wie Aethenyl, Allyl oder 1-Methyl-propenyl, oder Benzyl.

Gegebenenfalls in reaktionsfähiger Form vorliegendes Hydroxy $M_7$ ist insbesondere freies Hydroxy oder mit einer starken anorganischen oder organischen Säure verestertes Hydroxy, wie mit einer Halogenwasserstoff-, Niederalkancarbonsäure oder Aryl oder Alkylsulfonsäure, z. B. p-Toluolsulfonsäure, Methan- oder Aethansulfonsäure, verestertes Hydroxy. Der Rest $M_7$ kann aber auch für eine Phenoxy oder Niederalkoxygruppe stehen.

Diese Reaktion wird vorzugsweise in Gegenwart eines säurebindenden Mittels, wie Pyridin, Triniederalkylamin, z. B. Triäthylamin oder Trimethylamin, eines Imidazols, oder einer anorganischen Base, wie Natrium- oder Kaliumhydroxyd, oder in Gegenwart eines Natrium oder Kaliumalkoholats durchgeführt, wobei man als Lösungsmittel ein aprotisches Lösungsmittel, wie Dimethylsulfoxyd oder Acetonitril bevorzugt.

Ist in den erhaltenen Verbindungen $M_5$ ein Elektronenpaar, oxydiert man z. B. mit einer Persäure, wie Perbenzoesäure, oder einem Alkylhydroperoxyd.

Die Abspaltung einer Schutzgruppe $M_6$ steht meist gemeinsam mit der Abspaltung der übrigen Schutzgruppen. Diese können in an sich bekannter Weise, z. B. hydrogenolytisch, z. B. mit Wasserstoff in Gegenwart eines Edelmetall- wie Palladium- oder Platinkatalysators, oder durch saure Hydrolyse, entfernt werden.

Die Ausgangsstoffe sind bekannt und lassen sich auf an sich bekannte Weise, z. B. einer der vorstehend genannten entsprechend abgewandelten Methoden, herstellen.

Im weiteren kann man die neuen Verbindungen der Formel I auch herstellen, wenn man eine Verbindung der Formel

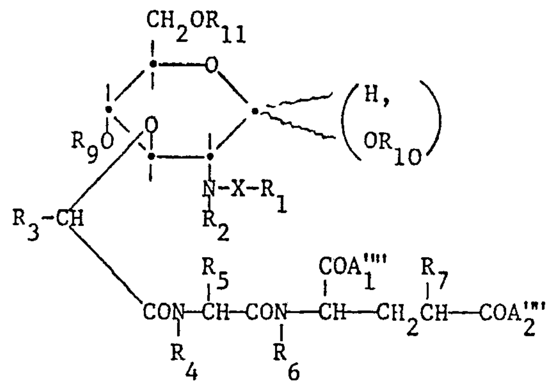

(XXIII)

worin X, $R_1$, $R_2$, $R_3$, $R_4$, $R_5$, $R_6$, $R_7$ die obgenannten Bedeutungen besitzen und gegebenenfalls darin vorhandene Hydroxygruppen mit einer leicht abspaltbaren Schutzgruppe geschützt sind, $R_9$, $R_{10}$ und $R_{11}$ leicht abspaltbare Schutzgruppen bedeuten und einer der Reste $A_1''''$ und $A_2''''$ für

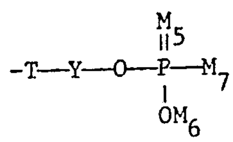

(XXIV)

und der andere für freies oder veräthertes Hydroxy, Amino, Niederalkylamino oder Aminocarbonylniederalkylamino steht, worin T, Y, $M_5$, $M_6$ und $M_7$ die obergenannte Bedeutung besitzen, mit einer Verbindung der Formel

$$HO—W \qquad (XXV)$$

worin W die obgenannte Bedeutung besitzt, umsetzt, falls $M_5$ ein Elektronenpaar ist, mit einem schwachen Oxydationsmittel oxydiert und vorhandene Schutzgruppen abspaltet.

Eine leicht abspaltbare Schutzgruppe $M_6$ ist insbesondere Niederalkyl, wie Methyl oder Aethyl, Niederalkenyl, wie Aethenyl, Allyl oder 1-Methyl-propenyl, oder Benzyl.

Gegebenenfalls in reaktionsfähiger Form vorliegendes Hydroxy $M_7$ ist insbesondere die freie Hydroxygruppe oder mit einer starken Säure verestertes Hydroxy, wie mit einer Halogenwasserstoff-, Nitroalkancarbonsäure oder Aryl- oder Alkylsulfonsäure, z. B. p-Toluolsulfonsäure, Methan- oder Aethansäure, verestertes Hydroxy. Sie kann aber auch für eine Phenoxy- oder Niederalkoxygruppe stehen.

Diese Reaktion wird vorzugsweise in Gegenwart eines säurebindenden Mittels, wie Pyridin, Triniederalkylamin, z. B. Triäthylamin oder Trimethylamin, eines Imidazols oder einer anorganischen Base, wie Natrium- oder Kaliumhydroxyd, oder eines Natrium oder Kaliumalkoholats durchgeführt, wobei man als Lösungsmittel ein aprotisches Lösungsmittel, wie Dimethylsulfoxyd oder Acetonitril bevorzugt.

Ist in den erhaltenen Verbindungen $M_5$ ein Elektronenpaar, oxydiert man z. B. mit einer Persäure, wie Perbenzoesäure oder einem Alkylhydroperoxyd.

Die Abspaltung einer Schutzgruppe $M_6$ geht meist gemeinsam mit der Abspaltung der übrigen Schutzgruppen. Diese können in an sich bekannter Weise, z. B. hydrogenolytisch, z. B. mit Wasserstoff in Gegenwart eines Edelmetall- wie Palladium- oder Platinkatalysators, oder durch saure Hydrolyse, entfernt werden.

Die Ausgangsstoffe sind bekannt und lassen sich auf an sich bekannte Weise, z. B. nach einer der vorstehend genannten entsprechend abgewandelten Methoden, herstellen.

Die oben beschriebenen Verfahren werden nach an sich bekannten Methoden durchgeführt, in Abwesenheit oder vorzugsweise in Anwesenheit von Verdünnungs- oder Lösungsmitteln, wenn notwendig unter Kühlen oder Erwärmen, unter erhöhtem Druck und/oder in einer Inertgas-, wie Stickstoffatmosphäre.

Dabei sind unter Berücksichtigung aller im Molekül befindlichen Substituenten, wenn erforderlich, insbesondere bei Anwesenheit leicht hydrolysierbarer O-Acylreste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die vorliegende Erfindung betrifft ebenfalls pharmazeutische Präparate, welche Verbindungen der Formel I enthalten. Bei den erfindungsgemässen pharmazeutischen Präparaten handelt es sich um solche zur enteralen, wie oralen, nasalen oder rektalen, sowie parenteralen Verabreichung an Warmblüter, welche den pharmakologischen Wirkstoff allein oder zusammen mit einem pharmazeutisch anwendbaren Trägermaterial enthalten. Die Dosierung des Wirkstoffes hängt von der Warmblüter-Spezies, dem Alter und dem individuellen Zustand sowie von der Applikationsweise ab.

Die neuen pharmazeutischen Präparate enthalten von etwa 10 % bis etwa 95 %, vorzugsweise von etwa 20 % bis etwa 90 % des Wirkstoffes. Erfindungsgemässe pharmazeutische Präparate können z. B. in Dosiseinheitsform, wie Dragées, Tabletten, Kapseln, Suppositorien oder Ampullen, vorliegen.

Die pharmazeutischen Präparate der vorliegenden Erfindung werden in an sich bekannter Weise, z. B. mittels konventioneller Misch-, Granulier-, Dragier-, Lösungs- oder Lyophilisierungsverfahren, hergestellt. Ausser den erwähnten Applikationsarten können auch pharmazeutische Präparate insbesondere zur oralen Anwendung erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z. B. Lactose, Saccharose, Mannit ober Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u. a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulose-präparaten, wie Acetylcellulosephthalat oder Hydroxypropylmethylcellulosephthalat, verwendet. Den Tabletten oder Dragée-Ueberzügen können Farbstoffe oder Pigmente, z. B. zur Identifizierung oder zur Kennzeichnung verschiedener Wirkstoffdosen, beigefügt werden.

Die nachfolgenden Beispiele illustrieren die oben beschriebene Erfindung ; sie sollen jedoch diese in ihrem Umfang in keiner Weise einschränken. Temperaturen werden in Celsiusgraden angegeben.

Die erfindungsgemässen Verbindungen der Formel I können weder durch einen Schmelzpunkt charakterisiert werden, noch sind spektroskopische Daten wie NMR- und IR-Spektren zur einwandfreien Charakterisierung geeignet.

Ungeeignet zur Feincharakterisierung ist ferner wegen der dominierenden Natur der Lipidteile auch die Angabe von Rf-Werten.

Da jedoch die Struktur der Ausgangsstoffe genau bekannt ist und da deren Verknüpfung eindeutig ist, ist damit auch die Sequenz der Bausteine im Endprodukt und dessen Struktur gesichert.

## Beispiel 1

Zu einer Lösung von 1.4 mMol 2-(Hexadecyloxy-hydroxyphosphoryloxy)-äthylamin und von 3 mMol Triäthylamin in 25 ml eines Gemisches aus Chloroform-Methanol-Wasser, 65 : 25 : 4, wird eine Lösung von 2 mMol N-Acetylmuramyl-L-alanyl-D-isoglutamin-N-hydroxysuccinimidester in 6,5 ml Dimethylacetamid zugetropft. Nach 18 Stunden Rühren bei 20 °C wird die Lösung bei reduziertem Druck auf ca 15 ml eingeengt ; dabei entsteht eine Emulsion. Diese wird mit 100 ml Wasser verdünnt und gefriergetrocknet. Der Rückstand wird in 25 ml Wasser suspendiert und extensiv gegen Wasser dialysiert. Das Innendialysat, das das gewünschte Produkt enthält, wird gefriergetrocknet. Das N-Acetylmuramyl-L-alanyl-D-isoglutamin-2-(hexadecyloxy-hydroxyphosphoryloxy)-äthylamid wird durch Chromatographie an einer Sephadex® LH-20 Säule gereinigt. Elutionsgemisch : Chloroform-Methanol-Essigsäure-Wasser, 25 : 15 : 4 : 2.

Die neue Verbindung wird analytisch dadurch charakterisiert, dass die Bausteine N-Acetylmuraminsäure, Hexadecanol, Phosphat, L-Alanin und D-Isoglutaminsäure quantitativ bestimmt werden :

N-Acetylmuraminsäure wird mit Hilfe der Morgan-Elson-Reaktion nach der Modifikation von J. M. Ghuyson et al. [in « Methods in Enzymology » *8*, 629 (1966)] spektrophotometrisch bestimmt.

Phosphat wird nach Lowry et al. [J. Biol. Chem. *207*, 1 (1954)] quantitativ bestimmt.

Die Aminosäuren und Hexadecanol werden in einem Totalhydrolysat (6 N HCl, 24 Std. 110 °C) mit Hilfe eines Aminosäureanalysators bzw. gaschromatographisch unter Verwendung von Norleucin bzw. Pentadecanol als interne Standards quantitativ bestimmt.

Der N-Acetylmuramyl-L-alanyl-D-isoglutamin-N-hydroxy-succinimidester, der als Ausgangsstoff verwendet wird, lässt sich z. B. wie folgt herstellen :

2 mMol N-Acetylmuramyl-L-alanyl-D-isoglutamin, 2,2 mMol N-Hydroxysuccinimid und 2,2 mMol Dicyclohexylcarbodiimid werden in 6,5 ml Dimethylacetamid gelöst und 18 Stunden bei 20 °C gerührt. Der ausgefallene Dicyclohexylharnstoff wird abgetrennt und die Lösung direkt für die Kondensation mit dem Phospholipid verwendet.

2-(Hexadecycloxy-hydroxyphosphoryloxy)-äthylamin, das als Ausgangsmaterial verwendet wird, ist ein kommerziell erhältliches synthetisches Präparat.

## Beispiel 2

In analoger Weise zu Beispiel 1, ausgehend von 2-(Hexadecyloxy-hydroxy-phosphoryloxy)-äthylamin bzw. 2-(Cholest-5-en-3β-oxy-hydroxyphosphoryloxy)-äthylamin bzw. 2-(Tetradecyloxy-hydroxyphosphoryloxy)-äthylamin und den N-Hydroxysuccinimidestern von entsprechenden Muramylpeptiden erhält man

N-Acetyl-desmethylmuramyl-L-alanyl-D-isoglutamin-2-(hexadecyloxy-hydroxyphosphoryloxy)-äthylamid,

N-Acetyl-desmethylmuramyl-L-alanyl-D-isoglutaminyl-L-alanin-2-(hexadecyloxy-hydroxyphosphoryloxy)-äthylamid,

N-Acetyl-muramyl-L-alanyl-D-isoglutaminyl-L-alanin-2-(hexadecyloxy-hydroxyphosphoryloxy)-äthylamid,

N-Acetyl-muramyl-L-alanyl-D-isoglutaminyl-oxymethylcarbonsäure-2-(hexadecyloxy-hydroxyphosphoryloxy)-äthylamid,

N-Acetyl-muramyl-L-alanyl-D-isoglutaminyl-L-alanyl-2-(tetradecyloxy-hydroxyphosphoryloxy)-äthylamid,

N-Acetyl-desmethylmuramyl-L-alanyl-D-isoglutaminyl-2-(cholest-5-en-3β-oxy-hydroxyphosphoryloxy)-äthylamid,

N-Acetyl-muramyl-L-alanyl-D-isoglutaminyl-L-alanin-2-(cholest-5-en-3β-oxy-hydroxyphosphoryloxy)-äthylamid,

N-Benzoyl-muramyl-L-alanyl-D-isoglutaminyl-L-alanin-2-(hexadecyloxy-hydroxyphosphoryloxy)-äthylamid.

## Beispiel 3

In analoger Weise zu Beispiel 1, ausgehend von 2-[(3'R)-Hydroxy-(2'S)-palmitoylamino-4't-octadecenyloxy-hydroxyphosphoryloxy]-äthylamin (2-[N-Palmitoyl-sphingosin-1-O-yl-hydroxyphosphoryloxy]-äthylamin) und N-Acetyl-muramyl-L-alanyl-D-isoglutamin-N-hydroxy-succinimidester bzw. N-Acetyl-muramyl-L-α-aminobutyryl-D-isoglutaminyl-L-alanin-N-hydroxy-succinimidester, erhält man N-Acetyl-muramyl-L-alanyl-D-isoglutamin-2-[(3'R)-hydroxy-(2'S)-palmitoylamino-4'-t-octadecenyloxy-hydroxyphosphoryloxy]-äthylamid bzw. N-Acetyl-muramyl-L-α-aminobutyryl-D-isoglutaminyl-L-alanin-2-[(3'R)-hydroxy-(2'S)-palmitoylamino-4't-octadecenyloxy-hydroxyphosphoryloxy]-äthylamid.

## Beispiel 4

In analoger Weise zu Beispiel 1, ausgehend von 2-[(3'R)-Hydroxy-(2'S)-palmitoylamino-4't-octadecenyloxy-hydroxyphosphoryloxy]-äthylamin und entsprechenden Muramylpeptid-N-hydroxysuccinimidestern, erhält man

N-Acetyl-desmethylmuramyl-L-alanyl-D-isoglutamin-2-[(3'R)-hydroxy-(2'S)-palmitoylamino-4't-octadecenyloxy-hydroxyphosphoryloxy]-äthylamid,

N-Acetyl-desmethylmuramyl-L-alanyl-D-isoglutaminyl-L-alanin-2-[(3'R)-hydroxy-(2'S)-palmitoylamino-4't-octadecenyloxy-hydroxyphosphoryloxy]-äthylamid,

N-Acetyl-muramyl-L-alanyl-D-isoglutaminyl-L-alanin-2-[(3'R)-hydroxy-(2'S)-palmitoylamino-4't-octadecenyloxy-hydroxyphosphoryloxy]-äthylamid,

N-Acetyl-muralyl-L-alanyl-D-isoglutaminyl-oxymethylcarbonsäure-2-[(3'R)-hydroxy-(2'S)-palmitoylamino-4't-octadecenyloxy-hydroxyphosphoryloxy]-äthylamid.

Beispiel 5

a) 1 mMol (350 mg) 1α-Benzyl-2-acetamido-2-desoxy-4,6-isopropylidenglucose lässt man in 10 ml absol. Dimethoxyäthan mit 1 mMol Natriumhydrid-Mineralöl-Dispersion bis zur beedeten $H_2$-Entwicklung reagieren. Dann kühlt man auf 0° und gibt unter gutem Rühren und Feuchtigkeitsausschluss 1 mMol Chloracetyl-L-alanyl-D-isoglutamin-2-(hexadecyloxy-hydroxyphosphoryloxy)-äthylamid als Pyridinsalz in 5 ml Dimethoxyäthan zu und lässt das Reaktionsgemisch auf Raumtemperatur erwärmen. Nach 3 Stunden bei Raumtemperatur dampft man das Gemisch im Vakuum zur Trockne und chromatographiert über Kieselgel Merck in Chloroform : Methanol = 7 : 3. Die Fraktionen, die das gesuchte Endprodukt enthalten, reagieren auf der Dünnschichtplatte (Kieselgel, Merck) positiv mit Phosphatreagenz nach V. E. Vaskovsky und E. Y. Kostetsky, J. Lipid. Res. 9, 396 (1968) und positiv mit 2N Schwefelsäure in der Hitze (Braunfärbung des Zuckers). Sie werden eingedampft und zur Entfernung der Schutzgruppen zuerst in einer Mischung aus 12 ml Eisessig und 8 ml Wasser 1 Stunde bei 50° gehalten und dann bei Raumtemperatur mit 10 proz. Pd/C unter Normaldruck hydriert. Nach 20 Stunden ist die α-Benzylgruppe entfernt ; man filtriert vom Katalysator ab und dampft das Filtrat im Vakuum zu einem Sirup ein. Man erhält so das sirupöse Pyridiniumsalz des N-Acetyl-normuramyl-L-alanyl-D-isoglutamin-2-(hexadecyloxy-hydroxyphosphoryloxy)-äthylamids, das durch Dialyse gegen 10 proz. NaCl-Lösung, danach gegen destilliertes Wasser in das Halbnatrimsalz überführt wird. Die Substanz wird charakterisiert durch Aminosäureanalyse, Bestimmung des Verhältnisses P : Na und die Bestimmung der Muraminsäure nach Morgan-Elson wie beschrieben im Beispiel 1.

$R_F = 0.25$ ; Laufmittel : $CHCl_3$ : Methanol : Wasser = 65 : 25 : 4 (V/V) auf Dünnschichtplatten, Kieselgel Merck.

b) Die als Ausgangsmaterial verwendete α-Benzyl-2-acetamido-2-desoxy-4,6-isopropyliden-glucose hat folgende physikalischen Eigenschaften : Smp. 136-137°, $[\alpha]_D^{20} = +110°$ ($CHCl_3$, c = 1), $R_F = 0,55$ ($CH_2Cl_2$ : Methanol = 5 : 1 auf Dünnschichtplatten Kieselgel, Merck).

Das Pyridiniumsalz des Chloracetyl-L-alanyl-D-isoglutamin-2-(hexadecyloxy-hydroxyphosphoryloxy)-äthylamids erhält man durch Reaktion von 2 mMol Chloracetyl-L-alanyl-D-isoglutamin-2-hydroxy-äthylamid in Pyridin mit 1,5 mMol Phosphorsäure-hexadecylester und 4,5 mMol Triisopropylbenzolsulfonsäurechlorid bei Raumtemperatur. Nach 15 Stunden gibt man 2 ml Wasser zu, lässt 1 Stunde bei Raumtemperatur stehen, dampft im Vakuum zur Trockne und dialysiert den Rückstand gegen destilliertes Wasser. Im Dialysierschlauch bleibt der gesuchte Phosphorsäurediester. Man dampft den Schlauchinhalt zum Sirup ein und dampft zur azeotropen Entfernung des Wassers 4mal mit Pyridin im Vakuum nach. Vor der weiteren Reaktion kann in Dimethoxyäthan mit Molekularsieb restliches Wasser entfernt werden. $R_F = 0.35$ ($CHCl_3$ : Methanol : $H_2O$ = 65 : 25 : 4, Dünnschichtplatten Kieselgel, Merck).

Beispiel 6

a) Zu 410 mg (1 mMol) 1α-Benzyl-N-acetyl-4,6-isopropylidennormuraminsäure, 0.9 mMol L-Alanyl-D-isoglutamin-2-(hexadecyloxy-hydroxyphosphoryloxy)-äthylamid in einer Mischung aus 15 ml Dimethylformamid, 10 ml Tetrahydrofuran und 2 ml Pyridin gibt man 1.2 mMol Dicyclohexylcarbodiimid und 1.3 mMol N-Hydroxysuccinimid. Nach 24 Stunden bei Raumtemperatur ist die Reaktion beendet. Man gibt einige Tropfen Wasser zu, saugt vom gebildeten Dicyclohexylharnstoff ab und dampft das Filtrat im Vakuum zur Trockne. Den Rückstand reinigt man durch Chromatographie an Kieselgel Merck in Chloroform/Methanol = 7 : 3 (s. Beispiel 5). Die das Endprodukt enthaltenden Fraktionen werden analog Beispiel 5 aufgearbeitet, von Schutzgruppen befreit und anschliessend wie beschrieben dialysert. Man erhält so das Halbnatriumsalz des N-Acetyl-normuramyl-L-alanyl-D-isoglutamin-2-(hexadecyloxy-hydroxyphosphoryloxy)-äthylamids, das in 1 proz. wässriger Lösung den pH-Wert 6.5 zeigt.

b) Das als Ausgangsmaterial eingesetzte L-Alanyl-D-isoglutamin-2-(hexadecyloxy-hydroxyphosphoryloxy)-äthylamid erhält man analog Beispiel 5b durch Kondensation von tertiär-Butoxycarbonyl-L-alanyl-D-isoglutamin-2-hydroxy-äthylamid mit Phosphorsäure-hexadecylester in Pyridin durch Reaktion mit 3 Aequivalenten Triisopropylbenzol-sulfonsäurechlorid und nachfolgende Abspaltung der tertiär-Butoxycarboylgruppe mit 20 proz. Trifluoressigsäure in Methylenchlorid bei Raumtemperatur. Danach dampft man im Vakuum zur Trockne und dialysiert den Rückstand gegen Phosphatpuffer pH = 7, dann gegen destilliertes Wasser. Das Aethylamid bleibt im Dialyseschlauch und wird durch Gefriertrocknen des Innendialysats erhalten. Eine Extraktion des Lyophilisats mit Essigester entfernt Reste von Salzen der Triisopropylbenzosulfonsäure.

1α-Benzyl-4,6-isopropyliden-N-acetyl-desmethylmuraminsäure erhält man in einer für die Kupplung geeigneten Form aus dem entsprechenden Methylester : Smp. 122-125° $[\alpha]_D^{20}$ = + 150° ($CHCl_3$, c = 1) $R_F$ = 0,53 ($CH_2Cl_2$ : Methanol = 15 : 1, Dünnschichtplatten, Kieselgel Merck) durch Verseifen mit KOH/Methanol bei Raumtemperatur und anschliessende Einstellung des pH-Wertes mit 1N Salzsäure auf pH = 6 mit einem pH-Meter.

## Beispiel 7

a) Analog Beispiel 6 kondensiert man 1 mMol 1α-Benzyl-N-acetyl-4,6-isopropyliden-normuramyl-L-alanin mit 1 mMol D-isoglutamin-2-(hexadecyloxy-hydroxyphosphoryloxy)-äthylamid in DMF/Tetrahydrofuren mit Dicyclohexylcarbodiimid und Hydroxysuccinimid. Nach analoger Aufarbeitung und Abspaltung der Schutzgruppen erhält man das Pyridiniumsalz des N-Acetyl-normuramyl-L-alanyl-D-isoglutamin-2-(hexadecyloxy-hydroxyphosphoryloxy)-äthylamids, das nach Analyse und $R_F$-Wert identisch ist mit der nach den Beispielen 5 und 6 erhaltenen Substanz. Durch Dialyse gegen Natriumchloridlösung bei pH = 7 kann das Pyridiniumsalz leicht in das Halb-Natriumsalz überführt werden.

b) Das als Ausgangsmaterial eingesetzte D-Isoglutamin-2-(hexadecyloxy-hydroxyphosphoryloxy)-äthylamid erhält man analog Beispiel 5b durch Kondensation von Phosphorsäure-hexadecylester mit tert.-Butoxycarbonyl-D-isoglutamin-2-hydroxy-äthylamid in Pyridin mit 3 Aequivalenten Triisopropylbenzol-sulfonsäurechlorid und nachfolgende Abspaltung der BOC-Gruppe mit 20 prozentiger Trifluoressigsäure in Methylenchlorid bei Raumtemperatur (4 Stunden). Dialyse gegen Wasser liefert das reine Hydroxyphosphoryloxy-äthylamid als inneres Salz.

1α-Benzyl-N-acetyl-4,6-isopropylidennormuramyl-L-alanin erhält man durch katalytische Hydrierung mit 5 proz. Pd/C in Tetrahydrofuran während 30 Minuten von 1α-Benzyl-N-acetyl-4,6-isopropyliden-normuramyl-L-alanin-benzylester mit den Konstanten : $[\alpha]_D^{20}$ = + 73° ($CHCl_3$, c = 1), $R_f$ = 0.25 (Essigester, Dünnschichtplatten Kieselgel, Merck).

## Beispiel 8

a) 1 mMol 1α-Benzyl-N-acetyl-4,6-isopropyliden-normuramyl-L-alanyl-D-isoglutamin verestert man mit 0.8 mMol 2-(Hexadecyloxy-hydroxyphosphoryloxy)-äthanol in Pyridin mit 1.2 mMol Dicyclohexylcarbodiimid, 1.2 mMol N-Hydroxysuccinimid und 0.1 mMol 4-Dimethylaminopyridin (Methode nach Steglich, siehe B. Neises und W. Steglich, Angew. Chem. *90*, 556 (1978) bei Raumtemperatur. Nach 24 Stunden bei Raumtemperatur gibt man einige Tropfen Wasser zu und saugt vom gebildeten Dicyclohexylharnstoff ab. Das Filtrat dampft man im Vakuum zur Trockne, nimmt mit 20 ml 80 proz. Essigsäure auf und spaltet die Isopropylidengruppe während 1 Stunde bei 50° ab. Danach hydriert man die Lösung analog Beispiel 5 und dialysiert die vom Katalysator befreite und mit 50 ml Wasser verdünnte Lösung gegen gepufferte NaCl-Lösung bei pH = 7, dann gegen destilliertes Wasser. Man erhält so das Halbnatriumsalz des N-Acetyl-normuramyl-L-alanyl-D-isoglutamin-2-(hexadecyloxy-hydroxyphosphoryloxy)-äthylesters mit $R_F$ = 0.25 ($CHCl_3$/Methanol/$H_2O$ = 65 : 25 : 4, Dünnschichtplatten, Kieselgel Merck).

b) Das als Ausgangsmaterial verwendete 2-(Hexadecyloxy-hydroxyphosphoryloxy)-äthanol wird nach an sich bekannten Methoden aus Phosphoroxychlorid und Hexadecanol in Tetrahydrofuran, nachfolgende Reaktion mit Aethylenglykol und Triäthylamin und basische Hydrolyse des erhaltenen Produkts in Tetrahydrofuran/Wasser/Natronlauge bei Raumtemperatur gewonnen vgl. H. Eibl und A. Nicksch, Deutsche Offenlegungsschrift Nr. 2 345 059 und P. Chabrier et al., C. R. Acad. Sci., Paris, Serie C *283* 229 (1976).

## Beispiel 9

a) 1 mMol Pyridinsalz des 1,2-[2-Phenyl-$\Delta^2$-oxazolin (4,5)]-5,6-isopropyliden-D-glucofuranosyl-3-O-methylcarbonyl-L-alanyl-D-isoglutamin-2-(hexadecyloxy-hydroxyphosphoryloxy)-äthylamids lässt man in einer Mischung aus 10 ml Methylenchlorid und 10 ml Trifluoressigsäure 20 Stunden bei Raumtemperatur stehen. Nach Eindampfen im Vakuum zur Trockne, Dialyse des Rückstandes gegen gepufferte NaCl-Lösung bei pH = 7, danach gegen destill. Wasser und Gefriertrocken des Schlauchinhalts erhält man N-Benzoyl-normuramyl-L-alanyl-D-isoglutamin-2-(hexadecyloxy-hydroxyphosphoryloxy)-äthylamid als Halb-Natriumsalz mit $R_F$ = 0.27 ($CHCl_3$ : Methanol : $H_2O$ = 65 : 25 : 4, Dünnschichtplatten, Kieselgel Merck).

b) Das Ausgangsmaterial erhält man wie folgt :

Analog Beispiel 6 kondensiert man 1 mMol 2-Phenyl-4,5-[3-carboxymethyl-5,6-isopropyliden-D-glucofurano]-$\Delta^2$-oxazolin mit 0.8 mMol L-Alanyl-D-isoglutamin-2-(hexadecyloxy-hydroxyphosphoryloxy)-äthylamid mit Hilfe von 1.2 mMol Dicyclohexylcarbodiimid und 1.3 mMol N-Hydroxysuccinimid in einer Mischung aus 10 ml Dimethylformamid und 10 ml Tetrahydrofuran. Nach 24 Stunden Rühren bei Raumtemperatur gibt man einige Tropfen Wasser zu, saugt vom ausgefallenen Dicyclohexylharnstoff ab

und dampft im Vakuum zur Trockne. Durch Chromatographie über Kieselgel Merck in Chloroform/Methanol = 7 : 3 erhält man das sirupöse 1,2-[2-Phenyl-$\Delta^2$-oxazolin (4,5)]-5,6-isopropyliden-D-glucofuranosyl-3-O-methylcarbonyl-L-alanyl-D-isoglutamin-2-(hexadecyloxy-hydroxyphosphoryloxy)-äthylamid als inneres Salz mit $R_F = 0,40$ ($CHCl_3$ : Methanol : $H_2O$ = 65 : 25 : 4, Dünnschichtplatten, Kieselgel Merck).

Beispiel 10

a) Analog Beispiel 8 verestert man 1 mMol 1α-Benzyl-N-acetyl-4,6-isopropyliden-desmethylmuramyl-L-alanyl-D-isoglutamin-2-hydroxy-äthylamid mit 0,8 mMol Hexadecyloxy-hydroxyphosphoryloxy-essigsäure nach der Methode von Steglich et al. [B. Neises und W. Steglich, Angew. Chem. *90*, 556 (1978)]. Nach 20 Stunden bei Raumtemperatur gibt man einige Tropfen Wasser zu, saugt vom gebildeten Dicyclohexylharnstoff ab und dampf das Filtrat im Vakuum zur Trockne ein. Durch Chromatographie an Kieselgel Merck in Chloroform/Methanol = 7 : 3 erhält man das Muramylpeptid-Phospholipid-Konjugat, das analog Beispiel 5 von Schutzgruppen befreit und durch Dialyse gereinigt wird. Man erhält das N-Acetyl-normuramyl-L-alanyl-D-isoglutamin-2-(hexadecyloxy-hydroxyphosphoryloxy-methylcarbonyloxy)-äthylamid als Halb-Natriumsalz, $R_F = 0.27$ ($CHCl_3$/Methanol/$H_2O$ = 65 : 25 : 4, Dünnschichtplatten Kieselgel, Merck).

b) Das als Ausgangsmaterial verwendete 1α-Benzyl-N-acetyl-4,6-isopropyliden-normuramyl-L-alanyl-D-isoglutamin-2-hydroxy-äthylamid erhält man analog Beispiel 6 durch Kondensation von 1α-Benzyl-N-acetyl-4,6-isopropyliden-normuramyl-L-alanyl-D-isoglutamin mit 2-Amino-äthanol als farblose amorphe Substanz mit $[\alpha]_D^{20} = +85^\circ$ ($CHCl_3$, c = 1), $R_F = 0.38$ ($CHCl_3$/Methanol/$H_2O$ = 70 : 30 : 5, Dünnschichtplatten Kieselgel, Merck).

Das Edukt, 1α-Benzyl-N-acetyl-4,6-isopropyliden-normuramyl-L-alanyl-D-isoglutamin zeigt in diesem Laufmittel $R_F = 0.34$. Die entsprechenden $R_F$-Werte im System Essigester/n-Butanol/Pyridin/Essigsäure/Wasser = 42 : 21 : 21 : 0,6 : 10 betragen für das Edukt 0.50 und für das Hydroxyäthylamid 0.64.

Beispiel 11

a) Das in Beispiel 10b beschriebene 1α-Benzyl-N-acetyl-4,6-isopropyliden-normuramyl-L-alanyl-D-isoglutamin-2-hydroxy-äthalamid kondensiert man mit Phosphorsäure-hexadecylester in Pyridin nach der in Beispiel 5b beschriebenen Methode. Man erhält das Pyridinsalz des entsprechenden Phosphorsäurediesters, das analog Beispiel 5 gereinigt, von Schutzgruppen befreit und dialysiert wird. Nach Gefriertrocknen des Innendialysats erhält man das Endprodukt N-Acetyl-normuramyl-L-alanyl-D-isoglutamin-2-(hexadecyloxy-hydroxyphosphoryloxy)-äthylamid, das 0.5 Aequivalente $Na^+$-Ionen enthält.

b) 1 mMol Pyridiniumsalz des 1α-Benzyl-N-acetyl-4,6-isopropyliden-normuramyl-L-alanyl-D-isoglutamin-2-[dihydroxyphosphoryloxy-äthyl]-amids kondensiert man nach der in Beispiel 5b beschriebenen Methode mit 2 mMol Hexadecanol in Pyridin. Nach Aufarbeitung analog Beispiel 5a, Abspaltung der Schutzgruppen und Dialyse erhält man nach Gefriertrocknen des Innendialysats das Endprodukt.

Beispiel 12

In analoger Weise wie im Beispiel 1 beschrieben werden die folgenden Verbindungen erhalten :

N-Acetyl-muramyl-L-valyl-D-isoglutamin-2-(tetradecyloxy-hydroxyphosphoryloxy)-äthylamid,
N-Acetyl-muramyl-L-valyl-D-isoglutaminyl-L-alanin-2-(cholest-5-en-3β-oxy-hydroxyphosphoryloxy)-äthylamid,
N-Propionyl-desmethylmuramyl-L-alanyl-D-isoglutamin-2-[(3′R)-hydroxy-(2′S)-palmitoylamino-4′t-octadecenyloxy-hydroxyphosphoryloxy]-äthylamid,
N-Propionyl-desmethylmuramyl-L-alanyl-D-isoglutaminyl-L-alanin-2-(tetradecyloxy-hydroxyphosphoryloxy)-äthylamid,
N-Acetyl-muramyl-L-propyl-D-isoglutamin-2-(cholest-5-en-3β-oxy-hydroxyphosphoryloxy)-äthylamid,
N-Acetyl-muramyl-L-prolyl-D-isoglutaminyl-L-alanin-2-(tetradecyloxy-hydroxyphosphoryloxy)-äthylamid,
N-Benzoyl-desmethylmuramyl-L-α-aminobutyryl-D-isoglutamin-2-[(3′R)-hydroxy-(2′S)-palmitoylamino-4′t-octadecenyloxy-hydroxyphosphoryloxy]-äthylamid,
N-Benzoyl-desmethylmuramyl-L-seryl-D-isoglutamin-2-(hexadecyloxy-hydroxyphosphoryloxy)-äthylamid,
N-Acetyl-desmethylmuramyl-L-seryl-D-glutamyl-L-alanin-2-(cholest-5-en-3β-oxy-hydroxyphosphoryloxy)-äthylamid,
N-Benzoyl-muramyl-L-cysteinyl-D-glutamin-2-(tetradecyloxy-hydroxyphosphoryloxy)-äthylamid,
N-Acetyl-muramyl-L-lysyl-D-glutamin-2-(hexadecyloxy-hydroxyphosphoryloxy)-äthylamid,

N-Propionyl-desmethylmuramyl-N-methyl-alanyl-isoglutamin-2-(cholest-5-en-3β-oxy-hydroxyphosphoryloxy)-äthylamid,

N-Acetyl-muramyl-L-alanyl-N-methyl-D-isoglutamin-2-(hexadecyloxy-hydroxyphosphoryloxy)-äthylamid,

N-Acetyl-desmethylmuramyl-L-arginyl-D-isoglutamin-2-(tetradecyloxy-hydroxyphosphoryloxy)-äthylamid,

N-Acetyl-desmethylmuramyl-L-histidyl-D-isoglutamin-2-(hexadecyloxy-hydroxyphosphoryloxy)-äthylamid,

N-Butyryl-desmethylmuramyl-L-phenylalanyl-D-isoglutamin-2-[(3'R)-hydroxy-(2'S)-palmitoylamino-4't-octadecenyloxy-hydroxyphosphoryloxy]-äthylamid,

N-Butyryl-desmethylmuramyl-L-methionyl-D-glutamin-2-(cholest-5-en-3β-oxy-hydroxyphosphoryloxy)-äthylamid,

N-(n-Pentanoyl)-muramyl-L-tyrosyl-D-isoglutamin-2-(hexadecyloxy-hydroxyphosphoryloxy)-äthylamid,

N-Acetyl-N-methyl-desmethylmuramyl-L-alanyl-D-glutamyl-α-glycinamid-γ-[2-(hexadecyloxy-hydroxyphosphoryloxy)-äthyl]-amid,

N-(4-methyl-benzoyl)-muramyl-glycyl-D-glutamyl-α-glycinamid-γ-L-alanin-2-(tetradecyloxy-hydroxyphosphoryloxy)-äthylamid,

N-Aethoxycarbonyl-muramyl-O-methyl-L-threonyl-D-isoglutamin-2-(hexadecyloxy-hydroxyphosphoryloxy)-äthylamid,

N-(2-Methoxy-äthylcarbonyl)-desmethylmuramyl-phenylglycyl-(γ-N-methylcarbamoyl-γ-aminobuttersäure-2-(tetradecyloxy-hydroxyphosphoryloxy)-äthylamid,

N-(4-Methoxy-benzoyl)-desmethylmuramyl-(α-methoxycarbonyl-D-isoglutaminyl)-glycyl-L-alanyl-2-(2-chlor-heptyloxy-hydroxyphosphoryloxy)-äthylamid,

N-(4-Chlor-benzoyl)-desmethylmuramyl-sarkosyl-D-isoglutaminyl-oxymethylcarbonyl-oxymethylcarbonsäure-2-(hexadecyloxy-hydroxyphosphoryloxy)-äthylamid.

## Beispiel 13

Zu einer Lösung von 1,4 mMol 2-(Hexadecyloxy-hydroxyphosphoryloxy)-äthylamid und von 3 mMol Triäthylamin in 25 ml eines Gemisches aus Chloroform-Methanol-Wasser, 65 : 25 : 4, wird eine Lösung von 2 mMol N-Acetylmuramyl-L-alanyl-D-isoglutamin-N-hydroxysuccinimidester in 6,5 ml Dimethylacetamid zugetropft. Nach 18 Stunden Rühren bei 20 °Cwird die Lösung bei reduziertem Druck auf ca 15 ml eingeengt ; dabei entsteht eine Emulsion. Diese wird mit 100 ml Wasser verdünnt und gefriergetrocknet. Der Rückstand wird in 25 ml Wasser suspendiert und bei 4 °C in folgender Reihenfolge dialysiert : 18 Stunden gegen Wasser, 24 Stunden gegen 0,1 M Natriumphosphatpuffer-0,1 M NaCl-Lösung mit pH 7, 48 Stunden gegen Wasser. Nach der letzten Dialyse muss das Innendialysat chloridfrei sein. Das Innendialysat, das das gewünschte Produkt enthält, wird bei 10 000 g und 20 °C 30 Minuten zentrifugiert, der Ueberstand wird gefriergetrocknet. Das isolierte Produkt ist chromatographisch reines N-Acetylmuramyl-L-alanyl-D-isoglutamin-2-(hexadecyloxy-hydroxyphosphoryloxy)-äthylamid, das zu 0,54 Moläquivalenten als $Na^+$-Salz vorliegt. Die Verbindung zeigt im Dünnschichtchromatogramm auf Silicagel folgende Rf-Werte : 0,24 (in Chloroform-Methanol-Wasser, 65 : 25 : 4) bzw. 0,58 (in Chloroform-Methanol-Essigsäure-Wasser, 25 : 15 : 4 : 2).

Die neue Verbindung wird analytisch dadurch charakterisiert, dass die Bausteine N-Acetylmuraminsäure, Hexadecanol, Phosphat, $Na^+$, L-Alanin und D-Glutaminsäure quantitativ bestimmt werden :

N-Acetylmuraminsäure wird mit Hilfe der Morgan-Elson-Reaktion nach der Modifikation von J. M. Ghuyson et al. [in « Methods in Enzymology » *8*, 629 (1966)] spektrophotometrish bestimmt.

Phosphat wird nach Lowry et al. [J. Biol. Chem. *207*, 1 (1954)] quantitativ bestimmt.

Die Aminosäuren und Hexadecanol werden in einem Totalhydrolysat (6 N HCl, 24 Std. 110 °C) mit Hilfe eines Aminosäureanalysators bzw. gaschromatographisch unter Verwendung von Norleucin bzw. Pentadecanol als interne Standards quantitativ bestimmt.

Die gefundenen molaren Verhältnisse bezogen auf Phosphat sind wie folgt :

$PO_4^{'''}$ : N-Acetylmuraminsäure : L-Alanin : D-Glutaminsäure : Hexadecanol : $Na^+$ = 1 : 0.93 : 0.94 : 0.91 : 1.1 : 0.94.

## Beispiel 14

In analoger Weise zu Beispiel 13 erhält man N-Acetyl-muramyl-L-alanyl-D-isoglutamin-2-[(3'R)-hydroxy-(2'S)-palmitoylamino-octadecyloxy-hydroxyphosphoryloxy]-äthylamid, N-Acetyl-muramyl-L-alanyl-D-isoglutaminyl-L-alanin-2-[(3'R)-hydroxy-(2'S)-palmitoylamino-octadecyloxy-hydroxyphosphoryloxy]-äthylamid und N-Acetyl-muramyl-L-α-aminobutyryl-D-isoglutaminyl-L-alanin-2-[(3'R)-hydroxy-(2'S)-palmitoylamino-octadecyloxy-hydroxyphosphoryloxy]-äthylamid.

Beispiel 15

Herstellung von 1 000 Kapseln mit 260 mg der aktiven Ingredienzen pro Kapsel :

| | |
|---|---|
| Zusammensetzung : | |
| Rifampicin | 250 g |
| N-Acetyl-muramyl-L-alanyl-D-isoglutamin-2-[(3'R)-hydroxy-(2'S)-palmitoylamino-octadecyloxy-hydroxyphosphoryloxy]-äthylamid | 10 g |
| Talk | 36 g |
| Weizenstärke | 24 g |
| Magnesiumstearat | 16 g |
| Laktose | 4 g |
| | 340 g |

Zubereitung : Die pulverfömigen Substanzen werden durch ein Sieb mit einer Maschenweite von 0,6 mm geschlagen und gründlich gemischt. Mit je 340 g dieser Mischung werden mit einer Kapselfüllmaschine Gelatine-Kapseln bereitet.

Beispiel 16

Herstellung von 1 000 Kapseln enthaltend 10 mg der aktiven Stoffe pro Kapsel :

| | |
|---|---|
| Zusammensetzung : | |
| Rifampicin | 100 g |
| N-Acetyl-desmethylmuramyl-L-alanyl-D-isoglutamin-2-(hexadecyloxy-hydroxy-phosphoryloxy)-äthylamid | 5 g |
| Aethyl-Cellulose | 3 g |
| Stearinsäure | 3 g |
| | 111 g |

Zubereitung : Die Aethyl-Cellulose und die Stearinsäure werden in 120 ml Methylenchlorid gelöst, mit dem Antibiotikum versetzt, und die Masse wird durch ein Sieb von 0,6 MM Maschenweite bei einer Temperatur von ca. 40° geschlagen, wobei das Methylenchlorid verdampft. 156 mg des erhaltenen Granulates werden in Gelatine-Kapseln zu 0,5 ml mit Hilfe einer Kapsel-Abfüllmaschine abgefüllt.

Beispiel 17

Herstellung eines Futtermittels enthaltend 0,005 % der aktiven Stoffe :

| | |
|---|---|
| Vor-Mischung : | |
| Rifampicin oder Chlortetracyclin | 30 g |
| N-Acetyl-desmethylmuramyl-L-alanyl-D-isoglutaminyl-L-alanin-2-(hexadecyloxy-hydroxyphosphoryloxy)-äthylamid | 10 g |
| Staubzucker | 50 g |
| Sojabohnen Futter (mit Lösungsmitteln extrahiert) | 275 g |
| | 365 g |

| | |
|---|---|
| Zusatzstoffe : | |
| Maismehl | 500,0 kg |
| Sojabohnenmehl, 44 % Protein | 300,0 kg |
| Alfalfamehl | 13,5 kg |
| Dicalciumphosphat | 18,0 kg |
| Calciumcarbonat (gemahlen) | 4,5 kg |
| Salz | 2,3 kg |
| Fischmehl, 60 % Protein | 18,0 kg |
| Stab. Fett | 27,0 kg |
| trockener Molkerückstand | 18,0 kg |
| Mangansulfat | 0,2 kg |
| Zinkoxid | 1,3 kg |
| d,1-Methionin | 0,7 kg |
| Vitamin-Vormischung | 4,5 kg |
| | 908,0 kg |

Die Vitamin-Vormischung enthält in 4,5 kg : 16 000 000 I.E. Vit. A, 1 000 000 I.E. Vit. $D_3$, 5 000 I.E. Vit. E Acetat, 6 g Vit. $K_3$, 6 mg Vit. $B_{12}$, 3 g Riboflavin, 30 g Niacin, 5 g Calcium Pantothenat und 100 g Ethoxyquin (1,2-Dihydro-6-äthoxy-2,2,4-trimethyl-chinolin) und Maismehl bis auf 4,5 kg.

Herstellungsweise : Die Wirkstoffe und Zucker werden gründlich miteinander gemischt, durch ein Sieb von 0,6 mm Maschenweite geschlagen und dann mit dem Sojabohnenmehl vermischt. Die Vormischung wird dann in der der gewünschten Endkonzentration entsprechenden Menge zum Futtermittel zugegeben und in einem horizontalen Trommelmischer homogenisiert.

Beispiel 18

In analoger Weise wie in den Beispielen 15 und 16 beschrieben erhält man Kombinationspräparate, die neben den Hilfs- und Trägerstoffen die folgenden aktiven Ingredienzien in den angegebenen Mengen pro Kapsel enthalten :

a) 500 mg Cephalexin und 5 mg N-Acetyl-muramyl-L-alanyl-D-isoglutaminyl-oxymethylcarbonsäure-2-(hexadecyloxy-hydroxyphosphoryloxy)-äthylamid,

b) 750 mg Ampicillin und 40 mg N-Acetyl-desmethylmuramyl-L-alanyl-D-isoglutamin-2-(cholest-5-en-3β-oxy-hydroxyphosphoryloxy)-äthylamid,

c) 100 mg Doxycyclin und 15 mg N-Acetyl-muramyl-L-valyl-D-isoglutamin-2-(tetradecyloxy-hydroxyphosphoryloxy)-äthylamid,

d) 300 mg Methacyclin und 15 mg B-Benzoyl-desmethylmuramyl-L-seryl-D-isoglutamin-2-(hexadecyloxy-hydroxyphosphoryloxy)-äthylamid,

e) 250 mg Erythromycin-Estolat und 30 mg N-Propionyl-desmethylmuramyl-N-methyl-alanyl-isoglutamin-2-(cholest-5-en-3β-oxy-hydroxyphosphoryloxy)-äthylamid.

Beispiel 19

Herstellung einer sterilen Trockensubstanz zur Injektion (Lyophilisation)

500 mg Cefsulodin und 10 mg N-Acetyl-muramyl-L-α-aminobutyryl-D-isoglutamin-2-(tetradecyloxy-hydroxyphosphoryloxy)-äthylamid werden unter Rühren in 5 ml Wasser gelöst. Die Lösung wird sterilfiltriert und unter aseptischen Bedingungen in ein steriles Ampullenglas (Vial) abgefüllt und lyophilisiert. Die Trockensubstanz kann nach Auflösen in Wasser oder physiologischen Lösungen für die parenterale Applikation verwendet werden.

Beispiel 20

Herstellung einer sterilen Trockensubstanz zur Injektion (Pulverabfüllung)

500 mg steriles Cefsulodin und 15 mg steriles N-Propionyl-desmethylmuramyl-L-alanyl-D-isoglutamin-2-(hexadecyloxy-hydroxyphosphoryl-oxy)-äthylamid werden unter aseptischen Bedingungen homogen gemischt und in ein Ampullenglas abgefüllt. Die Trockensubstanz kann nach Auflösen in Wasser oder physiologischen Lösungen für die parenterale Applikation verwendet werden.

**Patentansprüche** (für die Vertragsstaaten : BE, CH, DE, FR, GB, IT, LI, LU, NL, SE)

1. Phosphorylmuramylpeptide der allgemeinen Formel

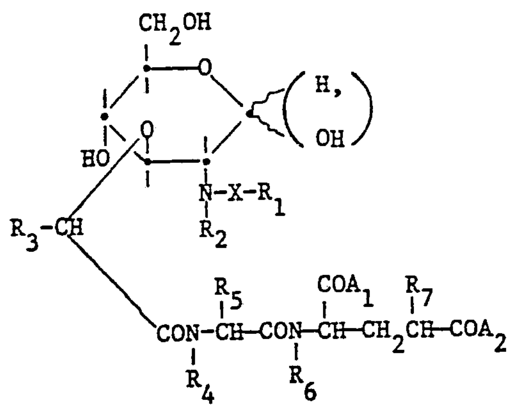

(I)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkylniederalkyl, gegebenenfalls substituiertes Aryl oder Aralkyl, stickstoffhaltiges Heterocyclyl oder Heterocyclylniederalkyl, oder $R_4$ und

$R_5$ zusammen auch Alkylen mit 3 bis 4 Kohlenstoffatomen, $R_7$ Wasserstoff oder gegebenenfalls verestertes oder amidiertes Carboxyl und einer der Reste $A_1$ und $A_2$ einen Rest der Formel

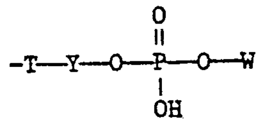

(II)

bedeutet, worin T für NH oder O steht, Y eine gegebenenfalls substituierte Alkylengruppe, die auch durch ein oder zwei Oxycarbonyl und/oder Iminocarbonyl unterbrochen sein kann, und W einen aliphatischen Rest oder einen Cycloalkyl- oder Cycloalkenylrest mit jeweils mehr als 6 Kohlenstoffatomen darstellen, und der andere der Reste $A_1$ und $A_2$ freies oder veräthertes Hydroxy, Amino, Niederalkylamino oder im Niederalkylrest gegebenenfalls substituiertes Aminocarbonylniederalkylamino ist, wobei das vorstehend verwendete Präfix « Nieder » einen Rest bis und mit 5 C-Atomen bezeichnet, und ihre Salze mit Ausnahme der in der europäischen Patentanmeldung mit der Veröffentlichungsnummer 0 025 495 offenbarten Verbindungen, nämlich von Muramylpeptiden der Formel

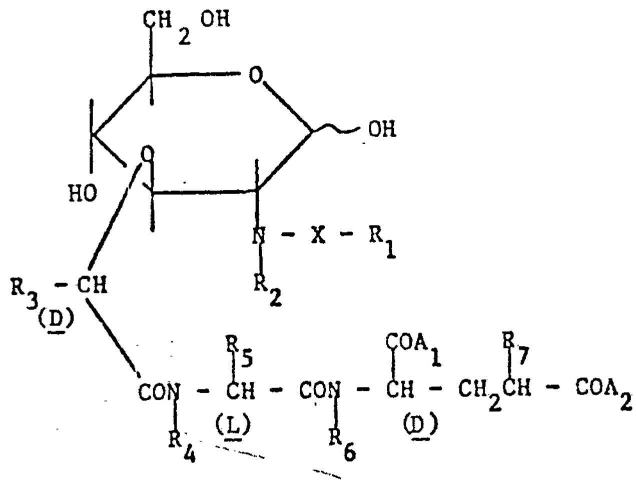

(Ia)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkyl-niederalkyl, gegebenenfalls substituiertes Aryl oder Aralkyl, stickstoffhaltiges Heterocyclyl oder Heterocyclylniederalkyl, oder $R_4$ und $R_5$ zusammen auch Alkylen mit 3-4 Kohlenstoffatomen, $R_7$ Wasserstoff oder gegebenenfalls verestertes oder amidiertes Carboxyl und einer der Reste $A_1$ und $A_2$ einen Rest der Formel

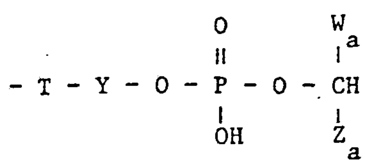

(IIa)

bedeuten, worin T für HN oder O steht, Y eine gegebenenfalls substituierte Alkylengruppe, die auch durch ein oder zwei Oxycarbonyl und/oder Iminocarbonyl unterbrochen sein kann, $W_a$ Wasserstoff und $Z_a$ eine 1,2-Dihydroxyäthyl- oder 2-Hydroxyäthylgruppe, in denen mindestens eine Hydroxygruppe mit einer gegebenenfalls ungesättigten aliphatischen Carbonsäure mit 12-90 C-Atomen verestert oder mit einem gegebenenfalls ungesättigten aliphatischen Alkohol mit 10-22 C-Atomen veräthert ist, oder $W_a$ und $Z_a$ je eine mit einer gegebenenfalls ungesättigten aliphatischen Carbonsäure mit 12-90 C-Atomen veresterte oder mit einem gegebenenfalls ungesättigten aliphatischen Alkohol mit 10-22 C-Atomen verätherte Hydroxymethylgruppe darstellen, und der andere der Reste $A_1$ und $A_2$ freies oder veräthertes Hydroxy, Amino, Niederalkylamino oder Carboxamidoniederalkyl-amino ist, und ihrer Salze, wobei das Präfix « Nieder » Reste bis und mit 7 C-Atomen bezeichnet, sowie von Muramylpeptiden der oben dargestellten Formel Ia, worin X Carbonyl oder Carbonyloxy, $R_1$ Niederalkyl mit 1-3 Kohlenstoffatomen oder Phenyl, $R_2$, $R_4$ und $R_6$ Wasserstoff, $R^3$ Wasserstoff oder Niederalkyl mit 1-3 Kohlenstoffatomen, $R^5$ Wasserstoff oder Niederalkyl, $R^7$ Wasserstoff, $A_1$ Amino, Niederalkylamino, Hydroxy oder Niederalkoxy und $A_2$ einen Rest der oben dargestellen Formel IIa, worin T für NH oder O steht, Y Niederalkylen mit 2-3 Kohlenstoffatomen oder einen Rest der Formel

$$—CH_2—CO—NH—CH_2—CH_2—$$

bedeuten, $W_a$ für Wasserstoff und $Z_a$ für eine 1,2-Dihydroxyäthyl- oder 2-Hydroxyäthyl-Gruppe stehen, in der eine oder zwei Hydroxygruppen mit gleichen oder verschiedenen gegebenenfalls ein- oder zweifach ungesättigten Alkancarbonsäuren mit 16-22 Kohlenstoffatomen verestert oder mit einem gegebenenfalls ein- oder zweifach ungesättigten Alkanol mit 12-18 Kohlenstoffatomen veräthert sind oder worin zwei vicinale Hydroxygruppen unter Bildung eines Dioxolanringes formal mit einem Bisniederalkylketon ketalisiert oder mit einem unsubstituierten aliphatischen Aldehyd mit bis zu 20 C-Atomen acetalisiert sind, oder worin $W_a$ und $Z_a$ je eine Hydroxymethylgruppe darstellen, die mit einer gegebenenfalls ein- oder zweifach ungesättigten Alkancarbonsäure mit 16-22 Kohlenstoffatomen verestert oder mit einem gegebenenfalls ein- oder zweifach ungesättigten Alkanol mit 12-18 Kohlenstoffatomen veräthert ist, und ihrer Salze, wobei das Präfix « Nieder » Reste bis und mit 7 C-Atomen bezeichnet, sowie von N-Acetyl-muramyl-L-alanyl-D-isoglutaminyl-L-alanin-2-(1',2'-hexadecyliden-sn-glycero-3'-hydroxyphosphoryloxy)-äthylamid und N-Acetyl-desmethylmuramyl-L-alanyl-D-isoglutaminyl-L-alanin-2-(1',2'-hexadecylide-n-sn-glycero-3'-hydroxyphosphoryloxy)-äthylamid und deren Salzen.

2. Verbindungen der Formel I nach Anspruch 1, worin W Alkyl oder Alkenyl mit bis zu 30 C-Atomen bedeutet, das unsubstituiert oder durch Hydroxy, Niederalkoxy, Niederalkanoyloxy, Halogen, Amino, Alkanoylamino oder Oxo substituiert ist, und ihre Salze.

3. Verbindungen der Formel I gemäss Patentanspruch 1, worin X Carbonyl, $R_1$ Niederalkyl mit 1 bis 7 Kohlenstoffatomen oder Phenyl, $R_2$, $R_4$, $R_6$ und $R_7$ Wasserstoff, $R_3$ Wasserstoff oder Niederalkyl, $R_5$ Wasserstoff, Niederalkyl, Hydroxymethyl, Mercaptomethyl, 1-Hydroxyäthyl, 2-Methylthio-äthyl, Phenylmethyl, p-Hydroxy-phenyl-methyl, 4-Amino-butyl, 4-Imidazolyl-methyl, 3-Indolyl-methyl oder $R_4$ und $R_5$ zusammen auch Trimethylen bedeuten, worin $A_1$ für Hydroxy, Niederalkoxy, Amino, Niederalkylamino oder Aminocarbonylniederalkylamino steht und $A_2$ einen Rest der Formel

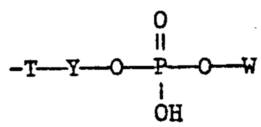

(II)

bedeutet, worin T für NH oder O und W für eine Alkyl- oder Alkenylgruppe, die unsubstituiert oder durch Hydroxy, Niederalkoxy, Niederalkanoyloxy, Amino, Niederalkylamino oder Alkanoylamino mit bis zu 25 Kohlenstoffatomen substituiert ist, oder für einen Cycloalkyl- oder Cycloalkenylrest mit 10 bis 30 Kohlenstoffatomen stehen, Y Aethylen oder einen Rest der Formeln

$$Y_1 - COO - Y_2 \qquad \text{(IIIa)}$$

oder

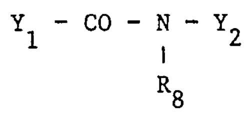

(IIIc)

bedeutet, worin $R_8$ für Wasserstoff steht und $Y_1$ und $Y_2$ unabhängig voneinander je für Niederalkylen mit 1 bis 7 Kohlenstoffatomen stehen, das gegebenenfalls durch Hydroxy, Niederalkoxy, Mercapto, Methylthio, Phenyl, 4-Imidazolyl oder 3-Indolyl substituiert ist, und ihre Salze.

4. Verbindungen der Formel I gemäss Patentanspruch 1, worin X Carbonyl, $R_1$ Niederalkyl mit 1 bis 3 Kohlenstoffatomen, $R_2$, $R_4$, $R_6$ und $R_7$ Wasserstoff, $R_3$ Wasserstoff oder Methyl, $R_5$ Wasserstoff oder Niederalkyl, $A_1$ Amino und $A_2$ einen Rest der Formel

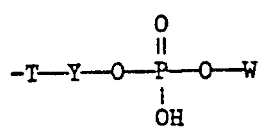

(II)

bedeuten, worin T für NH, W für eine Alkyl- oder Alkenylgruppe mit 10 bis 25 Kohlenstoffatomen, die unsubstituiert oder durch Hydroxy, Niederalkoxy, Niederalkanoyloxy, Amino, Niederalkylamino oder Alkanoylamino mit bis zu 25 Kohlenstoffatomen substituiert ist, oder für einen Cholesterylrest und Y für Aethylen oder einen Rest der Formel

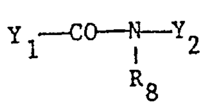

(IIIc)

stehen, worin $R_8$ Wasserstoff und $Y_1$ und $Y_2$ unabhängig voneinander je Niederalkylen bedeuten, und ihre Salze.

5. Verbindungen gemäss Patentanspruch 3 oder 4, dadurch gekennzeichnet, dass die Bedeutungen für $A_1$ und $A_2$ vertauscht sind, und ihre Salze.

6. Verbindungen der Formel I gemäss Patentanspruch 1, worin X Carbonyl, $R_1$ gegebenenfalls substituiertes Alkyl oder Aryl, $R_2$, $R_3$, $R_4$ und $R_6$ Wasserstoff oder Niederalkyl, $R_5$ Wasserstoff, gegebenenfalls durch Hydroxy, Niederalkoxy, Mercapto oder Niederalkylmercapto oder Halogen substituiertes Niederalkyl, Cycloalkyl oder Cycloalkylniederalkyl, worin der Cycloalkylrest 4 bis 6 Kohlenstoffatome enthält, gegebenenfalls substituiertes Phenyl oder Phenylniederalkyl, ein oder zwei Azaatome enthaltendes Heterocyclyl oder Heterocyclylniederalkyl oder $R_4$ und $R_5$ zusammen auch Alkylen mit 3 bis 4 Kohlenstoffatomen, $R_7$ Wasserstoff und einer der Reste $A_1$ und $A_2$ einen Rest der Formel

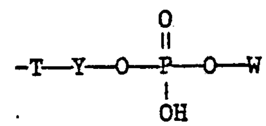

(II)

bedeuten, worin T für NH oder O steht, worin Y gegebenenfalls substituiertes Alkylen, das auch durch Carbonyloxy oder Carbonylimino unterbrochen sein kann und W eine gegebenenfalls durch Hydroxy, Niederalkanoyloxy, Amino, Alkanoylamino oder Oxo substituierte Alkylgruppe mit mehr als 6 Kohlenstoffatomen bedeuten, und der andere der Reste $A_1$ und $A_2$ Hydroxy, Niederalkoxy, Amino, Niederalkylamino oder im Niederalkylrest gegebenenfalls durch Hydroxy, Carboxy und/oder Amino substituiertes Aminocarbonylniederalkylamino ist, und ihre Salze.

7. Verbindungen der Formel I gemäss Patentanspruch 1, worin X Carbonyl, $R_1$ gegebenenfalls durch Hydroxy, Niederalkoxy oder Halogen substituiertes Niederalkyl oder gegebenenfalls durch Hydroxy, Niederalkoxy, Niederalkyl oder Halogen substituiertes Phenyl, $R_2$, $R_4$ und $R_6$ Wasserstoff, $R_3$ Wasserstoff oder Niederalkyl, $R_5$ Wasserstoff, gegebenenfalls durch Hydroxy, Niederalkoxy, Mercapto, Niederalkylmercapto oder Halogen substituiertes Niederalkyl mit 1 bis 3 Kohlenstoffatomen, Cycloalkyl oder Cycloalkylniederalkyl, worin der Cycloalkylrest 4 bis 6 Kohlenstoffatome und der Niederalkylrest 1 bis 3 Kohlenstoffatome enthält, gegebenenfalls durch Hydroxy, Niederalkoxy oder Halogen substituiertes Phenyl oder Phenylniederalkyl mit 1 bis 3 Kohlenstoffatomen im Niederalkylrest, ein oder zwei Azaatome enthaltendes Heterocyclyl oder Heterocyclylniederalkyl mit 5 bis 6 Ringgliedern und 1 bis 3 Kohlenstoffatomen im Niederalkylrest oder $R_4$ und $R_5$ zusammen auch Alkylen mit 3 bis 4 Kohlenstoffatomen, $R_7$ Wasserstoff, und einer der Reste $A_1$ und $A_2$ einen Rest der Formel

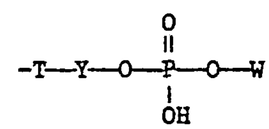

(II)

bedeuten, worin T für HN oder O steht, Y gegebenenfalls substituiertes Niederalkylen oder einen Rest einer der Formeln

$$Y_1—COO—Y_2 \qquad (IIIa)$$

$$Y_1—OOC—Y_2 \qquad (IIIb)$$

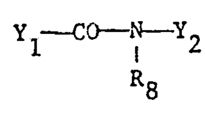

(IIIc)

oder

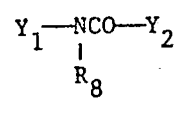

(IIId),

bedeutet, worin $Y_1$ und $Y_2$ je für gegebenenfalls substituiertes Niederalkylen und $R_8$ für Wasserstoff stehen, W eine Alkylgruppe mit 10 bis 25 Kohlenstoffatomen, die in 2-Stellung eine Hydroxygruppe, Alkanoyloxygruppe, Amino oder Alkanoylaminogruppe trägt, darstellen und der andere der Reste $A_1$ und $A_2$ Hydroxy, Niederalkoxy, Amino, Niederalkylamino oder im Niederalkylrest gegebenenfalls durch Hydroxy, Carboxy oder Aminogruppen substituiertes Aminocarbonylniederalkylamino ist, und ihre Salze.

8. Verbindungen nach einem der Ansprüche 1, 6 und 7, worin Y für gegebenenfalls durch $C_{1-4}$-Alkyl substituiertes $C_{1-4}$-Alkylen steht, das durch Carbonyloxy oder Carbonylimino unterbrochen sein kann.

9. Verbindungen der Formel I gemäss Patentanspruch 1, worin X Carbonyl, $R_1$ Niederalkyl mit 1 bis 3

Kohlenstoffatomen oder Phenyl, $R_2$, $R_4$ und $R_6$ Wasserstoff, $R_3$ Wasserstoff oder Niederalkyl mit 1 bis 3 Kohlenstoffatomen, $R_5$ Wasserstoff, gegebenfalls durch Hydroxy, Methoxy, Mercapto, Methylmercapto oder Halogen substituiertes Niederalkyl mit 1 bis 3 Kohlenstoffatomen, gegebenenfalls durch Hydroxy, Methoxy oder Halogen substituiertes Phenyl oder Phenylmethyl, ein oder zwei Azaatome enthaltendes Heterocyclyl oder Heterocyclylmethyl mit 5 Ringgliedern oder $R_4$ und $R_5$ zusammen auch Trimethylen, $R_7$ Wasserstoff und einer der Reste $A_1$ und $A_2$ einen Rest der Formel

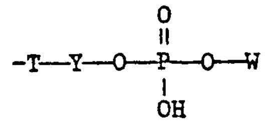

(II)

bedeuten, worin T für HN oder O steht, Y Niederalkylen mit 2 bis 3 Kohlenstoffatomen oder einen Rest der Formeln (IIIa) oder (IIIc)

$$Y_1 - COO - Y_2 \quad \text{(IIIa)}$$

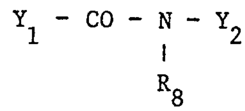

(IIIc)

bedeutet, worin $R_8$ für Wasserstoff steht und $Y_1$ und $Y_2$ unabhängig voneinander je gegebenenfalls durch Hydroxy, Niederalkoxy, Mercapto oder Niederalkylmercapto substituiertes Niederalkylen mit 1 bis 3 Kohlenstoffatomen oder Niederalkylen mit 1 bis 3 Kohlenstoffatomen bedeuten, das durch gegebenenfalls durch Hydroxy, Methoxy oder Halogen substituiertes Phenyl oder Phenylniederalkyl oder ein oder zwei Azaatome enthaltendes Heterocyclyl oder Heterocyclylniederalkyl mit 5 bis 6 Ringgliedern und 1 bis 3 Kohlenstoffatomen im Niederalkylrest substituiert ist, W eine Alkylgruppe mit 10 bis 25 Kohlenstoffatomen, die in 2-Stellung durch Hydroxy, Niederalkanoyloxy, Amino oder Alkanoylamino substituiert ist, und der andere der Reste $A_1$ und $A_2$ Hydroxy, Niederalkoxy, Amino, Niederalkylamino oder Aminocarbonylniederalkylamino ist, und ihre Salze.

10. Verbindungen der Formel I nach einem der Ansprüche 1-9, worin jeweils im Falle asymmetrischer Substitution am C-$R_3$ die D-, am C-$R_5$ die L- und C-$\underset{R_6}{N}$ die D-Konfiguration vorliegt, und ihre Salze.

11. N-Acetyl-desmethylmuramyl-L-alanyl-D-isoglutaminyl-L-alanin-2-(hexadecyloxy-hydroxyphosphoryloxy)-äthylamid und dessen Salze nach Anspruch 1.

12. N-Acetyl-muramyl-L-alanyl-D-isoglutaminyl-L-alanin-2-(hexadecyloxy-hydroxyphosphoryloxy)-äthylamid und dessen Salze nach Anspruch 1.

13. N-Acetyl-muramyl-L-alanyl-D-isoglutaminyl-L-alanin-2-(tetradecyloxy-hydroxyphosphoryloxy)-äthylamid und dessen Salze nach Anspruch 1.

14. N-Acetyl-muramyl-L-alanyl-D-isoglutaminyl-L-alanin-2-[(3'R)-hydroxy-(2'S)-palmitoylamino-4't-octadecenyloxy-hydroxyphosphoryloxy]-äthylamid und dessen Salze nach Anspruch 1.

15. N-Acetyl-muramyl-L-α-aminobutyryl-D-isoglutaminyl-L-alanin-2-[(3'R)-hydroxy-(2'S)-palmitoylamino-4't-octadecenyloxy-hydroxyphosphoryloxy]-äthylamid und dessen Salze nach Anspruch 1.

16. N-Acetyl-muramyl-L-α-aminobutyryl-D-isoglutaminyl-L-alanin-2-[(3'-R)-hydroxy-(2'S)-palmitoylamino-octadecyloxy-hydroxyphosphoryloxy]-äthylamid und dessen Salze nach Anspruch 1.

17. N-Acetyl-muramyl-L-alanyl-D-isoglutaminyl-L-alanin-2-(cholest-5-en-3β-oxy-hydroxyphosphoryloxy)-äthylamid und dessen Salze nach Anspruch 1.

18. N-Benzoyl-muramyl-L-alanyl-D-isoglutaminyl-L-alanin-2-(hexadecyloxy-hydroxyphosphoryloxy)-äthylamid und dessen Salze nach Anspruch 1.

19. Eine Verbindung ausgewählt aus der Gruppe N-Acetyl-muramyl-L-alanyl-D-isoglutamin-2-(hexadecyloxy-hydroxyphosphoryloxy)-äthylamid, N-Acetyl-desmethylmuramyl-L-alanyl-D-isoglutamin-2-(hexadecyloxy-hydroxyphosphoryloxy)-äthylamid, N-Acetyl-muramyl-L-alanyl-D-isoglutaminyl-oxymethylcarbonsäure-2-(hexadecyloxy-hydroxyphosphoryloxy)-äthylamid, N-Acetyl-muramyl-L-alanyl-D-isoglutamin-2-[(3'R)-hydroxy-(2'S)-palmitoylamino-4't-octadecenyloxy-hydroxyphosphoryloxy]-äthylamid, N-Acetyl-desmethylmuramyl-L-alanyl-D-isoglutamin-2-[(3'R)-hydroxy-(2'S)-palmitoylamino-4't-octadecenyloxyhydroxyphosphoryloxy]-äthylamid, N-Acetyl-desmethylmuramyl-L-alanyl-D-isoglutaminyl-L-alanin-2-[(3'R)-hydroxy-(2'S)-palmitoylamino-4't-octadecenyloxy-hydroxyphosphoryloxy]-äthylamid, N-Acetyl-muramyl-L-alanyl-D-isoglutaminyl-oxymethylcarbonsäure-[(3'R)-hydroxy-(2'S)-palmitoylamino-4't-octadecenyloxy-hydroxyphosphoryloxy]-äthylamid oder ein Salz dieser Verbindungen nach Anspruch 1.

20. Die immunpotenzierend wirksamen Verbindungen der Formel I und ihre Salze gemäss einem der Patentansprüche 1 bis 19.

21. Pharmazeutisch verwendbare Präparate enthaltend Verbindungen der Formel I gemäss einem der Ansprüche 1 bis 19 zusammen mit einem pharmazeutisch verwendbaren Trägermaterial.

22. Verwendung von Verbindungen der Formel I gemäss einem der Ansprüche 1 bis 19 zur Herstellung von pharmazeutischen Präparaten.

23. Eine Verbindung gemäss einem der Ansprüche 1 bis 19 zur Verwendung als Arzneimittel.

24. Eine Verbindung gemäss einem der Ansprüche 1 bis 19 zur Verwendung als immunstimulierendes Mittel.

25. Pharmazeutische Präparate, die mindestens eine Verbindung gemäss einem der Ansprüche 1 bis 19 und mindestens ein Antibiotikum zusammen mit einem pharmazeutisch verwendbaren Trägermaterial enthalten.

26. Ein Muramylpeptid nach einem der Ansprüche 1 bis 19 zur Anwendung in einem Verfahren zur Steigerung der antibiotischen Aktivität von Antibiotika, welches dadurch gekennzeichnet ist, dass man das Muramylpeptid und ein Antibiotikum zeitlich im wesentlichen gemeinsam (im Laufe eines Tages) verabreicht.

27. Verfahren zur Herstellung der Verbindungen gemäss Patentanspruch 1, dadurch gekennzeichnet, dass man

a) eine Verbindung der Formel

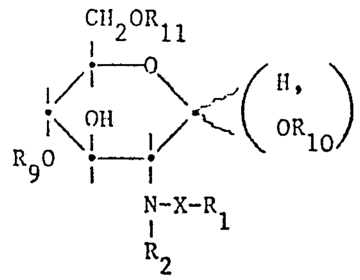

(V)

worin X, $R_1$ und $R_2$ die in Anspruch 1 genannte Bedeutung haben und gegebenenfalls darin vorhandene Hydroxygruppen mit einer leicht abspaltbaren Schutzgruppe geschützt sind, $R_9$, $R_{10}$, und $R_{11}$ je eine leicht abspaltbare Schutzgruppe bedeuten, oder eine Metallverbindung davon mit einer Verbindung der Formel

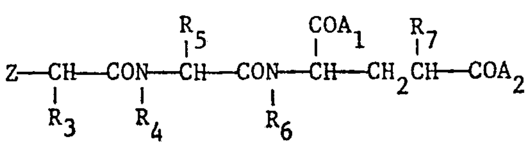

(VI)

umsetzt, worin Z eine reaktionsfähige veresterte Hydroxygruppe darstellt, $R_3$, $R_4$, $R_5$, $R_6$, $R_7$, $A_1$ und $A_2$ die in Anspruch 1 angegebene Bedeutung haben und gegebenenfalls darin vorhandene Hydroxygruppen durch eine leicht abspaltbare Schutzgruppe geschützt sind, und vorhandene Schutzgruppen abspaltet,

b) in an sich bekannter Weise eine Verbindung der Formel

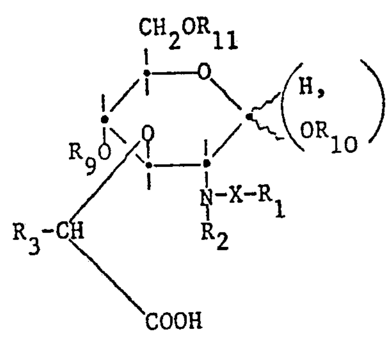

(VII)

worin X, $R_1$, $R_2$ und $R_3$ die in Anspruch 1 genannte Bedeutung besitzen und $R_9$, $R_{10}$ und $R_{11}$ für Wasserstoff oder eine leicht abspaltbare Schutzgruppe stehen, oder ein Derivat davon mit einer Verbindung der Formel

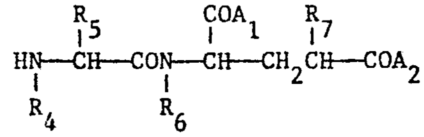

(VIII)

worin $R_4$, $R_5$, $R_6$, $R_7$, $COA_1$ und $COA_2$ die in Anspruch 1 genannte Bedeutung besitzen, mit der Massgabe, dass in diesen Resten vorhandene Carboxy- und, wenn erwünscht, freie Hydroxygruppen durch leicht abspaltbare Schutzgruppen geschützt sind, oder einem Derivat davon kondensiert und vorhandene Schutzgruppen abspaltet,

c) eine Verbindung der Formel

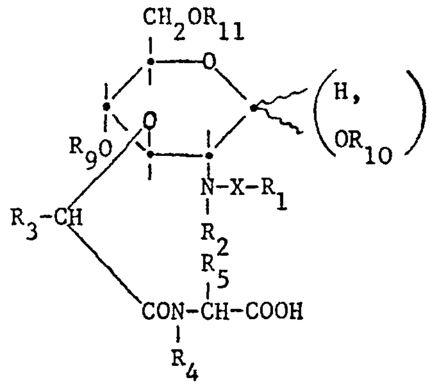

(IX)

worin X, $R_1$, $R_2$, $R_3$, $R_4$ und $R_5$ die in Anspruch 1 genannte Bedeutung haben, mit der Massgabe, dass darin enthaltene freie Hydroxygruppen gegebenenfalls mit einer leicht abspaltbaren Schutzgruppe geschützt sind, und $R_9$, $R_{10}$ und $R_{11}$ Wasserstoff oder leicht abspaltbare Schutzgruppen darstellen, oder Derivate davon mit einer Verbindung der Formel

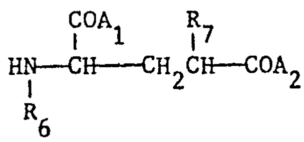

(X)

worin $R_6$, $R_7$, $A_1$ und $A_2$ die in Anspruch 1 genannte Bedeutung haben, mit der Massgabe, dass in den Resten $R_7$, $—COA_1$ und $—COA_2$ vorhandene freie Carboxylgruppen durch leicht abspaltbare Schutzgruppen geschützt sind, kondensiert und vorhandene Schutzgruppen abspaltet,

d) in an sich bekannter Weise eine Verbindung der Formel

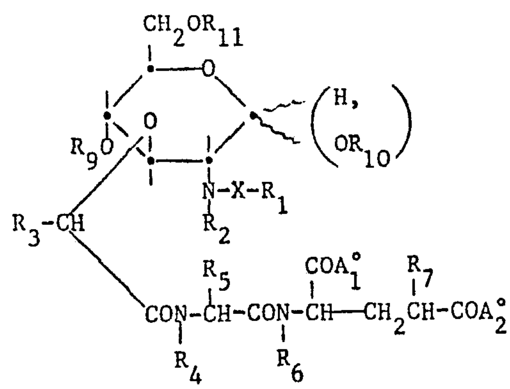

(XI)

worin X, $R_1$, $R_2$, $R_3$, $R_4$, $R_5$, $R_6$ und $R_7$ die in Anspruch 1 genannte Bedeutung haben, $R_9$, $R_{10}$ und $R_{11}$ Wasserstoff oder eine leicht abspaltbare Schutzgruppe darstellen und einer der Reste $A_1°$ und $A_2°$ eine aktivierte Hydroxygruppe darstellt und der andere veräthertes Hydroxy, Amino, Niederalkylamino oder Aminocarbonylniederalkylamino ist, mit einer Verbindung der Formel

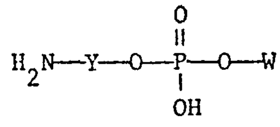

(XII)

worin Y und W die in Anspruch 1 genannte Bedeutung besitzen, kondensiert und gegebenenfalls vorhandene Schutzgruppen abspaltet,

e) in an sich bekannter Weise eine Verbindung der Formel

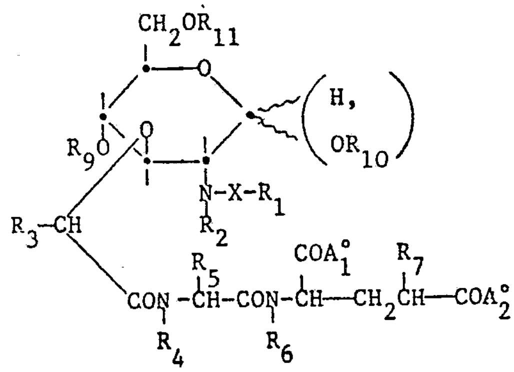

(XIa)

worin X, $R_1$, $R_2$, $R_3$, $R_4$, $R_5$, $R_6$ und $R_7$ die in Anspruch 1 genannte Bedeutung haben, $R_9$, $R_{10}$ und $R_{11}$ Wasserstoff oder eine leicht abspaltbare Schutzgruppe darstellen und einer der Reste $A_1^{\circ}$ und $A_2^{\circ}$ eine Hydroxygruppe darstellt und der andere veräthertes Hydroxy, Amino, Niederalkylamino oder Aminocarbonylniederalkylamino ist, mit einer Verbindung der Formel

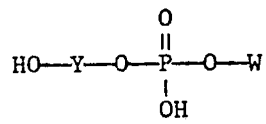

(XIIa)

worin Y und W die in Anspruch 1 genannte Bedeutung besitzen, wobei die Säure XIa oder der Alkohol XIIa in reaktionsfähiger Form vorliegt, in an sich bekannter Weise verestert und gegebenenfalls vorhandene Schutzgruppen abspaltet,

f) in einer Verbindung der Formel

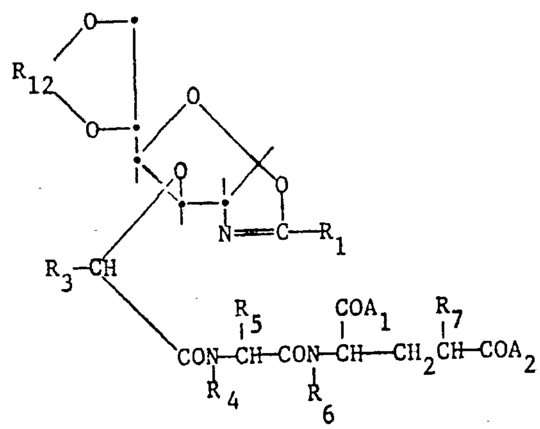

(XIII)

worin $R_1$, $R_3$, $R_4$, $R_5$, $R_6$, $R_7$, $A_1$ und $A_2$ die in Anspruch 1 genannte Bedeutung haben und $R_{12}$ eine Alkyliden- oder Cycloalkylidengruppe ist, den Oxazolin- und den Dioxolanring sauer aufspaltet, und gegebenenfalls vorhandene Schutzgruppen abspaltet,

g) eine Verbindung der Formel

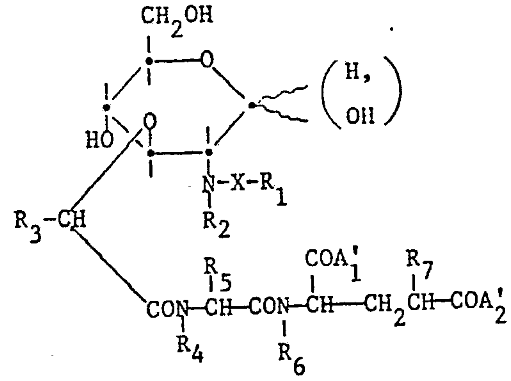

(XIV)

worin einer der Reste $A_1'$ und $A_2'$ einen Rest der Formel

$$-T-Y_1-M_1 \quad (XV)$$

und der andere der Reste $A_1'$ und $A_2'$ veräthertes Hydroxy oder Amino, Niederalkylamino oder Aminocarbonylniederalkylamino ist, und einer Verbindung der Formel

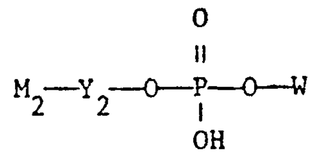

(XVI)

worin X, $R_1$, $R_2$, $R_3$, $R_4$, $R_5$, $R_6$, $R_7$, T und W die in Anspruch 1 genannten Bedeutungen haben, $Y_1$ und $Y_2$ je für gegebenenfalls substituiertes Niederalkylen stehen und darin vorhandene Hydroxygruppen gegebenenfalls durch leicht abspaltbare Schutzgruppen geschützt sind und einer der Reste $M_1$ und $M_2$ eine freie Aminogruppe oder ein aktiviertes Derivat davon und der andere eine Carbonsäuregruppe oder ein aktiviertes Derivat davon bedeutet, kondensiert und gegebenenfalls vorhandene Schutzgruppen abspaltet,

h) eine Verbindung der Formel

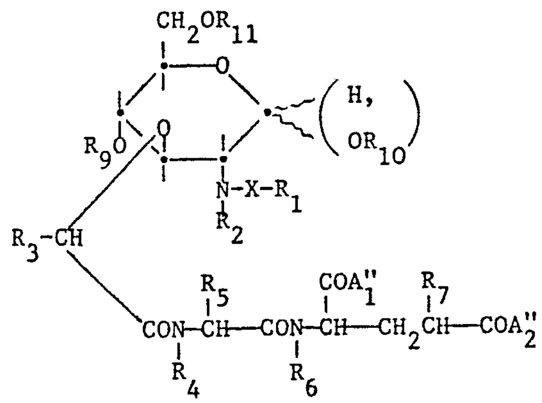

(XVII)

worin einer der Reste $A_1''$ und $A_2''$ einen Rest der Formel

$$-T-Y_1-M_3 \quad (XVIII)$$

darstellt, mit einer Verbindung der Formel

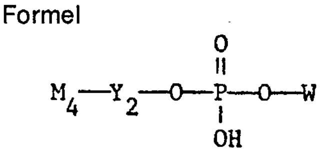

(XIX)

worin X, $R_1$, $R_2$, $R_3$, $R_4$, $R_5$, $R_6$, $R_7$, T und W die in Anspruch 1 genannte Bedeutung haben, $Y_1$ und $Y_2$ je für gegebenenfals substituiertes Niederalkylen stehen und gegebenenfalls darin vorhandene Hydroxygruppen durch leicht abspaltbare schutzgruppen geschützt sind, $R_9$, $R_{10}$ und $R_{11}$ leicht abspaltbare Schutzgruppen darstellen, und der andere der Reste $A_1''$ und $A_2''$ veräthertes Hydroxy, Amino, Niederalkylamino oder Aminocarbonylniederalkylamino ist, und einer der Reste $M_3$ und $M_4$ eine freie Hydroxygruppe und der andere eine freie Carboxylgruppe darstellt, wobei gegebenenfalls einer der beiden Reste $M_3$ und $M_4$ in reaktionsfähiger Form vorliegt, in an sich bekannter Weise verestert und gegebenenfalls vorhandene Schutzgruppen abspaltet,

i) eine Verbindung der Formel

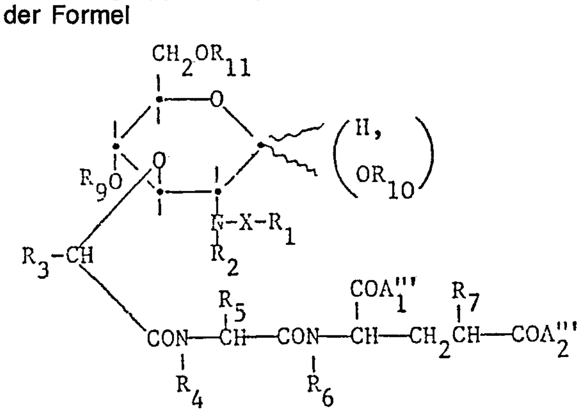

(XX)

worin X, $R_1$, $R_2$, $R_3$, $R_4$, $R_5$, $R_6$ und $R_7$ die in Anspruch 1 genannten Bedeutungen haben und gegebenenfalls darin vorhandene Hydroxygruppen mit einer leicht abspaltbaren Schutzgruppe geschützt sind, $R_9$, $R_{10}$ und $R_{11}$ leicht abspaltbare Schutzgruppen bedeuten und einer der Reste $A_1'''$ und $A_2'''$ für —T—Y—OH steht, worin Y und T die in Anspruch 1 genannten Bedeutungen besitzen, und der andere der Reste $A_1'''$ und $A_2'''$ für freies oder veräthertes Hydroxy, Amino, Niederalkylamino oder im Niederalkylrest gegebenenfalls substituiertes aminocarbonylniederalkylamino steht, mit einer den Rest der Formel XXI,

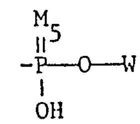
(XXI)

worin $=M_5$ ein Elektronenpaar oder Oxo bedeutet und W die in Anspruch 1 genannte Bedeutung hat, abgebenden Verbindung umsetzt, falls $=M_5$ ein Elektronenpaar ist, mit einem schwachen Oxidationsmittel oxidiert, und vorhandene Schutzgruppen abspaltet,

k) eine Verbindung der Formel

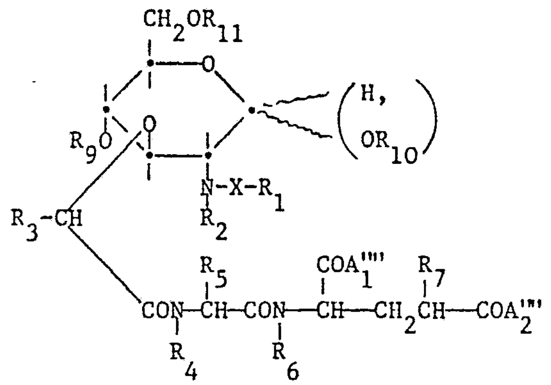
(XXIII)

worin X, $R_1$, $R_2$, $R_3$, $R_4$, $R_5$, $R_6$ und $R_7$ die in Anspruch 1 genannten Bedeutungen besitzen und gegebenenfalls darin vorhandene Hydroxygruppen mit einer leicht abspaltbaren Schutzgruppe geschützt sind, $R_9$, $R_{10}$ und $R_{11}$ leicht abspaltbare Schutzgruppen bedeuten und einer der Reste $A_1''''$ und $A_2''''$ für

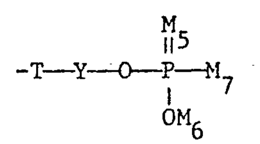
(XXIV)

und der andere für freies oder veräthertes Hydroxy, Amino, Niederalkylamino oder Aminocarbonylniederalkylamino steht, worin T und Y die in Anspruch 1 genannte Bedeutung besitzen, $=M_5$ ein Elektronenpaar oder Oxo, $M_6$ Wasserstoff oder eine leicht abspaltbare Schutzgruppe und $M_7$ eine gegebenenfalls in reaktionsfähiger Form vorliegende Hydroxygruppe bedeuten, mit einer Verbindung der Formel

HO—W (XXV)

worin W die in Anspruch 1 genannte Bedeutung besitzt, umsetzt, falls $=M_5$ ein Elektronenpaar ist, mit einem schwachen Oxidationsmittel oxidiert, und vorhandene Schutzgruppen abspaltet,

l) in einer Verbindung der Formel I, in der eine oder mehrere funktionelle Gruppen durch Schutzgruppen geschützt sind, diese Schutzgruppen abspaltet, und, wenn erwünscht, nach Durchführung eines der Verfahren a-l) eine erhaltene verbindung der Formel I in ihr Salz überführt.

28. Verfahren nach Anspruch 27, dadurch gekennzeichnet, dass man die Ausgangsstoffe so wählt, dass man eine Verbindung gemäss Anspruch 10 erhält.

29. Die nach einem Verfahren des Anspruchs 27 erhältlichen Verbindungen und ihre Salze.

**Patentansprüche** (für den Vertragsstaat AT)

1. Verfahren zur Herstellung von Muramylpeptiden der allgemeinen Formel

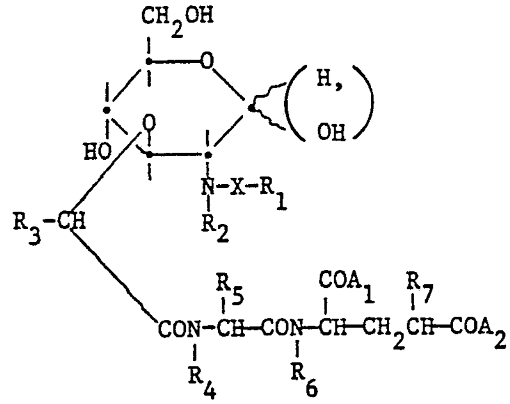

(I)

worin X Carbonyl oder carbonyloxy, $R_1$ gegebenenfalls substituiertyes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkylniederalkyl, gegebenenfalls substituiertes Aryl oder Aralkyl, stickstoffhaltiges Heterocyclyl oder Heterocyclylniederalkyl, oder $R_4$ und $R_5$ zusammen auch Alkylen mit 3 bis 4 Kohlenstoffatomen, $R_7$ Wasserstoff oder gegebenenfalls verestertes oder amidiertes Carboxyl und einer der Reste $A_1$ und $A_2$ einen Rest der Formel

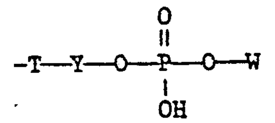

(II)

bedeutet, worin T für NH oder O steht, Y eine gegebenenfalls substituierte Alkylengruppe, die auch durch ein oder zwei Oxycarbonyl und/oder Iminocarbonyl unterbrochen sein kann, und W einen aliphatischen Rest oder einen Cycloalkyl- oder Cycloalkenylrest mit jeweils mehr als 6 Kohlenstoffatomen darstellen, und der andere der Reste $A_1$ oder $A_2$ freies oder veräthertes Hydroxy, Amino, Niederalkylamino oder im Niederalkylrest gegebenenfalls substituiertes Aminocarbonylniederalkylamino ist, wobei das vorstehend verwendete Präfix « Nieder » einen Rest bis und mit 7 C-Atomen bezeichnet, und/oder ihrer Salze mit Ausnahme der in der europäischen Patentanmeldung mit der Veröffentlichungsnummer 0 025 495 offenbarten Verbindungen, nämlich ausgenommen von Muramylpeptiden der Formel

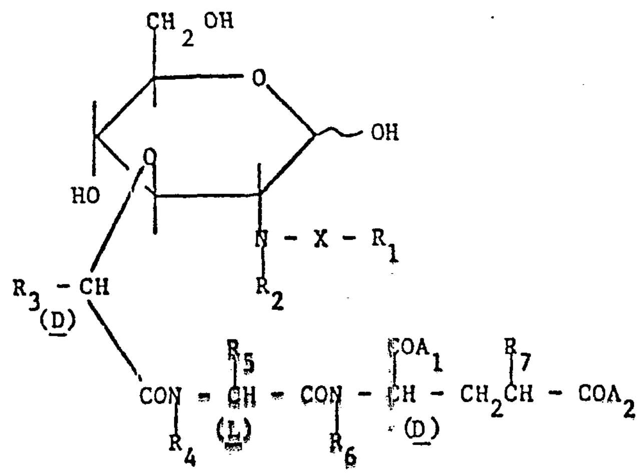

(Ia)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkyl-niederalkyl, gegebenenfalls substituiertes Aryl oder Aralkyl, stickstoffhaltiges Heterocyclyl oder Heterocyclylniederalkyl, oder $R_4$ und $R_5$ zusammen auch Alkylen mit 3-4 Kohlenstoffatomen, $R_7$ Wasserstoff oder gegebenenfalls verestertes oder amidiertes Carboxyl und einer der Reste $A_1$ und $A_2$ einen Rest der Formel

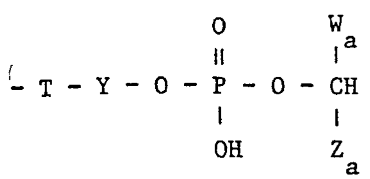

(IIa)

bedeuten, worin T für HN oder O steht, Y eine gegebenenfalls substituierte Alkylengruppe, die auch durch ein oder zwei Oxycarbonyl und/oder Iminocarbonyl unterbrochen sein kann, $W_a$ Wasserstoff und $Z_a$ eine 1,2-Dihydroxyäthyl- oder 2-Hydroxyäthylgruppe, in denen mindestens eine Hydroxygruppe mit einer gegebenenfalls ungesättigten aliphatischen Carbonsäure mit 12-90 C-Atomen verestert oder mit einem gegebenenfalls ungesättigten aliphatischen Alkohol mit 10-22 C-Atomen veräthert ist, oder $W_a$ und $Z_a$ je eine mit einer gegebenenfalls ungesättigten aliphatischen Carbonsäure mit 12-90 C-Atomen veresterte oder mit einem gegebenenfalls ungesättigten aliphatischen Alkohol mit 10-22 C-Atomen verätherte Hydroxymethylgruppe darstellen, und der andere der Reste $A_1$ und $A_2$ freies oder veräthertes Hydroxy, Amino, Niederalkylamino oder Carboxamidoniederalkyl-amino ist, und ihrer Salze, wobei das Präfix « Nieder » Reste bis und mit 7 C-Atomen bezeichnet, sowie von Muramylpeptiden der oben dargestellten Formel Ia, worin X Carbonyl oder Carbonyloxy, $R_1$ Niederalkyl mit 1-3 Kohlenstoffatomen oder Phenyl, $R_2$, $R_4$ und $R_6$ Wasserstoff, $R^3$ Wasserstoff oder Niederalkyl mit 1-3 Kohlenstoffatomen, $R^5$ Wasserstoff oder Niederalkyl, $R^7$ Wasserstoff, $A_1$ Amino, Niederalkylamino, Hydroxy oder Niederalkoxy und $A_2$ einen Rest der oben dargestellen Formel IIa, worin T für NH oder O steht, Y Niederalkylen mit 2-3 Kohlenstoffatomen oder einen Rest der Formel

$$—CH_2—CO—NH—CH_2—CH_2—$$

bedeuten, $W_a$ für Wasserstoff und $Z_a$ für eine 1,2-Dihydroxyäthyl- oder 2-Hydroxyäthyl-Gruppe stehen, in der eine oder zwei Hydroxygruppen mit gleichen oder verschiedenen gegebenenfalls ein- oder zweifach ungesättigten Alkancarbonsäuren mit 16-22 Kohlenstoffatomen verestert oder mit einem gegebenenfalls ein- oder zweifach ungesättigten Alkanol mit 12-18 Kohlenstoffatomen veräthert sind oder worin zwei vicinale Hydroxygruppen unter Bildung eines Dioxolanringes formal mit einem Bisniederalkylketon ketalisiert oder mit einem unsubstituierten aliphatischen Aldehyd mit bis zu 20 C-Atomen acetalisiert sind, oder worin $W_a$ und $Z_a$ je eine Hydroxymethylgruppe darstellen, die mit einer gegebenenfalls ein- oder zweifach ungesättigten Alkancarbonsäure mit 16-22 Kohlenstoffatomen verestert oder mit einem gegebenenfalls ein-oder zweifach ungesättigten Alkanol mit 12-18 Kohlenstoffatomen veräthert ist, und ihrer Salze, wobei das Präfix « Nieder » Reste bis und mit 7 C-Atomen bezeichnet, sowie von N-Acetyl-muramyl-L-alanyl-D-isoglutaminyl-L-alanin-2-(1′,2′-hexadecyliden-sn-glycero-3′-hydroxy-phosphoryloxy)-äthylamid und N-Acetyl-desmethylmuramyl-L-alanyl-D-isoglutaminyl-L-alanin-2-(1′-2′-hexadecyliden-sn-glycero-3′-hydroxyphosphoryloxy)-äthylamid und deren Salzen, dadurch gekennzeichnet, dass man

a) eine Verbindung der Formel

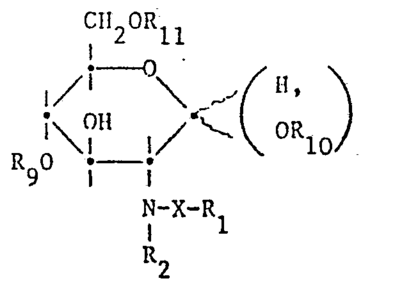

(V)

worin X, $R_1$ und $R_2$ die obengenannte Bedeutung haben und gegebenenfals darin vorhandene Hydroxygruppen mit einer leicht abspaltbaren Schutzgruppe geschützt sind, $R_9$, $R_{10}$ und $R_{11}$ je eine leicht abspaltbare Schutzgruppe bedeuten, oder eine Metallverbindung davon mit einer Verbindung der Formel

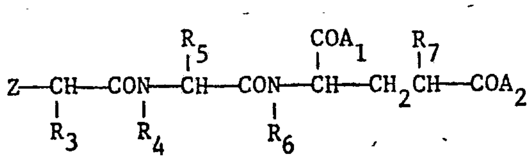

(VI)

umsetzt, worin Z eine reaktionsfähige veresterte Hydroxygruppe darstellt, $R_3$, $R_4$, $R_5$, $R_6$, $R_7$, $A_1$ und $A_2$ die obengenannte Bedeutung haben und gegebenenfalls darin vorhandene Hydroxygruppen durch eine leicht abspaltbare Schutzgruppe geschützt sind, und vorhandene Schutzgruppen abspaltet,

b) in an sich bekannter Weise eine Verbindung der Formel

(Siehe die Figur, Seite 39 f.)

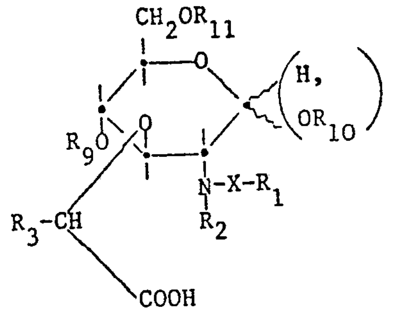

(VII)

worin X, $R_1$, $R_2$ und $R_3$ die obengenannte Bedeutung besitzen und $R_9$, $R_{10}$ und $R_{11}$ für Wasserstoff oder eine leicht abspaltbare Schutzgruppe stehen, oder ein Derivat davon mit einer Verbindung der Formel

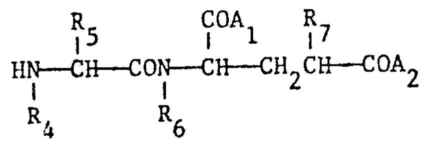

(VIII)

worin $R_4$, $R_5$, $R_6$, $R_7$, $A_1$ und $A_2$ die obengenannte Bedeutung besitzen, mit der Massgabe, dass in diesen Resten vorhandene Carboxy- und, wenn erwünscht, freie Hydroxygruppen durch leicht abspaltbare Schutzgruppen geschützt sind, oder einem Derivat davon kondensiert und vorhandene Schutzgruppen abspaltet,

c) eine Verbindung der Formel

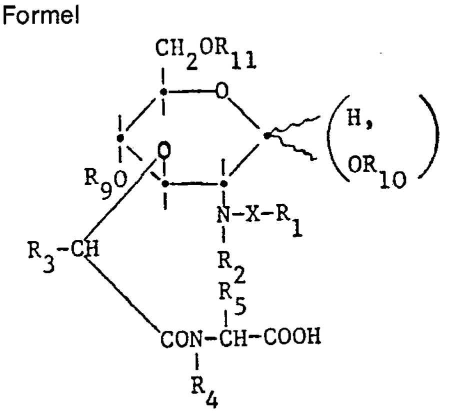

(IX)

worin X, $R_1$, $R_2$, $R_3$, $R_4$ und $R_5$ die obengenannte Bedeutung haben, mit der Massgabe, dass darin enthaltene freie Hydroxygruppen gegebenenfalls mit einer leicht abspaltbaren Schutzgruppe geschützt sind, und $R_9$, $R_{10}$ und $R_{11}$ Wasserstoff oder leicht abspaltbare Schutzgruppen darstellen, oder Derivate davon mit einer Verbindung der Formel

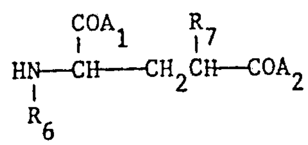

(X)

worin $R_6$, $R_7$, $A_1$ und $A_2$ die obengenannte Bedeutung haben, mit der Massgabe, dass in den Resten $R_7$, $COA_1$ und $COA_2$ vorhandene freie Carboxylgruppen durch leicht abspaltbare Schutzgruppen geschützt sind, kondensiert und vorhandene Schutzgruppen abspaltet,

d) in an sich bekannter Weise eine Verbindung der Formel

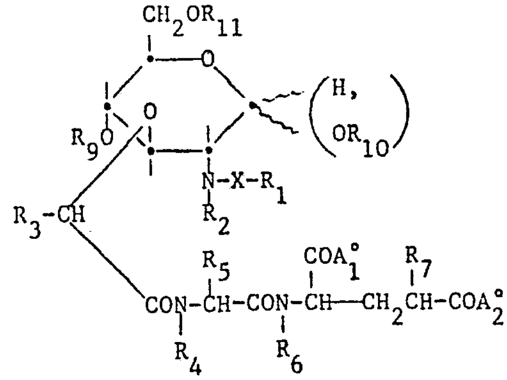

(XI)

worin X, $R_1$, $R_2$, $R_3$, $R_4$, $R_5$, $R_6$ und $R_7$ die obengenannte Bedeutung haben, $R_9$, $R_{10}$ und $R_{11}$ Wasserstoff oder eine leicht abspaltbare Schutzgruppe darstellen und einer der Reste $A_1^o$ und $A_2^o$ eine aktivierte Hydroxygruppe darstellt und der andere veräthertes Hydroxy, Amino, Niederalkylamino oder Aminocarbonylniederalkylamino ist, mit einer Verbindung der Formel

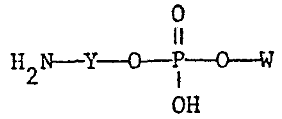

(XII)

worin Y und W die in Anspruch 1 genannte Bedeutung besitzen, kondensiert und gegebenenfalls vorhandene Schutzgruppen abspaltet,

e) in an sich bekannter Weise eine Verbindung der Formel

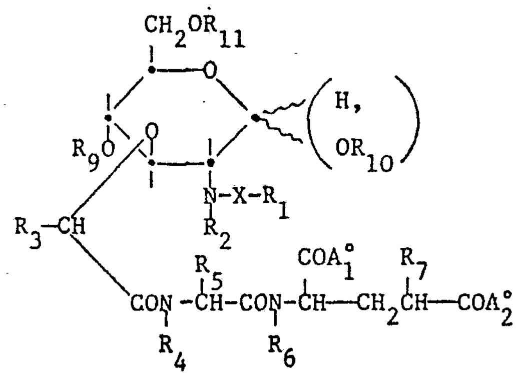

(XIa)

worin X, $R_1$, $R_2$, $R_3$, $R_4$, $R_5$, $R_6$ und $R_7$ die obengenannte Bedeutung haben, $R_9$, $R_{10}$ und $R_{11}$ Wasserstoff oder eine leicht abspaltbare Schutzgruppe darstellen und einer der Reste $A_1^o$ und $A_2^o$ eine Hydroxygruppe darstellt und der andere veräthertes Hydroxy, Amino, Niederalkylamino oder Aminocarbonylniederalkylamino ist, mit einer Verbindung der Formel

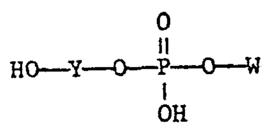

(XIIa)

worin Y und W die obengenannte Bedeutung besitzen, wobei die Säure XIa oder der Alkohol XIIa in reaktionsfähiger Form vorliegt, in an sich bekannter Weise verestert und gegebenenfalls vorhandene Schutzgruppen abspaltet,

f) in einer Verbindung der Formel

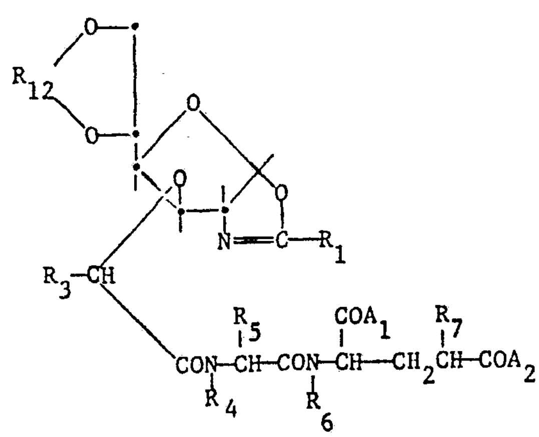

(XIII)

worin $R_1$, $R_3$, $R_4$, $R_5$, $R_6$, $R_7$, $A_1$ und $A_2$ die obengenannte Bedeutung haben und $R_{12}$ eine Alkyliden- oder Cycloalkylidengruppe ist, den Oxazolin- und den Dioxolanring sauer aufspaltet, und gegebenenfalls vorhandene Schutzgruppen abspaltet,

g) eine Verbindung der Formel

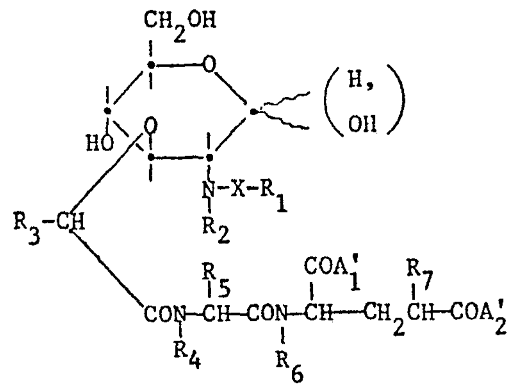

(XIV)

worin einer der Reste $A_1'$ und $A_2'$ einen Rest der Formel

—T—$Y_1$—$M_1$ (XV)

und der andere der Reste $A_1'$ und $A_2'$ veräthertes Hydroxy oder Amino, Niederalkylamino oder Aminocarbonylniederalkylamino ist, und einer Verbindung der Formel

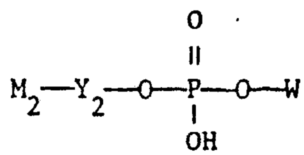

(XVI)

worin X, $R_1$, $R_2$, $R_3$, $R_4$, $R_5$, $R_6$, $R_7$, T und W die obengenannten Bedeutungen haben, $Y_1$ und $Y_2$ je für gegebenenfalls substituiertes Niederalkylen stehen und darin vorhandene Hydroxygruppen gegebenenfalls durch leicht abspaltbare Schutzgruppen geschützt sind und einer der Reste $M_1$ und $M_2$ eine freie Aminogruppe oder ein aktiviertes Derivat davon und der andere eine Carbonsäuregruppe oder ein aktiviertes Derivat davon bedeutet, kondensiert und gegebenenfalls vorhandene Schutzgruppen abspaltet,

h) eine Verbindung der Formel

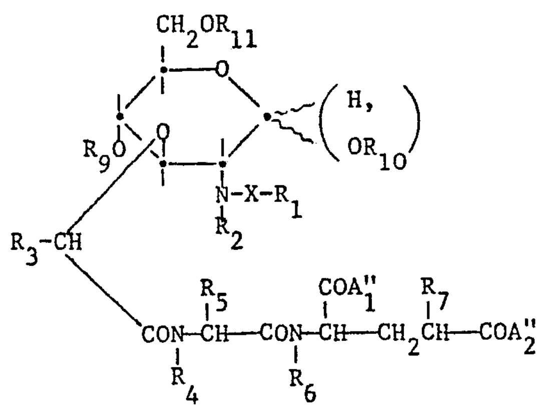

(XVII)

worin einer der Reste $A_1''$ und $A_2''$ einen Rest der Formel

—T—$Y_1$—$M_3$ (XVIII)

darstellt, mit einer Verbindung der Formel

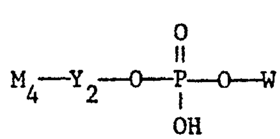

(XIX)

worin X, $R_1$, $R_2$, $R_3$, $R_4$, $R_5$, $R_6$, $R_7$, T und W die obengenannte Bedeutung haben, $Y_1$ und $Y_2$ je für gegebenenfalls substituiertes Niederalkylen stehen und gegebenenfalls darin vorhandene Hydroxygruppen durch leicht abspaltbare Schutzgruppen geschützt sind, $R_9$, $R_{10}$ und $R_{11}$ leicht abspaltbare Schutzgruppen darstellen, und der andere der Reste $A_1$" und $A_2$" veräthertes Hydroxy, Amino, Niederalkylamino oder Aminocarbonylniederalkylamino ist, und einer der Reste $M_3$ und $M_4$ eine freie Hydroxygruppe und der andere eine freie Carboxylgruppe darstellt, wobei gegebenenfalls einer der beiden Reste $M_3$ und $M_4$ in reaktionsfähiger Form vorliegt, in an sich bekannter Weise verestert und gegebenenfalls vorhandene Schutzgruppen abspaltet,

i) eine Verbindung der Formel

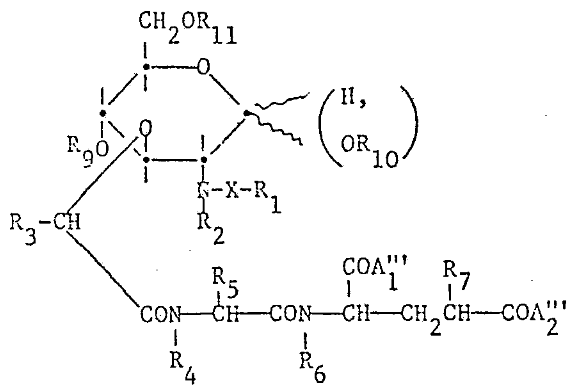

(XX)

worin X, $R_1$, $R_2$, $R_3$, $R_4$, $R_5$, $R_6$ und $R_7$ die in Anspruch 1 genannten Bedeutungen haben und gegebenenfalls darin vorhandene Hydroxygruppen mit einer leicht abspaltbaren Schutzgruppe geschützt sind, $R_9$, $R_{10}$ und $R_{11}$ leicht abspaltbare Schutzgruppen bedeuten und einer der Reste $A_1'''$ und $A_2'''$ für —T—Y—OH steht, worin Y und T die obengenannten Bedeutungen besitzen, und der andere der Reste $A_1'''$ und $A_2'''$ für freies oder veräthertes Hydroxy, Amino, Niederalkylamino oder im Niederalkylrest gegebenenfalls substituiertes Aminocarbonylniederalkylamino steht, mit einer den Rest der Formel

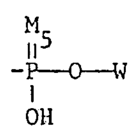

(XXI)

worin $=M_5$ ein Elektronenpaar oder Oxo bedeutet und W die obengenannte Bedeutung hat, abgebenden Verbindungen umsetzt, falls $=M_5$ ein Elektronenpaar ist, mit einem schwachen Oxidationsmittel oxidiert, und vorhandene Schutzgruppen abspaltet,

k) eine Verbindung der Formel

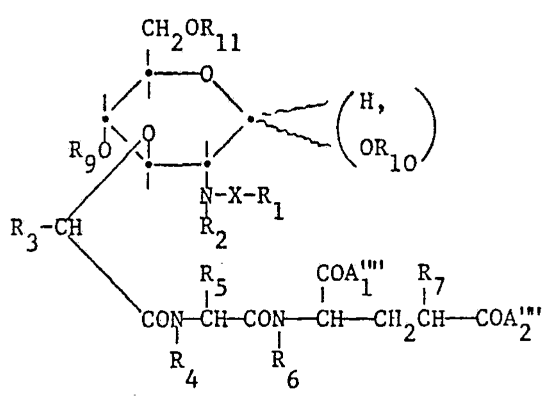

(XXIII)

worin X, $R_1$, $R_2$, $R_3$, $R_4$, $R_5$, $R_6$ und $R_7$ die obengenannten Bedeutungen besitzen und gegebenenfalls darin vorhandene Hydroxygruppen mit einer leicht abspaltbaren Schutzgruppe geschützt sind, $R_9$, $R_{10}$ und $R_{11}$ leicht abspaltbare Schutzgruppen bedeuten und einer der Reste $A_1'''$ und $A_2'''$ für

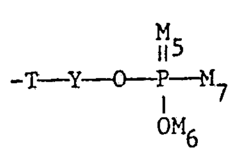

(XXIV)

und der andere für freies oder veräthertes Hydroxy, Amino, Niederalkylamino oder Aminocarbonylniederalkylamino steht, worin T und Y die in Anspruch 1 genannte Bedeutung besitzen, $=M_5$ ein Elektro-

nenpaar oder Oxo, $M_6$ Wasserstoff oder eine leicht abspaltbare Schutzgruppe und $M_7$ eine gegebenenfalls in reaktionsfähiger Form vorliegende Hydroxygruppe bedeuten, mit einer Verbindung der Formel

HO—W (XXV)

worin W die obengenannte Bedeutung besitzt, umsetzt, falls $=M_5$ ein Elektronenpaar ist, mit einem schwachen Oxidationsmittel oxidiert, und vorhandene Schutzgruppen abspaltet, oder

l) in einer Verbindung der Formel I, in der eine oder mehrere funktionelle Gruppen durch Schutzgruppen geschützt sind, diese Schutzgruppen abspaltet, und, wenn erwünscht, nach Durchführung eines der Verfahren a-l) eine erhaltene Verbindung der Formel I in ein Salz überführt.

2. Verfahren nach Anspruch 1, dadurch gekennzeichnet, dass man Verbindungen der Formel I gemäss Anspruch 1, worin W Alkyl oder Alkenyl mit bis zu 30 C-Atomen bedeutet, das unsubstituiert oder durch Hydroxy, Niederalkoxy, Niederalkanoyloxy, Halogen, Amino, Alkanoylamino oder Oxo substituiert ist, und/oder ihre Salze herstellt.

3. Verfahren nach Anspruch 1, dadurch gekennzeichnet, dass man Verbindungen der Formel I gemäss Patentanspruch 1 herstellt, worin X Carbonyl, $R_1$ Niederalkyl mit 1 bis 7 Kohlenstoffatomen oder Phenyl, $R_2$, $R_4$, $R_6$ und $R_7$ Wasserstoff, $R_3$ Wasserstoff oder Niederalkyl, $R_5$ Wasserstoff, Niederalkyl, Hydroxymethyl, Mercaptomethyl, 1-Hydroxy-äthyl, 2-Methylthio-äthyl, Phenylmethyl, p-Hydroxy-phenylmethyl, 4-Amino-butyl, 4-Imidazolyl-methyl, 3-Indolyl-methyl oder $R_4$ und $R_5$ zusammen auch Trimethylen bedeuten, worin $A_1$ für Hydroxy, Niederalkoxy, Amino, Niederalkylamino oder Aminocarbonylniederalkylamino steht und $A_2$ einen Rest der Formel

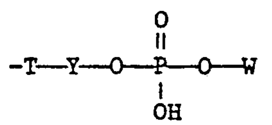
(II)

bedeutet, worin T für NH oder O und W für eine Alkyl- oder Alkenylgruppe, die unsubstituiert oder durch Hydroxy, Niederalkoxy, Niederalkanoyloxy, Amino, Niederalkylamino oder Alkanoylamino mit bis zu 25 Kohlenstoffatomen substituiert ist, oder für einen Cycloalkyl- oder Cycloalkenylrest mit 10 bis 30 Kohlenstoffatomen stehen, Y Aethylen oder einen Rest der Formeln

$Y_1 - COO - Y_2$ (IIIa)

oder

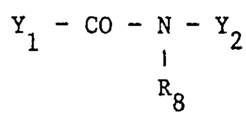
(IIIc)

bedeutet, worin $R_8$ für Wasserstoff steht und $Y_1$ und $Y_2$ unabhängig voneinander je für Niederalkylen mit 1 bis 7 Kohlenstoffatomen stehen, das gegebenenfalls durch Hydroxy, Niederalkoxy, Mercapto, Methylthio, Phenyl, 4-Imidazolyl oder 3-Indolyl substituiert ist, und/oder ihre Salze.

4. Verfahren nach Anspruch 1, dadurch gekennzeichnet, dass man Verbindungen der Formel I gemäss Patentanspruch 1 herstellt, worin X Carbonyl, $R_1$ Niederalkyl mit 1 bis 3 Kohlenstoffatomen, $R_2$, $R_4$, $R_6$ und $R_7$ Wasserstoff, $R_3$ Wasserstoff oder Methyl, $R_5$ Wasserstoff oder Niederalkyl, $A_1$ Amino und $A_2$ einen Rest der Formel

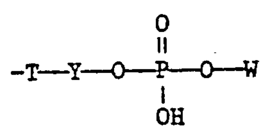
(II)

bedeuten, worin T für NH, W für eine Alkyl- oder Alkenylgruppe mit 10 bis 25 Kohlenstoffatomen, die unsubstituiert oder durch Hydroxy, Niederalkoxy, Niederalkanoyloxy, Amino, Niederalkylamino oder Alkanoylamino mit bis zu 25 Kohlenstoffatomen substituiert ist, oder für einen Cholesterylrest und Y für Aethylen oder einen Rest der Formel

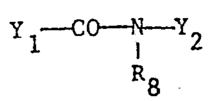
(IIIc)

stehen, worin $R_8$ Wasserstoff und $Y_1$ und $Y_2$ unabhängig voneinander je Niederalkylen bedeuten, und/oder ihre Salze.

5. Verfahren nach Anspruch 1, dadurch gekennzeichnet, dass man Verbindungen gemäss Patentanspruch 3 oder 4 herstellt, worin die Bedeutungen für $A_1$ und $A_2$ vertauscht sind, und/oder ihre Salze.

6. Verfahren nach Anspruch 1, dadurch gekennzeichnet, dass man Verbindungen der Formel I gemäss Patentanspruch 1 herstellt, worin X Carbonyl, $R_1$ gegebenenfalls substituiertes Alkyl oder Aryl, $R_2$, $R_3$, $R_4$ und $R_6$ Wasserstoff oder Niederalkyl, $R_5$ Wasserstoff, gegebenenfalls durch Hydroxy, Niederalkoxy, Mercapto oder Niederalkylmercapto oder Halogen substituiertes Niederalkyl, Cycloalkyl oder Cycloalkylniederalkyl, worin der Cycloalkylrest 4 bis 6 Kohlenstoffatome enthält, gegebenenfalls substituiertes Phenyl oder Phenylniederalkyl, ein oder zwei Azaatome enthaltendes Heterocyclyl oder Heterocyclylniederalkyl oder $R_4$ und $R_5$ zusammen auch Alkylen mit 3 bis 4 Kohlenstoffatomen, $R_7$ Wasserstoff und einer der Reste $A_1$ und $A_2$ einen Rest der Formel

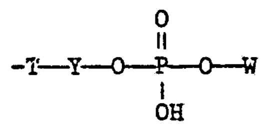
(II)

bedeuten, worin T für NH oder O steht, worin Y gegebenenfalls substituiertes Alkylen, das auch durch Carbonyloxy oder Carbonylimino unterbrochen sein kann und W eine gegebenenfalls durch Hydroxy, Niederalkanoyloxy, Amino, Alkanoylamino oder Oxo substituierte Alkylgruppe mit mehr als 6 Kohlenstoffatomen bedeuten, und der andere der Reste $A_1$ und $A_2$ Hydroxy, Niederalkoxy, Amino, Niederalkylamino oder im Niederalkylrest gegebenenfalls durch Hydroxy, Carboxy und/oder Amino substituiertes Aminocarbonylniederalkylamino ist, und/oder ihre Salze.

7. Verfahren nach Anspruch 1, dadurch gekennzeichnet, dass man Verbindungen der Formel I gemäss Patentanspruch 1 herstellt, worin X Carbonyl, $R_1$ gegebenenfalls durch Hydroxy, Niederalkoxy oder Halogen substituiertes Niederalkyl oder gegebenenfalls durch Hydroxy, Niederalkoxy, Niederalkyl oder Halogen substituiertes Phenyl, $R_2$, $R_4$ und $R_6$ Wasserstoff, $R_3$ Wasserstoff oder Niederalkyl, $R_5$ Wasserstoff, gegebenenfalls durch Hydroxy, Niederalkoxy, Mercapto, Niederalkylmercapto oder Halogen substituiertes Niederalkyl mit 1 bis 3 Kohlenstoffatomen, Cycloalkyl oder Cycloalkylniederalkyl, worin der Cycloalkylrest 4 bis 6 Kohlenstoffatome und der Niederalkylrest 1 bis 3 Kohlenstoffatome enthält, gegebenenfalls durch Hydroxy, Niederalkoxy oder Halogen substituiertes Phenyl oder Phenylniederalkyl mit 1 bis 3 Kohlenstoffatomen im Niederalkylrest, ein oder zwei Azaatome enthaltendes Heterocyclyl oder Heterocyclylniederalkyl mit 5 bis 6 Ringgliedern und 1 bis 3 Kohlenstoffatomen im Niederalkylrest oder $R_4$ und $R_5$ zusammen auch Alkylen mit 3 bis 4 Kohlenstoffatomen, $R_7$ Wasserstoff, und einer der Reste $A_1$ und $A_2$ einen Rest der Formel

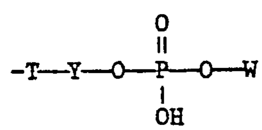
(II)

bedeuten, worin T für HN oder O steht, Y gegebenenfalls substituiertes Niederalkylen oder einen Rest einer der Formeln

$$Y_1—COO—Y_2 \qquad (IIIa)$$

$$Y_1—OOC—Y_2 \qquad (IIIb)$$

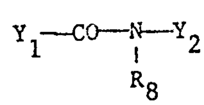
(IIIc)

oder

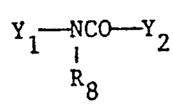
(IIId),

bedeutet, worin $Y_1$ und $Y_2$ je für gegebenenfalls substituiertes Niederalkylen und $R_8$ für Wasserstoff stehen, W eine Alkylgruppe mit 10 bis 25 Kohlenstoffatomen, die in 2-Stellung eine Hydroxygruppe, Alkanoyloxygruppe, Amino oder Alkanoylaminogruppe trägt, darstellen und der andere der Reste $A_1$ und $A_2$ Hydroxy, Niederalkoxy, Amino, Niederalkylamino oder im Niederalkylrest gegebenenfalls durch Hydroxy, Carboxy oder Aminogruppen substituiertes Aminocarbonylniederalkylamino ist, und/oder ihre Salze.

8. Verfahren nach einem der Ansprüche 1, 6 und 7, dadurch gekennzeichnet, dass man Verbindungen der Formel I, worin Y für gegebenenfalls durch $C_{1-4}$-Alkyl substituiertes $C_{1-4}$-Alkylen steht, das durch Carbonyloxy oder Carbonylimino unterbrochen sein kann, und/oder ihre Salze herstellt.

9. Verfahren nach Anspruch 1, dadurch gekennzeichnet, dass man Verbindungen der Formel I gemäss Patentanspruch 1 herstellt, worin X Carbonyl, $R_1$ Niederalkyl mit 1 bis 3 Kohlenstoffatomen oder Phenyl, $R_2$, $R_4$ und $R_6$ Wasserstoff, $R_3$ Wasserstoff oder Niederalkyl mit 1 bis 3 Kohlenstoffatomen, $R_5$ Wasserstoff, gegebenenfalls durch Hydroxy, Methoxy, Mercapto, Methylmercapto oder Halogen substituiertes Niederalkyl mit 1 bis 3 Kohlenstoffatomen, gegebenenfalls durch Hydroxy, Methoxy oder Halogen substituiertes Phenyl oder Phenylmethyl, ein oder zwei Azaatome enthaltendes Heterocyclyl oder Heterocyclylmethyl mit 5 Ringgliedern oder $R_4$ und $R_5$ zusammen auch Trimethylen, $R_7$ Wasserstoff und einer der Reste $A_1$ und $A_2$ einen Rest der Formel

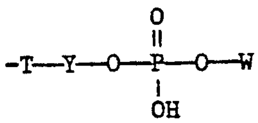

(II)

bedeuten, worin T für HN oder O steht, Y Niederalkylen mit 2 bis 3 Kohlenstoffatomen oder einen Rest der Formeln (IIIa) oder (IIIc)

$$Y_1 - COO - Y_2 \qquad \text{(IIIa)}$$

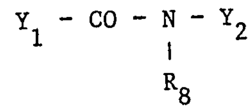

(IIIc)

bedeutet, worin $R_8$ für Wasserstoff steht und $Y_1$ und $Y_2$ unabhängig voneinander je gegebenenfalls durch Hydroxy, Niederalkoxy, Mercapto oder Niederalkylmercapto substituiertes Niederalkylen mit 1 bis 3 Kohlenstoffatomen oder Niederalkylen mit 1 bis 3 Kohlenstoffatomen bedeuten, das durch gegebenenfalls durch Hydroxy, Methoxy oder Halogen substituiertes Phenyl oder Phenylniederalkyl oder ein oder zwei Azaatome enthaltendes Heterocyclyl oder Heterocyclylniederalkyl mit 5 bis 6 Ringgliedern und 1 bis 3 Kohlenstoffatomen im Niederalkylrest substituiert ist, W eine Alkylgruppe mit 10 bis 25 Kohlenstoffatomen, die in 2-Stellung durch Hydroxy, Niederalkanoyloxy, Amino oder Alkanoylamino substituiert ist, und der andere der Reste $A_1$ und $A_2$ Hydroxy, Niederalkoxy, Amino, Niederalkylamino oder Aminocarbonylniederalkylamino ist, und/oder ihre Salze.

10. Verfahren nach einem der Ansprüche 1-9, dadurch gekennzeichnet, dass man die Ausgangsstoffe so wählt, dass man eine Verbindung der Formel I, worin jeweils im Falle asymmetrischer Substitution am C-$R_3$ die D-, am C-$R_5$ die L- und am C-N-$R_6$ die D-Konfiguration vorliegt, und/oder ein Salz dieser Verbindung erhält.

11. Verfahren nach Anspruch 1, dadurch gekennzeichnet, dass man N-Acetyl-desmethylmuramyl-L-alanyl-D-isoglutaminyl-L-alanin-2-(hexadecyloxy-hydroxyphosphoryloxy)-äthylamid und/oder dessen Salze herstellt.

12. Verfahren nach Anspruch 1, dadurch gekennzeichnet, dass man N-Acetyl-muramyl-L-alanyl-D-isoglutaminyl-L-alanin-2-(hexadecyloxy-hydroxyphosphoryloxy)-äthylamid und/oder dessen Salze herstellt.

13. Verfahren nach Anspruch 1, dadurch gekennzeichnet, dass man N-Acetyl-muramyl-L-alanyl-D-isoglutaminyl-L-alanin-2-(tetradecyloxy-hydroxyphosphoryloxy)-äthylamid und/oder dessen Salze herstellt.

14. Verfahren nach Anspruch 1, dadurch gekennzeichnet, dass man N-Acetyl-muramyl-L-alanyl-D-isoglutaminyl-L-alanin-2-[(3'R)-hydroxy-(2'S)-palmitoylamino-4't-octadecenyloxy-hydroxyphosphoryloxy]-äthylamid und/oder dessen Salze herstellt.

15. Verfahren nach Anspruch 1, dadurch gekennzeichnet, dass man N-Acetyl-muramyl-L-α-aminobutyryl-D-isoglutaminyl-L-alanin-2-[(3'R)-hydroxy-(2'S)-palmitoylamino-4't-octadecenyloxy-hydroxyphosphoryloxy]-äthylamid und/oder dessen Salze herstellt.

16. Verfahren nach Anspruch 1, dadurch gekennzeichnet, dass man N-Acetyl-muramyl-L-α-aminobutyryl-D-isoglutaminyl-L-alanin-2-[(3'R)-hydroxy-(3'S)-palmitoylamino-octadecyloxy-hydroxyphosphoryloxy]-äthyl-amid und/oder dessen Salze herstellt.

17. Verfahren nach Anspruch 1, dadurch gekennzeichnet, dass man N-Acetyl-muramyl-L-alanyl-D-isoglutaminyl-L-alanin-2-(cholest-5-en-3β-oxy-hydroxyphosphoryloxy)-äthylamid und/oder dessen Salze herstellt.

18. Verfahren nach Anspruch 1, dadurch gekennzeichnet, dass man N-Benzoyl-muramyl-L-alanyl-D-isoglutaminyl-L-alanin-2-(hexadecyloxy-hydroxyphosphoryloxy)-äthylamid herstellt und/oder dessen Salze.

19. Verfahren nach Anspruch 1, dadurch gekennzeichnet, dass man eine Verbindung auswählt aus der Gruppe N-Acetyl-muramyl-L-alanyl-D-isoglutamin-2-(hexadecyloxy-hydroxyphosphoryloxy)-äthylamid, N-Acetyl-desmethylmuramyl-L-alanyl-D-isoglutamin-2-(hexadecyloxy-hydroxyphosphoryloxy)-äthylamid, N-Acetyl-muramyl-L-alanyl-D-isoglutaminyl-oxymethylcarbonsäure-2-(hexadecyloxy-hydroxyphosphoryloxy)-äthylamid, N-Acetyl-muramyl-L-alanyl-D-isoglutamin-2-[(3'R)-hydroxy-(2'S)-palmitoylamino-4't-octadecenyloxy-hydroxyphosphoryloxy]-äthylamid, N-Acetyl-desmethylmuramyl-L-alanyl-D-isoglutamin-2-[(3'R)-hydroxy-(2'S)-palmitoylamino-4't-octadecenyloxy-hydroxyphosphoryloxy]-äthylamid, N-Acetyl-desmethylmuramyl-L-alanyl-D-isoglutaminyl-L-alanin-2-[(3'R)-hydroxy-(2'S)-palmitoylamino-4't-octadecenyloxy-hydroxyphosphoryloxy]-äthylamid, N-Acetyl-muramyl-L-alanyl-D-isoglutaminyl-oxymethylcarbonsäure-2-[(3'R)-hydroxy-(2'S)-palmitoylamino-4't-octadecenyloxy-hydroxyphosphoryloxy]-äthylamid und/oder ein Salz dieser Verbindungen herstellt.

20. Verfahren nach Anspruch 1, dadurch gekennzeichnet, dass man immunpotenzierend wirksame Verbindungen der Formel I gemäss einem der Patentansprüche 1 bis 16 und/oder ihre Salze herstellt.

21. Verfahren nach einem der Ansprüche 1 bis 20, dadurch gekennzeichnet, dass man zur Abspaltung von Schutzgruppen mit einer Säure oder mit Wasserstoff in Gegenwart eines Katalysators behandelt.

22. Verfahren nach einem der Ansprüche 1 bis 20, dadurch gekennzeichnet, dass man als Verbindungen mit einer oder mehreren reaktionsfähigen veresterten Hydroxygruppen Verbindungen verwendet, worin die Hydroxygruppe(n) mit einer starken anorganischen Säure oder einer Sulfonsäure verestert ist (sind).

23. Verfahren nach einem der Ansprüche 1 bis 20, dadurch gekennzeichnet, dass man als Verbindungen mit einer oder mehreren reaktionsfähigen veresterten Hydroxygruppen Verbindungen verwendet, worin die Hydroxygruppe(n) mit einer Halogenwasserstoffsäure verestert ist (sind).

24. Verfahren nach einem der Ansprüche 1 bis 20, dadurch gekennzeichnet, dass man als Verbindungen mit einer oder mehreren Hydroxyschutzgruppen Verbindungen verwendet, worin die Hydroxygruppe(n) mit Acyl-, Aroyl-, oder von Kohlensäurederivaten sich ableitenden Resten oder mit in α-Stellung verzweigten Alkylresten oder mit α-Mono-, -Di- oder -Triarylniederalkylresten oder mit acetalbildenden Resten geschützt ist (sind).

25. Verfahren nach Anspruch 24, dadurch gekennzeichnet, dass man als Verbindungen mit einer oder mehreren Hydroxyschutzgruppen Verbindungen verwendet, worin die Hydroxygruppe(n) mit Niederalkanoyl-, Benzoyl-, Benzyloxycarbonyl-, Niederalkoxycarbonyl-, tert.Butyl- oder mit gegebenenfalls substituierten Benzyl-, Triphenylmethyl- oder Tetrahydropyranylresten geschützt ist (sind).

26. Verfahren nach einem der Ansprüche 1 bis 20, dadurch gekennzeichnet, dass man als Verbindungen mit einer oder mehreren Carboxyschutzgruppen Verbindungen verwendet, worin die Carboxygruppe(n) mit tert.Butyl-, Benzyl- oder mit gegebenenfalls durch Halogen oder Niederalkoxy substituierten Triphenylmethyl- oder Benzhydrylresten geschützt ist (sind).

27. Verfahren nach einem der Ansprüche 1 bis 20, dadurch gekennzeichnet, dass man als Verbindungen mit einer oder mehreren aktivierten Carbonsäuregruppen Verbindungen verwendet, worin die Carbonsäuregruppe(n) als Anhydrid, Azid, aktiviertes Amid oder als aktivierter Ester vorliegt (vorliegen).

28. Verfahren nach Anspruch 27, dadurch gekennzeichnet, dass man als Säureanhydride solche mit Kohlensäureniederalkylester, als Säureamide Imidazolide oder Isooxazolide und als aktivierte Ester Cyan- oder Carboxymethylester, Acetylaminoäthylthioester, p-Nitro- oder 2,4,5-Trichlor-phenylester, N-Hydroxy-succinimid-, N-Hydroxy-phthalimid- oder N-Hydroxy-piperidinester, 8-Hydroxy-chinolinester, Methoxyäthylthioester oder durch Umsetzung mit Carbodiimid unter Zusatz von N-Hydroxy-succinimid oder einem 1-Hydroxybenzotriazol oder 3-Hydroxy-4-oxo-3,4-dihydro-benzo-[d]-1,2,3-triazin erhaltene Ester verwendet.

29. Verfahren zur Herstellung eines pharmazeutischen Präparats, dadurch gekennzeichnet, dass man eine Verbindung, die gemäss einem der Ansprüche 1 bis 28 erhalten wurde, mit einem Antibiotikum und einem pharmazeutisch verwendbaren Trägermaterial mischt.

**Claims** (for the Contracting States : BE, CH, DE, FR, GB, IT, LI, LU, NL, SE)

1. A phosphorylmuramyl peptide of the general formula

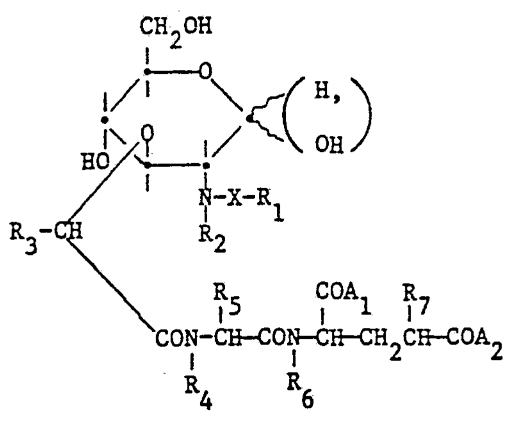

(I)

wherein X is carbonyl or carbonyloxy, $R_1$ is alkyl or aryl, each unsubstituted or substituted, $R_2$, $R_4$ and $R_6$ are hydrogen or lower alkyl, $R_3$ is hydrogen or lower alkyl, $R_5$ is hydrogen, lower alkyl, free or functionally modified hydroxy-lower alkyl, free or functionally modified mercapto-lower alkyl, amino-lower alkyl which is unsubstituted or substituted, cycloalkyl, cycloalkyl-lower alkyl, or is aryl or aralkyl, each unsubstituted or substituted, or is nitrogen-containing heterocyclyl or heterocyclyl-lower alkyl, or $R_4$ and $R_5$ together are also $C_3$-$C_4$ alkylene, $R_7$ is hydrogen or free, esterified or amidated carboxyl, and one of $A_1$ and $A_2$ is a radical of the formula

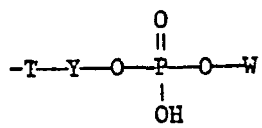

(II)

wherein T is NH or O, Y is an alkylene group which is unsubstituted or substituted and may also be interrupted by one or two oxycarbonyl and/or iminocarbonyl groups, and W is an aliphatic radical or a cycloalkyl or cycloalkenyl radical, each containing more than 6 carbon atoms, and the other radical $A_1$ or $A_2$ is free or etherified hydroxy, amino, lower alkylamino, or is aminocarbonyl-lower alkylamino which is unsubstituted or substituted in the lower alkyl moiety, with the term « lower » employed above denoting a radical containing up to and including 7 carbon atoms, or a salt thereof, with the exception of those compounds disclosed in European patent application 0 025 495, i. e. of muramyl peptides of the formula

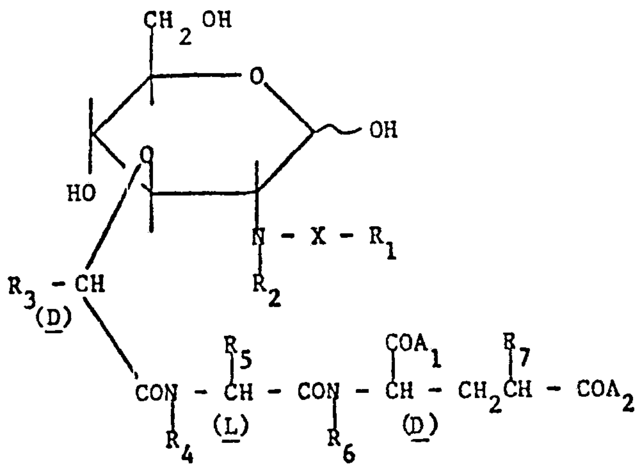

(Ia)

wherein X is carbonyl or carbonyloxy, $R_1$ is alkyl or aryl, each unsubstituted or substituted, $R_2$, $R_4$ and $R_6$ are hydrogen or lower alkyl, $R_3$ is hydrogen or lower alkyl, $R_5$ is hydrogen, lower alkyl, free or functionally modified hydroxy-lower alkyl, free or functionally modified mercapto-lower alkyl, amino-lower alkyl which is unsubstituted or substituted, cycloalkyl, cycloalkyl-lower alkyl, or is aryl or aralkyl, each unsubstituted or substituted, or is nitrogen-containing heterocyclyl or heterocyclyl-lower alkyl, or $R_4$ and $R_5$ together are also $C_3$-$C_4$ alkylene, $R_7$ is hydrogen or free, esterified or amidated carboxyl, and one of $A_1$ and $A_2$ is a radical of the formula

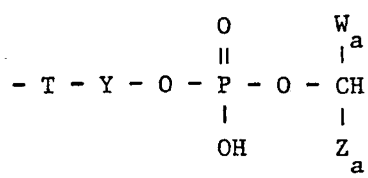

(IIa),

wherein T is HN or O, Y is an alkylene group which is unsubstituted or substituted and may be interrupted by one or two oxycarbonyl and/or iminocarbonyl groups, $W_a$ is hydrogen, and $Z_a$ is a 1,2-dihydroxyethyl or 2-hydroxyethyl group, in which at least one hydroxyl group is esterified with an unsaturated or saturated aliphatic $C_{12}$-$C_{90}$ carboxylic acid or etherified with an unsatured or saturated aliphatic $C_{10}$-$C_{22}$ alcohol, or $W_a$ and $Z_a$ are each a hydroxymethyl group which is esterified with an unsaturated or saturated aliphatic $C_{12}$-$C_{90}$ carboxylic acid or etherified with an unsaturated or saturated aliphatic $C_{10}$-$C_{22}$ alcohol, and the other radical $A_1$ or $A_2$ is free or etherified hydroxy, amino, lower alkylamino or carboxamido-lower alkylamino, and salts thereof, with the term « lower » denoting radicals containing up to and including 7 carbon atoms, and also with the exception of muramyl peptides of formula Ia as indicated above, wherein X is carbonyl or carbonyloxy, $R_1$ is lower $C_1$-$C_3$ alkyl or phenyl, $R_2$, $R_4$ and $R_6$ are hydrogen, $R_3$ is hydrogen or lower $C_1$-$C_3$ alkyl, $R_5$ is hydrogen or lower alkyl, $R_7$ is hydrogen, $A_1$ is

amino, lower alkylamino, hydroxy or lower alkoxy, and $A_2$ is a radical of formula IIa as indicated above, wherein T is NH or O, Y is lower $C_2$-$C_3$ alkylene or a radical of the formula

$$—CH_2—CO—NH—CH_2—CH_2—$$

$W_a$ is hydrogen and $Z_a$ is a 1,2-dihydroxyethyl or 2-hydroxyethyl group wherein one or two hydroxyl groups are esterified with identical or different $C_{16}$-$C_{22}$ alkanecarboxylic acids which are unsaturated or mono- or disaturated, or are etherified with a $C_{12}$-$C_{18}$ alkanol which is unsaturated or mono- or disaturated, or in which two vicinal hydroxyl groups are formally ketalised with a bis-lower alkyl ketone to form a dioxolane ring or are acetalised with an unsubstituted aliphatic aldehyde containing up to 20 carbon atoms, or wherein $W_a$ and $Z_a$ are each a hydroxymethyl group which is esterified with a $C_{16}$-$C_{22}$ alkanecarboxylic acid which is unsaturated or mono- or disaturated, or etherified with a $C_{12}$-$C_{18}$ alkanol which is unsaturated or mono- or disaturated, and salts thereof, with the term « lower » denoting radicals containing up to and including 7 carbon atoms, and also with the exception of N-acetylmuramyl-1-alanyl-D-isoglutaminyl-L-alanine 2-(1',2'-hexadecylidene-sn-glycero-3'-hydroxyphosphoryloxy)ethylamide and N-acetyldesmethylmuramyl-L-alanyl-D-isoglutaminyl-L-alanine 2-(1',2'-hexadecylidene-sn-glycero-3'-hydroxyphosphoryloxy)ethylamide, and salts thereof.

2. A compound of formula I according to claim 1, wherein W is alkyl or alkenyl containing up to 30 carbon atoms which is unsubstituted or substituted by hydroxy, lower alkoxy, lower alkanoyloxy, halogen, amino, alkanoylamino or oxo, or a salt thereof.

3. A compound of formula I according to claim 1, wherein X is carbonyl, $R_1$ is lower $C_1$-$C_7$ alkyl or phenyl, $R_2$, $R_4$, $R_6$ and $R_7$ are hydrogen, $R_3$ is hydrogen or lower alkyl, $R_5$ is hydrogen, lower alkyl, hydroxymethyl, mercaptomethyl, 1-hydroxyethyl, 2-methylthioethyl, phenylmethyl, p-hydroxyphenylmethyl, 4-aminobutyl, 4-imidazolylmethyl, or 3-indolylmethyl, or $R_4$ and $R_5$ together are also trimethylene, wherein $A_1$ is hydroxy, lower alkoxy, amino, lower alkylamino or aminocarbonyl-lower alkylamino and $A_2$ is a radical of the formula

$$-T-Y-O-\underset{OH}{\underset{|}{\overset{O}{\overset{\|}{P}}}}-O-W \qquad (II)$$

wherein T is NH or O and W is an alkyl or alkenyl group which is unsubstituted or substituted by hydroxy, lower alkoxy, lower alkanoyloxy, amino, lower alkylamino or alkanoylamino containing up to 25 carbon atoms, or is a $C_{10}$-$C_{30}$ cycloalkyl or $C_{10}$-$C_{30}$ cycloalkenyl radical, Y is ethylene or a radical of the formula

$$Y_1 - COO - Y_2 \qquad (IIIa)$$

or

$$Y_1 - CO - \underset{R_8}{\underset{|}{N}} - Y_2 \qquad (IIIc),$$

wherein $R_8$ is hydrogen and each of $Y_1$ and $Y_2$ independently of the other is lower $C_1$-$C_7$ alkylene which is unsubstituted or substituted by hydroxy, lower alkoxy, mercapto, methylthio, phenyl, 4-imidazolyl or 3-indolyl, or a salt thereof.

4. A compound formula I according to claim 1, wherein X is carbonyl, $R_1$ is lower $C_1$-$C_3$ alkyl, $R_2$, $R_4$, $R_6$ and $R_7$ are hydrogen, $R_3$ is hydrogen or methyl, $R_5$ is hydrogen or lower alkyl, $A_1$ is amino and $A_2$ is a radical of the formula

$$-T-Y-O-\underset{OH}{\underset{|}{\overset{O}{\overset{\|}{P}}}}-O-W \qquad (II)$$

wherein T is NH, W is a $C_{10}$-$C_{25}$ alkyl or $C_{10}$-$C_{25}$ alkenyl group which is unsubstituted or substituted by hydroxy, lower alkoxy, lower alkanoyloxy, amino, lower alkylamino or alkanoylamino containing up to 25 carbon atoms, or is a cholesteryl radical, and Y is ethylene or a radical of the formula

$$Y_1—CO—\underset{R_8}{\underset{|}{N}}—Y_2 \qquad (IIIc),$$

wherein $R_8$ is hydrogen and each of $Y_1$ and $Y_2$ independently of the other is lower alkylene, or a salt thereof.

5. A compound according to either claim 3 or claim 4, wherein the meanings for $A_1$ and $A_2$ are interchanged, or a salt thereof.

6. A compound of formula I according to claim 1, wherein X is carbonyl, $R_1$ is alkyl or aryl, each unsubstituted or substituted, $R_2$, $R_3$, $R_4$ and $R_6$ are hydrogen or lower alkyl, $R_5$ is hydrogen, lower alkyl which is unsubstituted or substituted by hydroxy, lower alkoxy, mercapto, lower alkylmercapto or halogen, or is cycloalkyl or cycloalkyl-lower alkyl, in each of which the cycloalkyl moiety contains 4 to 6 carbon atoms, or is phenyl or phenyl-lower alkyl, each unsubstituted or substituted, or is heterocyclyl or heterocyclyl-lower alkyl, each containing one or two aza atoms, or $R_4$ and $R_5$ together are also $C_3$-$C_4$ alkylene, $R_7$ is hydrogen and one of $A_1$ and $A_2$ is a radical of the formula

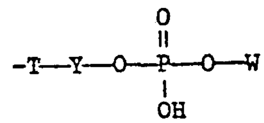
(II)

wherein T is NH or O, Y is alkylene which is unsubstituted or substituted and may also be interrupted by carbonyloxy or carbonylimino, and W is an alkyl group containing more than 6 carbon atoms which is unsubstituted or substituted by hydroxy, lower alkanoyloxy, amino, alkanoylamino or oxo, and the other radical $A_1$ or $A_2$ is hydroxy, lower alkoxy, amino or lower alkylamino, or is aminocarbonyl-lower alkylamino which is unsubstituted or substituted in the lower alkyl moiety by hydroxy, carboxy and/or amino, or a salt thereof.

7. A compound of formula I according to claim 1, wherein X is carbonyl, $R_1$ is lower alkyl which is unsubstituted or substituted by hydroxy, lower alkoxy or halogen, or is phenyl which is unsubstituted or substituted by hydroxy, lower alkoxy, lower alkyl or halogen, $R_2$, $R_4$ and $R_6$ are hydrogen, $R_3$ is hydrogen or lower alkyl, $R_5$ is hydrogen, lower $C_1$-$C_3$ alkyl which is unsubstituted or substituted by hydroxy, lower alkoxy, mercapto, lower alkylmercapto or halogen, or is cycloalkyl or cycloalkyl-lower alkyl wherein the lower alkyl moiety contains 1 to 3 carbon atoms, and in each of which cycloalkyl contains 4 to 6 carbon atoms, or is phenyl or phenyl-lower alkyl containing 1 to 3 carbon atoms in the lower alkyl moiety, each unsubstituted or substituted by hydroxy, lower alkoxy or halogen, or is heterocyclyl or heterocyclyl-lower alkyl containing 1 to 3 carbon atoms in the lower alkyl moiety, and each containing one or two aza atoms and having 5 or 6 ring members, or $R_4$ and $R_5$ together are also $C_3$-$C_4$ alkylene, $R_7$ hydrogen, and one of $A_1$ and $A_2$ is a radical of the formula

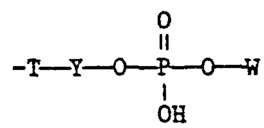
(II)

wherein T is HN or O, Y is lower alkylene which is unsubstituted or substituted or is a radical of the formula

$$Y_1—COO—Y_2 \quad \text{(IIIa)}$$

$$Y_1—OOC—Y_2 \quad \text{(IIIb)}$$

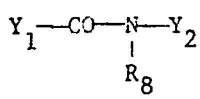
(IIIc)

or

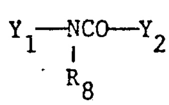
(IIId),

wherein each of $Y_1$ and $Y_2$ is lower alkylene which is unsubstituted or substituted and $R_8$ is hydrogen, W is a $C_{10}$-$C_{25}$ alkyl group carrying in the 2-position a hydroxyl group, an alkanoyloxy group, an amino group or an alkanoylamino group, and the other radical $A_1$ or $A_2$ is hydroxy, lower alkoxy, amino or lower alkylamino, or is aminocarbonyl-lower alkylamino which is unsubstituted or substituted in the lower alkyl moiety by hydroxy, carboxy or amino groups, or a salt thereof.

8. A compound according to any one of claims 1, 6 and 7, wherein Y is $C_1$-$C_4$ alkylene which is unsubstituted or substituted by $C_1$-$C_4$ alkyl and may be interrupted by carbonyloxy or carbonylimino.

9. A compound of formula I according to claim 1, wherein X is carbonyl, $R_1$ is lower $C_1$-$C_3$ alkyl or phenyl, $R_2$, $R_4$ and $R_6$ are hydrogen, $R_3$ is hydrogen or lower $C_1$-$C_3$ alkyl, $R_5$ is hydrogen, lower $C_1$-$C_3$

alkyl which is unsubstituted or substituted by hydroxy, methoxy, mercapto, methylmercapto or halogen, or is phenyl or phenylmethyl, each unsubstituted or substituted by hydroxy, methoxy or halogen, or is heterocyclyl or heterocyclylmethyl, each containing one or two aza atoms and having 5 ring members, or $R_4$ and $R_5$ together are also trimethylene, $R_7$ is hydrogen and one of $A_1$ and $A_2$ is a radical of the formula

$$-T-Y-O-\overset{\overset{\displaystyle O}{\|}}{\underset{\underset{\displaystyle OH}{|}}{P}}-O-W \qquad \text{(II)}$$

wherein T is HN or O, Y is lower $C_2$-$C_3$ alkylene, or is a radical of the formula (IIIa) or (IIIc)

$$Y_1 - COO - Y_2 \qquad \text{(IIIa)}$$

$$Y_1 - CO - \underset{\underset{\displaystyle R_8}{|}}{N} - Y_2 \qquad \text{(IIIc)}$$

wherein $R_8$ is hydrogen, and each of $Y_1$ and $Y_2$ independently of the other is lower $C_1$-$C_3$ alkylene which is unsubstituted or substituted by hydroxy, lower alkoxy, mercapto or lower alkylmercapto, or is lower $C_1$-$C_3$ alkylene which is substituted by phenyl or phenyl-lower alkyl, which may themselves be unsubstituted or substituted by hydroxy, methoxy or halogen ; or is lower $C_1$-$C_3$ alkylene which is substituted by heterocyclyl or heterocyclyl-lower alkyl containing 1 to 3 carbon atoms in the lower alkyl radical, and each containing one or two aza atoms and having 5 or 6 ring members, W is a $C_{10}$-$C_{25}$ alkyl group substituted in the 2-position by hydroxy, lower alkanoyloxy, amino or alkanoylamino, and the other radical $A_1$ or $A_2$ is hydroxy, lower alkoxy, amino, lower alkylamino or aminocarbonyl-lower alkylamino, or a salt thereof.

10. A compound of formula I according to any one of claims 1 to 9, wherein in each asymmetric substitution at C-$R_3$ the D-configuration, at C-$R_5$ the L-configuration and at C-$\underset{\underset{\displaystyle R_6}{|}}{N}$ the D-configuration is present, or a salt thereof.

11. N-Acetyldesmethylmuramyl-L-alanyl-D-isoglutaminyl-L-alanine 2-(hexadecyloxyhydroxyphosphoryloxy) ethylamide according to claim 1, or a salt thereof.

12. N-Acetylmuramyl-L-alanyl-D-isoglutaminyl-L-alanine 2-(hexadecyloxyhydroxyphosphoryloxy) ethylamide according to claim 1, or a salt thereof.

13. N-Acetylmuramyl-L-alanyl-D-isoglutaminyl-L-alanine 2-(tetradecyloxyhydroxyphosphoryloxy) ethylamide according to claim 1, or a salt thereof.

14. N-Acetylmuramyl-L-alanyl-D-isoglutaminyl-L-alanine 2-[(3'R)-hydroxy-(2'S)-palmitoylamino-4't-octadecenyloxyhydroxyphosphoryloxyl]ethylamide according to claim 1, or a salt thereof.

15. N-Acetylmuramyl-L-α-aminobutyryl-D-isoglutaminyl-L-alanine 2-[(3'R)-hydroxy-(2'S)-palmitoylamino-4't-octadecenyloxyhydroxyphosphoryloxy]ethylamide according to claim 1, or a salt there-of.

16. N-Acetylmuramyl-L-α-aminobutyryl-D-isoglutaminyl-L-alanine 2-[(3'R)-hydroxy-(2'S)-palmitoylaminooctadecyloxyhydroxyphosphoryloxy]ethylamide according to claim 1, or a salt thereof.

17. N-Acetylmuramyl-L-alanyl-D-isoglutaminyl-L-alanine 2-(cholest-5-ene-3β-oxyhydroxyphosphoryloxy)ethylamide according to claim 1, or a salt thereof.

18. N-Benzoylmuramyl-L-alanyl-D-isoglutaminyl-L-alanine 2-(hexadecyloxyhydroxyphosphoryloxy) ethylamide according to claim 1, or a salt thereof.

19. A compound selected from the group consisting of N-acetylmuramyl-L-alanyl-D-isoglutamine 2-(hexadecyloxyhydroxyphosphoryloxy)ethylamide, N-acetyldesmethylmuramyl-L-alanyl-D-isoglutamine 2-(hexadecyloxyhydroxyphosphoryloxy)-ethylamide, N-acetylmuramyl-L-alanyl-D-isoglutaminyloxymethylcarboxylic acid 2-(hexadecyloxyhydroxyphosphoryloxy)ethylamide, N-acetylmuramyl-L-alanyl-D-isoglutamine 2-[(3'R)-hydroxy-(2'S)-palmitoylamino-4't-octadecenyloxyhydroxyphosphoryloxy]ethylamide, N-acetyldesmethylmuramyl-L-alanyl-D-isoglutamine 2-[(3'R)-hydroxy-(2'S)-palmitoylamino-4't-octadecenyloxyhydroxyphosphoryloxy]ethylamide, N-acetyldesmethylmuramyl-L-alanyl-D-isoglutaminyl-L-alanine 2-[(3'R)-hydroxy-(2'S)-palmitoylamino-4't-octadecenyloxyhydroxyphosphoryloxy]ethylamide, N-acetylmuramyl-L-alanyl-D-isoglutaminyloxymethylcarboxylic acid [(3'R)-hydroxy-(2'S)-palmitoylamino-4't-octadecenyloxyhydroxyphosphoryloxy]-ethylamide according to claim 1, or a salt thereof.

20. An immunopotentiatingly active compound of formula I according to any one of claims 1 to 19, or a salt thereof.

21. A pharmaceutically acceptable composition containing a compound of formula I according to any one of claims 1 to 19, together with a pharmaceutically acceptable carrier.

22. Use of a compound of formula I according to any one of claims 1 to 19 for the preparation of a pharmaceutical composition.

23. A compound according to any one of claims 1 to 19 for use as medicament.

24. A compound according to any one of claims 1 to 19 for use as immunostimulating agent.

25. A pharmaceutical composition containing at least one compound according to any one of claims 1 to 19 and at least one antibiotic, together with a pharmaceutically acceptable carrier.

26. A muramyl peptide according to any one of claims 1 to 19 for use in a method of increasing the antibiotic activity of antibiotics, which method comprises administering said muramyl peptide and an antibiotic substantially together over a period of time (in the course of one day).

27. A process for the preparation of a compound according to claim 1, which comprises

a) reacting a compound of the formula

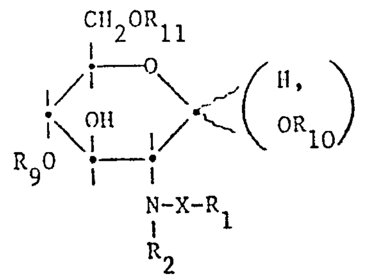

(V)

wherein X, $R_1$ and $R_2$ are as defined in claim 1, and any hydroxyl groups present are protected by an easily removable protecting group, and each of $R_9$, $R_{10}$ and $R_{11}$ is an easily removable protecting group, or a metal compound thereof, with a compound of the formula

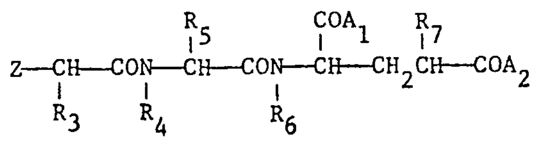

(VI)

wherein Z is a reactive esterified hydroxyl group, $R_3$, $R_4$, $R_5$, $R_6$, $R_7$, $A_1$ and $A_2$ are as defined in claim 1 and any hydroxyl groups present are protected by an easily removable protecting group, and removing protecting groups present,

b) condensing in a manner known per se a compound of the formula

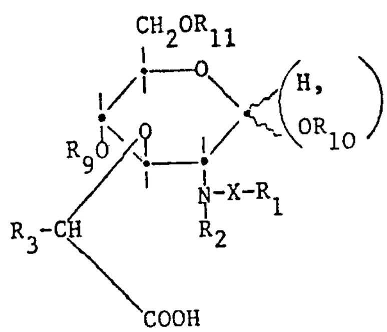

(VII)

wherein X, $R_1$, $R_2$ and $R_3$ are as defined in claim 1 and each of $R_9$, $R_{10}$ and $R_{11}$ is hydrogen or an easily removable protecting group, or a derivative thereof, with a compound of the formula

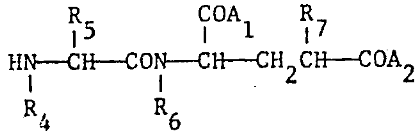

(VIII)

wherein $R_4$, $R_5$, $R_6$, $R_7$, $COA_1$ and $COA_2$ are as defined in claim 1, with the proviso that carboxyl groups and, if desired, free hydroxyl groups present in these radicals are protected by easily removable protecting groups, or with a derivative thereof, and removing protecting groups present,

c) condensing a compound of the formula

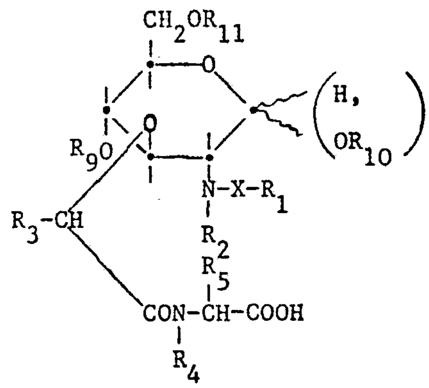

(IX)

wherein X, $R_1$, $R_2$, $R_3$, $R_4$ and $R_5$ are as defined in claim 1, with the proviso that free hydroxyl groups contained therein may be protected by an easily removable protecting group, and $R_9$, $R_{10}$ and $R_{11}$ are hydrogen or easily removable protecting groups, or a derivative thereof, with a compound of the formula

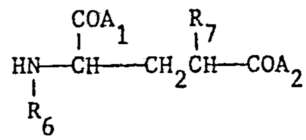

(X)

wherein $R_6$, $R_7$, $A_1$ and $A_2$ are as defined in claim 1, with the proviso that free carboxyl groups present in the radicals $R_7$, $COA_1$ and $COA_2$ are protected by easily removable protecting groups, and removing protecting groups present,

d) condensing in a manner known per se a compound of the formula

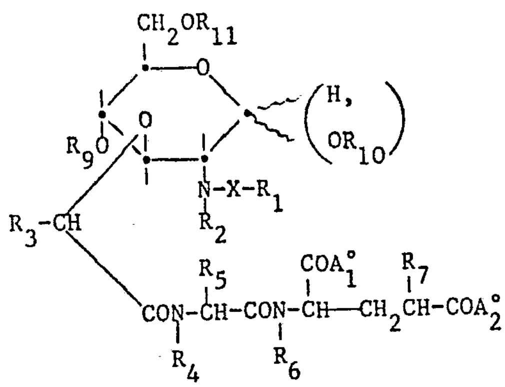

(XI)

wherein X, $R_1$, $R_2$, $R_3$, $R_4$, $R_5$, $R_6$ and $R_7$ are as defined in claim 1, $R_9$, $R_{10}$ and $R_{11}$ are hydrogen or an easily removable protecting group, and one of $A_1°$ and $A_2°$ is an activated hydroxyl group and the other is etherified hydroxy, amino, lower alkylamino or aminocarboyl-lower alkylamino, with a compound of the formula

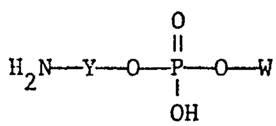

(XII)

wherein Y and W are as defined in claim 1, and removing any protecting groups present,

e) esterifying in a manner known per se a compound of the formula

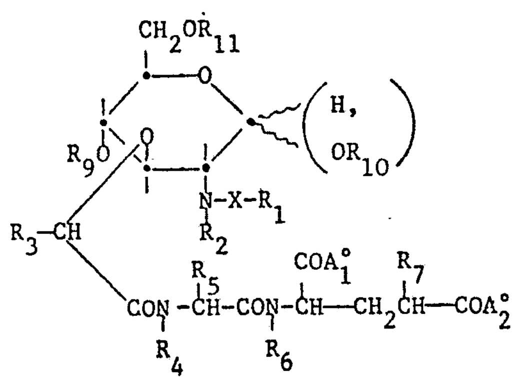

(XIa)

wherein X, $R_1$, $R_2$, $R_3$, $R_4$, $R_5$, $R_6$ and $R_7$ are as defined in claim 1, $R_9$, $R_{10}$ and $R_{11}$ are hydrogen or an easily removable protecting group and one of $A_1^o$ and $A_2^o$ is a hydroxyl group and the other is estherified hydroxy, amino, lower alkylamino or aminocarbonyl-lower alkylamino, with a compound of the formula

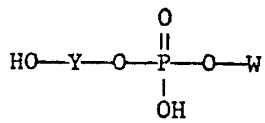

(XIIa)

wherein Y and W are as defined in claim 1, with the acid XIa or the alcohol XIIa being present in reactive form, and removing any protecting groups present,

f) in a compound of the formula

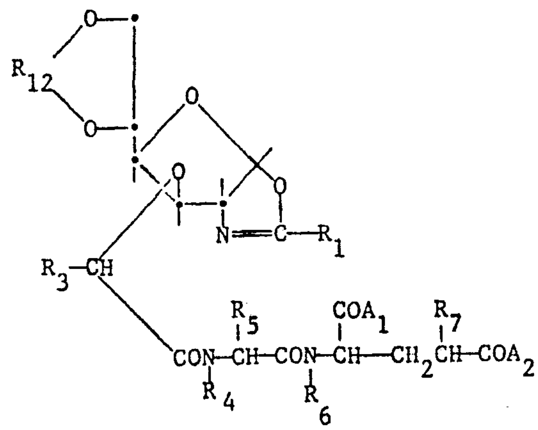

(XIII)

wherein $R_1$, $R_3$, $R_4$, $R_5$, $R_6$, $R_7$, $A_1$ and $A_2$ are as defined in claim 1 and $R_{12}$ is an alkylidene or cycloalkylidene group, opening the oxazoline and dioxolane rings by acid means and removing any protecting groups present,

g) condensing a compound of the formula

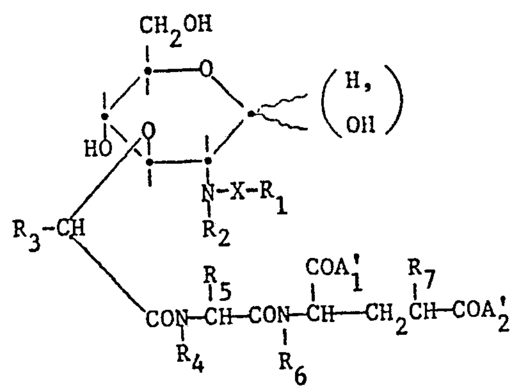

(XIV)

wherein one of $A_1'$ and $A_2'$ is a radical of the formula

$$—T—Y_1—M_1 \qquad (XV)$$

and the other radical $A_1'$ or $A_2'$ is etherified hydroxy or amino, lower alkylamino or aminocarbonyl-lower alkylamino, with a compound of the formula

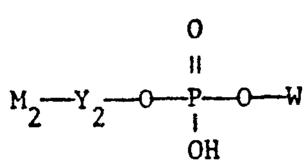

(XVI)

wherein X, $R_1$, $R_2$, $R_3$, $R_4$, $R_5$, $R_6$, $R_7$, T and W are as defined in claim 1, each of $Y_1$ and $Y_2$ is lower alkylene, which is unsubstituted or substituted, and hydroxyl groups present may be protected by easily removable protecting groups, and one of $M_1$ and $M_2$ is a free amino group or an activated derivative thereof, and the other is a carboxylic acid group or an activated derivative thereof, and removing any protecting groups present,

h) esterifying a compound of the formula

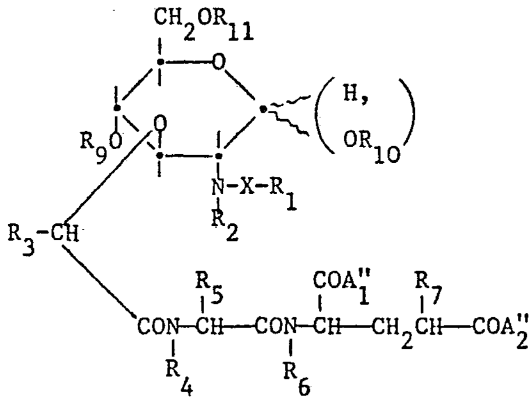

(XVII)

wherein one of $A_1''$ and $A_2''$ is a radical of the formula

$$—T—Y_1—M_3 \qquad \text{(XVIII)}$$

in a manner known per se with a compound of the formula

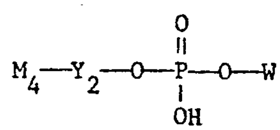

(XIX)

wherein X, $R_1$, $R_2$, $R_3$, $R_4$, $R_5$, $R_6$, $R_7$, T and W are as defined in claim 1, each of $Y_1$ and $Y_2$ is lower alkylene which is unsubstituted or substituted and any hydroxyl groups present are protected by easily removable protecting groups, $R_9$, $R_{10}$ and $R_{11}$ are easily removable protecting groups, and the other radical $A_1''$ or $A_2''$ is etherified hydroxy, amino, lower alkyl-amino or aminocarbonyl-lower alkylamino, and one of $M_3$ and $M_4$ is a free hydroxyl group and the other is a free carboxyl group, and one of $M_3$ and $M_4$ may be present in reactive form, and removing any protecting groups present,

i) reacting a compound of the formula

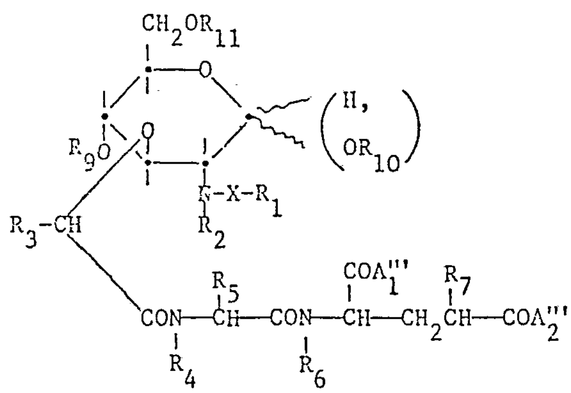

(XX)

wherein X, $R_1$, $R_2$, $R_3$, $R_4$, $R_5$, $R_6$ and $R_7$ are as defined in claim 1, and any hydroxyl groups present are protected by an easily removable protecting group, $R_9$, $R_{10}$ and $R_{11}$ are easily removable protecting groups and one of $A_1''$ and $A_2''$ is —T—Y—OH, wherein Y and T are as defined in claim 1 and the other radical $A_1''$ or $A_2''$ is free or etherified hydroxy amino or lower alkylamino, or is aminocarbonyl-lower alkylamino which is unsubstituted or substituted in the lower alkyl moiety with a compound affording the radical of the formula

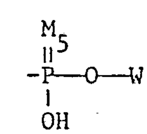

(XXI)

wherein $M_5$ is an electron pair or oxo and W is as defined in claim 1, and if $M_5$ is an electron pair oxidising

the reaction product with a weak oxidising agent, and removing protecting groups present,

k) reacting a compound of the formula

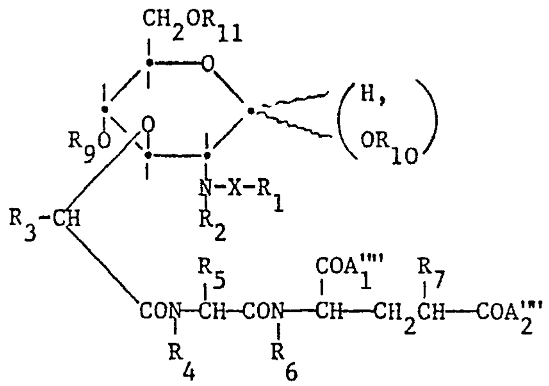

(XXIII)

wherein X, $R_1$, $R_2$, $R_3$, $R_4$, $R_5$, $R_6$ and $R_7$ are as defined in claim 1, and any hydroxyl groups present are protected by an easily removable protecting group, $R_9$, $R_{10}$ and $R_{11}$ are easily removable protecting groups and one of $A_1''''$ and $A_2''''$ is

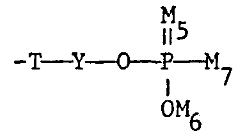

(XXIV)

and the other is free or etherified hydroxy, amino, lower alkylamino or aminocarbonyl-lower alkylamino, in which formula XXIV T and Y are as defined in claim 1, $M_5$ is an electron pair or oxo, $M_6$ is hydrogen or an easily removable protecting group and $M_7$ is a hydroxyl group which may be present in reactive form, with a compound of the formula

HO—W (XXV)

wherein W is as defined in claim 1, and if $M_5$ is an electron pair oxidising the reactive product with a weak oxidising agent, and removing protecting groups present, or

l) in a compound of formula I in which one or more functional groups are protected by protecting groups, removing said protecting groups, and if desired, after carrying out one of the processes a)-l), converting a resultant compound of formula I into a salt thereof.

28. A compound according to claim 27, wherein the starting materials are chosen such that a compound according to claim 10 is obtained.

29. A compound obtainable in accordance with a process as claimed in claim 27, or a salt thereof.

**Claims** (for the Contracting State AT)

1. A process for the preparation of a muramyl peptide of the general formula

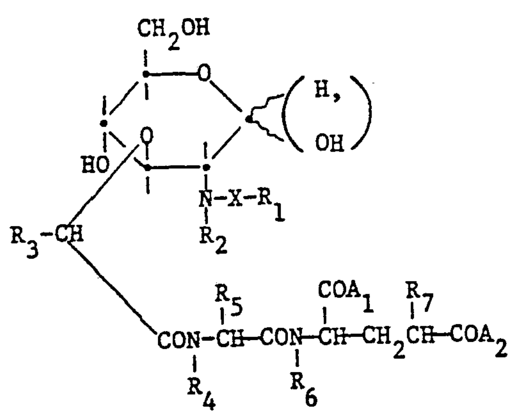

(I)

wherein X is carbonyl or carbonyloxy, $R_1$ is alkyl or aryl, each unsubstituted or substituted, $R_2$, $R_4$ and $R_6$ are hydrogen or lower alkyl, $R_3$ is hydrogen or lower alkyl, $R_5$ is hydrogen, lower alkyl, free or functionally modified hydroxy-lower alkyl, free or functionally modified mercapto-lower alkyl, amino-lower alkyl which

is unsubstituted or substituted, cycloalkyl, cycloalkyllower alkyl, or is aryl or aralkyl, each unsubstituted or substituted, or nitrogen-containing heterocyclyl or heterocyclyl-lower alkyl, or $R_4$ and $R_5$ together are also $C_3$-$C_4$ alkylene, $R_7$ is hydrogen or free, esterified or amidated carboxyl, and one of $A_1$ and $A_2$ is a radical of the formula

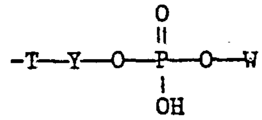

(II)

wherein T is NH or O, Y is an alkylene group which is unsubstituted or subsituted and may also be interrupted by one or two oxycarbonyl and/or iminocarbonyl groups, and W is an aliphatic radical or a cycloalkyl or cycloalkenyl radical, each containing more than 6 carbons atoms, and the other radical $A_1$ or $A_2$ is free or etherified hydroxy, amino, lower alkylamino, or is aminocarbonyl-lower alkylamino which is unsubstituted or substituted in the lower alkyl moiety, with the term « lower » employed above denoting a radical containing up to and including 7 carbon atoms, or a salt thereof, with the exception of those compounds disclosed in European patent application 0 025 495, i. e. of muramyl peptides of the formula

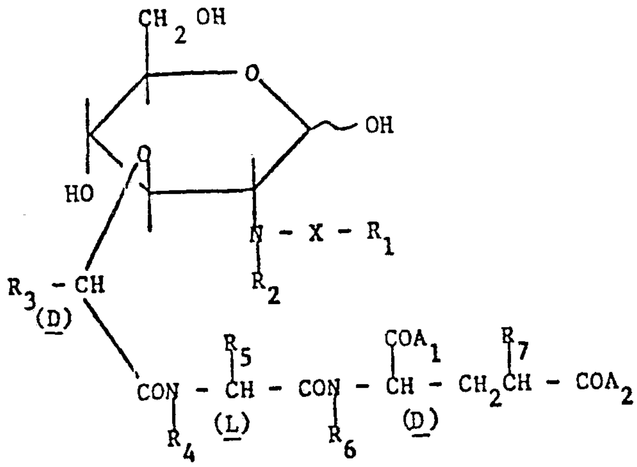

(Ia)

wherein X is carbonyl or carbonyloxy, $R_1$ is alkyl or aryl, each unsubstituted or substituted, $R_2$, $R_4$ and $R_6$ are hydrogen or lower alkyl, $R_3$ is hydrogen or lower alkyl, $R_5$ is hydrogen, lower alkyl, free or functionnally modified hydroxy-lower alkyl, free or functionally modified mercapto-lower alkyl, amino-lower alkyl which is unsubstituted or substituted, cycloalkyl, cycloalkyllower alkyl, or is aryl or aralkyl, each unsubstituted or substituted, or is nitrogen-containing heterocyclyl or heterocyclyl-lower alkyl, or $R_4$ and $R_5$ together are also $C_3$-$C_4$ alkylene, $R_7$ is hydrogen or free, esterified or amidated carboxyl, and one of $A_1$ and $A_2$ is a radical of the formula

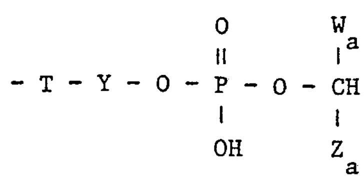

(IIa)

wherein T is HN or O, Y is an alkylene group which is unsubstituted or substituted and may be interrupted by one or two oxycarbonyl and/or iminocarbonyl groups, $W_a$ is hydrogen, and $Z_a$ is a 1,2-dihydroxyethyl or 2-hydroxyethyl group, in which at least one hydroxyl group is esterified with an unsaturated or saturated aliphatic $C_{12}$-$C_{90}$ carboxylic acid or etherified with an unsaturated or saturated aliphatic $C_{10}$-$C_{22}$ alcohol, or $W_a$ and $Z_a$ are each a hydroxymethyl group which is esterified with an unsaturated or saturated aliphatic $C_{12}$-$C_{90}$ carboxylic acid or etherified with an unsaturated or saturated aliphatic $C_{10}$-$C_{22}$ alcohol, and the other radical $A_1$ or $A_2$ is free or etherified hydroxy, amino, lower alkylamino or carboxamido-lower alkylamino, and salts thereof, with the term « lower » denoting radicals containing up to and including 7 carbon atoms, and also with the exception of muramyl peptides of formula Ia as indicated above, wherein X is carbonyl or carbonyloxy, $R_1$ is lower $C_1$-$C_3$ alkyl or phenyl, $R_2$, $R_4$ and $R_6$ are hydrogen, $R_3$ is hydrogen or lower $C_1$-$C_3$alkyl, $R_5$ is hydrogen or lower alkyl, $R_7$ is hydrogen, $A_1$ is amino, lower alkylamino, hydroxy or lower alkoxy, and $A_2$ is a radical of formula IIa as indicated above, wherein T is NH or O, Y is lower $C_2$-$C_3$ alkylene or a radical of the formula

$$—CH_2—CO—NH—CH_2—CH_2—$$

$W_a$ is hydrogen and $Z_a$ is a 1,2-dihydroxyethyl or 2-hydroxyethyl group wherein one or two hydroxyl

groups are esterified with identical or different $C_{16}$-$C_{22}$ alkanecarboxylic acids which are unsaturated or mono- or disaturated, or are etherified with a $C_{12}$-$C_{18}$ alkanol which is unsuaturated or mono- or disaturated, or in which two vicinal hydroxyl groups are formally ketalised with a bis-lower alkyl ketone to form a dioxolane ring or are acetalised with an unsubstituted aliphatic aldehyde containing up to 20 carbon atoms, or wherein $W_a$ and $Z_a$ are each a hydroxymethyl group which is esterified with a $C_{16}$-$C_{22}$ alkanecarboxylic acid which is unsaturated or mono- or disaturated, or etherified with a $C_{12}$-$C_{18}$ alkanol which is unsaturated or mono- or disaturated, and salts thereof, with the term « lower » denoting radicals containing up to and including 7 carbon atoms, and also with the exception of N-acetylmuramyl-1-alanyl-D-isoglutaminyl-L-alanine 2-(1',2'-hexadecylidene-sn-glycero-3'-hydroxyphosphoryloxy) ethylamide and N-acetyldesmethylmuramyl-L-alanyl-D-isoglutaminyl-L-alanine 2-(1',2'-hexadecylidene sn-glycero-3'-hydroxyphosphoryloxy) ethylamide, and salts thereof, which comprises

a) reacting a compound of the formula

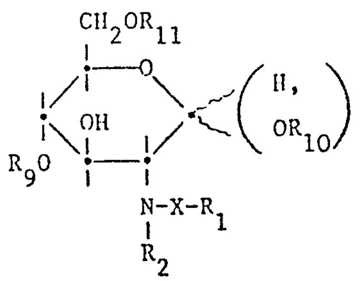

(V)

wherein X, $R_1$ and $R_2$ are as defined above, and any hydroxyl groups present are protected by an easily removable protecting group, and each of $R_9$, $R_{10}$ and $R_{11}$ is an easily removable protecting group, or a metal compound thereof, with a compound of the formula

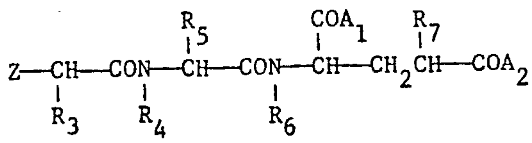

(VI)

wherein Z is a reactive esterified hydroxyl group, $R_3$, $R_4$, $R_5$, $R_6$, $R_7$, $A_1$ and $A_2$ are as defined in claim 1 and any hydroxyl groups present are protected by an easily removable protecting group, and removing protecting groups present,

b) condensing in a manner know per se a compound of the formula

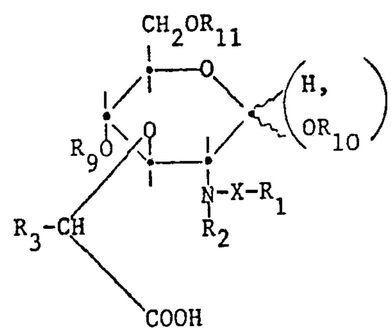

(VII)

wherein X, $R_1$, $R_2$ and $R_3$ are as defined above and each of $R_9$, $R_{10}$ and $R_{11}$ is hydrogen or an easily removable protecting group, or a derivative thereof, with a compound of the formula

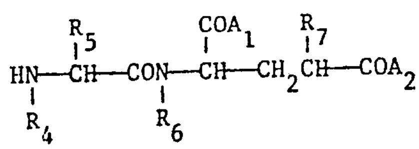

(VIII)

wherein $R_4$, $R_5$, $R_6$, $R_7$, $COA_1$ and $COA_2$ are as defined above, with the proviso that carboxyl groups and, if desired, free hydroxyl groups present in these radicals are protected by easily removable protecting groups, or with a derivative thereof, and removing protecting groups present,

c) condensing a compound of the formula

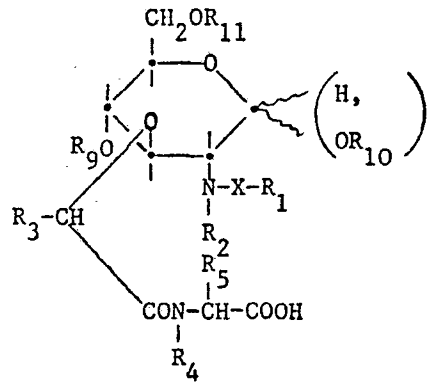

(IX)

wherein X, $R_1$, $R_2$, $R_3$, $R_4$ and $R_5$ are as defined above, with the proviso that free hydroxyl groups contained therein may be protected by an easily removable protecting group, and $R_9$, $R_{10}$ and $R_{11}$ are hydrogen or easily removable protecting groups, or a derivative thereof, with a compound of the formula

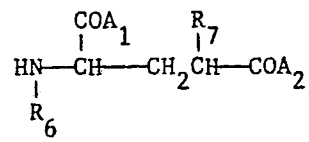

(X)

wherein $R_6$, $R_7$, $A_1$ and $A_2$ are as defined above, with the proviso that free carboxyl groups present in the radicals $R_7$, $COA_1$ and $COA_2$ are protected by easily removable protecting groups, and removing protecting groups present,

d) condensing in a manner known per se a compound of the formula

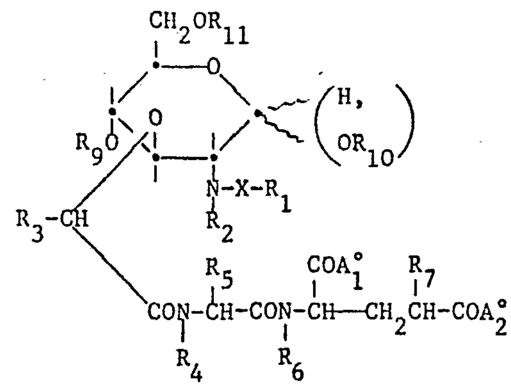

(XI)

wherein X, $R_1$, $R_2$, $R_3$, $R_4$, $R_5$, $R_6$ and $R_7$ are as defined above, $R_9$, $R_{10}$ and $R_{11}$ are hydrogen or an easily removable protecting group, and one of $A^o_1$ and $A^o_2$ is an activated hydroxyl group and the other is etherified hydroxy, amino, lower alkylamino or aminocarbonyl-lower alkylamino, with a compound of the formula

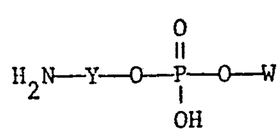

(XII)

wherein Y and W are as defined above, and removing any protecting groups present,

e) esterifying in a manner known per se a compound of the formula

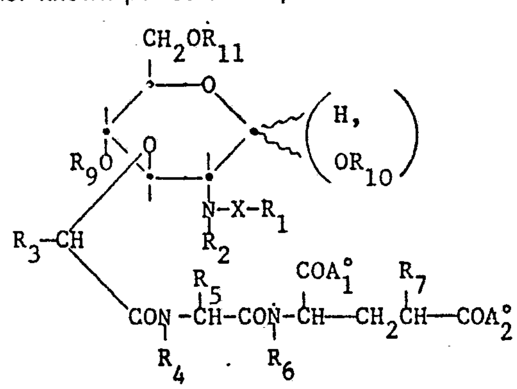

(XIa)

wherein X, $R_1$, $R_2$, $R_3$, $R_4$, $R_5$, $R_6$ and $R_7$ are as defined above, $R_9$, $R_{10}$ and $R_{11}$ are hydrogen or an easily removable protecting group and one of $A_1^o$ and $A_2^o$ is a hydroxyl group and the other is etherified hydroxy, amino, lower alkylamino or aminocarbonyl-lower alkylamino, with a compound of the formula

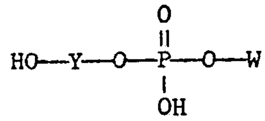

(XIIa)

wherein Y an W are as defined above, with the acid XIa or the alcohol XIIa being present in reactive form, and removing any protecting groups present,

f) in a compound of the formula

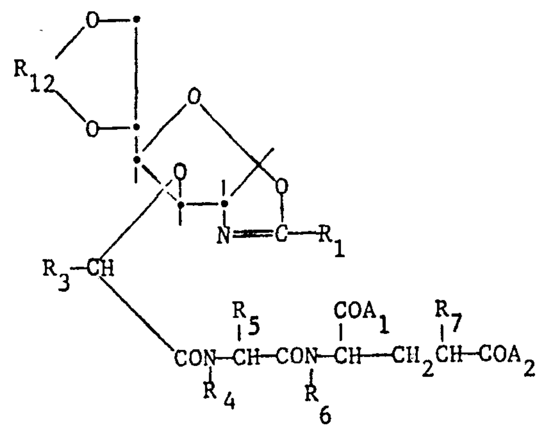

(XIII)

wherein $R_1$, $R_3$, $R_4$, $R_5$, $R_6$, $R_7$, $A_1$, and $A_2$ are as defined above and $R_{12}$ is an alkylidene or cycloalkylidene group, opening the oxazoline and dioxolane rings by acid means and removing any protecting groups present,

g) condensing a compound of the formula

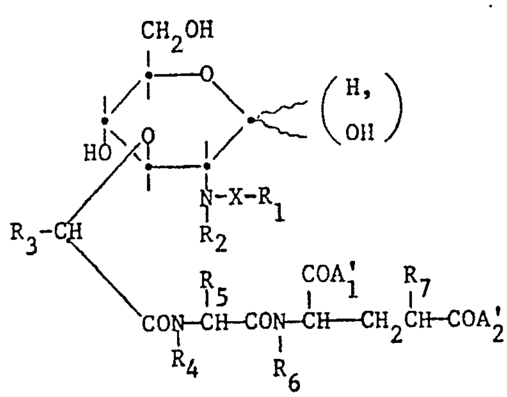

(XIV)

wherein one of $A_1'$ and $A_2'$ is a radical of the formula

$$—T—Y_1—M_1 \qquad (XV)$$

and the other radical $A_1'$ or $A_2'$ is etherified hydroxy or amino, lower alkylamino or aminocarbonyl-lower alkylamino, with a compound of the formula

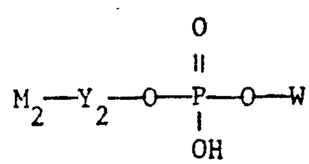

(XVI)

wherein X, $R_1$, $R_2$, $R_3$, $R_4$, $R_5$, $R_6$, $R_7$, T and W are as defined above, each of $Y_1$ and $Y_2$ is lower alkylene, which is unsubstituted or substituted, and hydroxyl groups present may be protected by easily removable protecting groups, and one of $M_1$ and $M_2$ is a free amino group or an activated derivative thereof, and the other is a carboxylic acid group or an activated derivative thereof, and removing any protecting groups present,

h) esterifying a compound of the formula

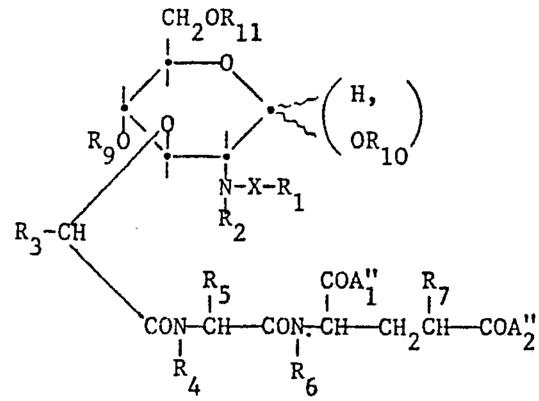

(XVII)

wherein one of $A_1''$ and $A_2''$ is a radical of the formula

$$—T—Y_1—M_3 \quad \text{(XVIII)}$$

in a manner know per se with a compound of the formula

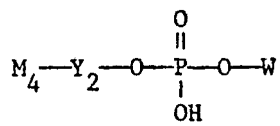

(XIX)

wherein X, $R_1$, $R_2$, $R_3$, $R_4$, $R_5$, $R_6$, $R_7$, T and W are as defined above, each of $Y_1$ and $Y_2$ is lower alkylene which is unsubstituted or substituted and any hydroxyl groups present are protected by easily removable protecting groups, $R_9$, $R_{10}$ and $R_{11}$ are easily removable protecting groups, and the other radical $A''_1$ or $A''_2$ is etherified hydroxy, amino, lower alkylamino or aminocarbonyl-lower alkylamino, and one of $M_3$ and $M_4$ is a free hydroxyl group and the other is a free carboxyl group, and one of $M_3$ and $M_4$ may be present in reactive form, and removing any protecting groups present,

i) reacting a compound of the formula

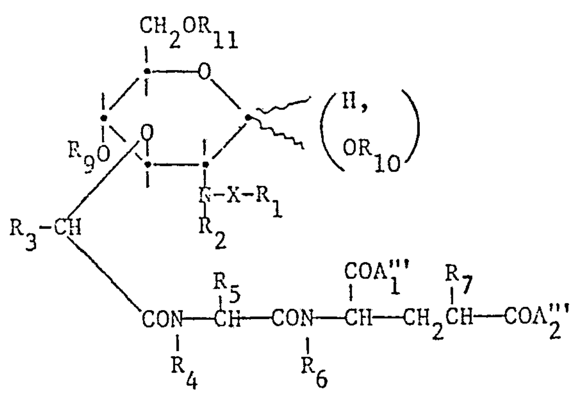

(XX)

wherein X, $R_1$, $R_2$, $R_3$, $R_4$, $R_5$, $R_6$ and $R_7$ are as defined above, and any hydroxyl groups present are protected by an easily removable protecting group, $R_9$, $R_{10}$ and $R_{11}$ are easily removable protecting groups and one of $A'''_1$ and $A'''_2$ is —T—Y—OH, wherein Y and T are as defined above and the other radical $A'''_1$ or $A'''_2$ is free or etherified hydroxy, amino or lower alkylamino, or is aminocarbonyl-lower alkylamino which is unsubstituted or substituted in the lower alkyl moiety with a compound affording the radical of the formula

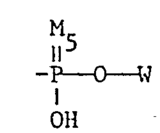

(XXI)

wherein $M_5$ is an electron pair or oxo and W is as defined above, and if $M_5$ is an electron pair oxidising the

reaction product with a weak oxidising agent, and removing protecting groups present,

k) reacting a compound of the formula

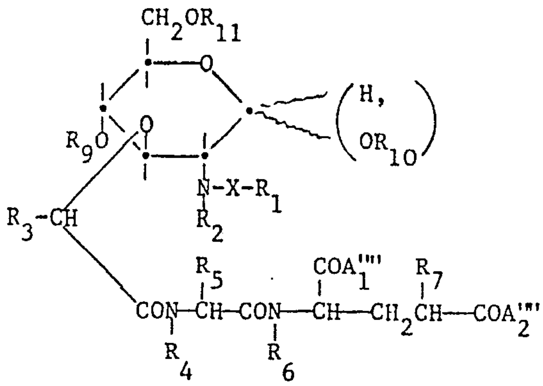

(XXIII)

wherein X, $R_1$, $R_2$, $R_3$, $R_4$, $R_5$, $R_6$ and $R_7$ are as defined above, and any hydroxyl groups present are protected by an easily removable protecting group, $R_9$, $R_{10}$ and $R_{11}$ are easily removable protecting groups and one of $A_1''''$ and $A_2''''$ is

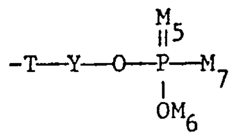

(XXIV)

and the other is free or etherified hydroxy, amino, lower alkylamino or aminocarbonyl-lower alkylamino, in which formula XXIV T and Y are as defined in claim 1, $M_5$ is an electron pair or oxo, $M_6$ is hydrogen or an easily removable protecting group and $M_7$ is a hydroxyl group which may be present in reactive form, with a compound of the formula

HO—W (XXV)

wherein W is as defined above, and if $M_5$ is an electron pair oxidising the reactive product with a weak oxidising agent, and removing protecting groups present, or

l) in a compound of formula I in which one or more functional groups are protected by protecting groups, removing said protecting groups, and if desired, after carrying out one of the processes a)-l), converting a resultant compound of formula I into a salt thereof.

2. A process according to claim 1, which comprises preparing a compound of formula I according to claim 1, wherein W is alkyl or alkenyl containing up to 30 carbon atoms which is unsubstituted or substituted by hydroxy, lower alkoxy, lower alkanoyloxy, halogen, amino, alkanoylamino or oxo, and/or a salt thereof.

3. A process according to claim 1, which comprises preparing a compound of formula I according to claim 1, wherein X is carbonyl, $R_1$ is lower $C_1$-$C_7$ alkyl or phenyl, $R_2$, $R_4$, $R_6$ and $R_7$ are hydrogen, $R_3$ is hydrogen or lower alkyl, $R_5$ is hydrogen, lower alkyl, hydroxymethyl, mercaptomethyl, 1-hydroxyethyl, 2-methylthioethyl, phenylmethyl, p-hydroxyphenylmethyl, 4-aminobutyl, 4-imidazolylmethyl, or 3-indolylmethyl, or $R_4$ and $R_5$ together are also trimethylene, wherein $A_1$ is hydroxy, lower alkoxy, amino, lower alkylamino or aminocarbonyl-lower alkylamino and $A_2$ is a radical of the formula

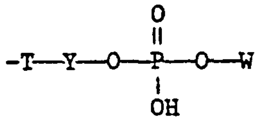

(II)

wherein T is NH or O and W is an alkyl or alkenyl group which is unsubstituted or substituted by hydroxy, lower alkoxy, lower alkanoyloxy, amino, lower alkylamino or alkanoylamino containing up to 25 carbon atoms, or is a $C_{10}$-$C_{30}$ cycloalkyl or $C_{10}$-$C_{30}$ cycloalkenyl radical, Y is ethylene or a radical of the formula

$$Y_1 - COO - Y_2 \qquad \text{(IIIa)}$$

or

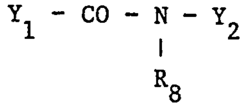

(IIIc)

wherein $R_8$ is hydrogen and each of $Y_1$ and $Y_2$ independently of the other is lower $C_1$-$C_7$ alkylene which is unsubstituted or substituted by hydroxy, lower alkoxy, mercapto, methylthio, phenyl, 4-imidazolyl or 3-indolyl, and/or a salt thereof.

4. A process according to claim 1, which comprises preparing a compound of formula I according to claim 1, wherein X is carbonyl, $R_1$ is lower $C_1$-$C_3$ alkyl, $R_2$, $R_4$, $R_6$ and $R_7$ are hydrogen, $R_3$ is hydrogen or methyl, $R_5$ is hydrogen or lower alkyl, $A_1$ is amino and $A_2$ is a radical of the formula

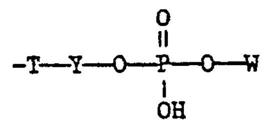

(II)

wherein T is NH, W is a $C_{10}$-$C_{25}$ alkyl or $C_{10}$-$C_{25}$ alkenyl group which is unsubstituted or substituted by hydroxy, lower alkoxy, lower alkanoyloxy, amino, lower alkylamino or alkanoylamino containing up to 25 carbon atoms, or is a cholesteryl radical, and Y is ethylene or a radical of the formula

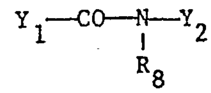

(IIIc)

wherein $R_8$ is hydrogen and each of $Y_1$ and $Y_2$ independently of the other is lower alkylenen, and/or a salt thereof.

5. A process according to claim 1, which comprises preparing a compound according to either claim 3 or claim 4, wherein the meanings for $A_1$ and $A_2$ are interchanged, and/or a salt thereof.

6. A process according to claim 1, which comprises preparing a compound of formula I according to claim 1, wherein X is carbonyl, $R_1$ is alkyl or aryl, each unsubstituted or substituted, $R_2$, $R_3$, $R_4$ and $R_6$ are hydrogen or lower alkyl, $R_5$ is hydrogen, lower alkyl which is unsubstituted or substituted by hydroxy, lower alkoxy, mercapto, lower alkylmercapto or halogen, or is cycloalkyl or cycloalkyl-lower alkyl, in each of which the cycloalkyl moiety contains 4 to 6 carbon atoms, or is phenyl-lower alkyl, each unsubstituted or substituted, or is heterocyclyl or heterocyclyl-lower alkyl, each containing one or two aza atoms, or $R_4$ and $R_5$ together are also $C_3$-$C_4$ alkylene, $R_7$ is hydrogen and one of $A_1$ and $A_2$ is a radical of the formula

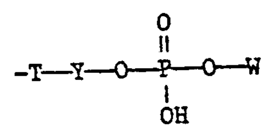

(II)

wherein T is NH or O, Y is alkylene which is unsubstituted or substituted and may also be interrupted by carbonyloxy or carbonylimino, and W is an alkyl group containing more than 6 carbon atoms which is unsubstituted or substituted by hydroxy, lower alkanoyloxy, amino, alkanoylamino or oxo, and the other radical $A_1$ or $A_2$ is hydroxy, lower alkoxy, amino or lower alkylamino, or is aminocarbonyl-lower alkylamino which is unsubstituted or substituted in the lower alkyl moiety by hydroxy, carboxy and/or amino, and/or a salt thereof.

7. A process according to claim 1, which comprises preparing a compound of formula I according to claim 1, wherein X is carbonyl, $R_1$ is lower alkyl which is unsubstituted or substituted by hydroxy, lower alkoxy or halogen, or is phenyl which is unsubstituted or substituted by hydroxy, lower alkoxy, lower alkyl or halogen, $R_2$, $R_4$ and $R_6$ are hydrogen, $R_3$ is hydrogen or lower alkyl, $R_5$ is hydrogen, lower $C_1$-$C_3$ alkyl which is unsubstituted or substituted by hydroxy, lower alkoxy, mercapto, lower alkylmercapto or halogen, or is cycloalkyl or cycloalkyl-lower alkyl wherein the lower alkyl moiety contains 1 to 3 carbon atoms, and in each of which cycloalkyl contains 4 to 6 carbon atoms, or is phenyl or phenyl-lower alkyl containing 1 to 3 carbon atoms in the lower alkyl moiety, each unsubstituted or substituted by hydroxy, lower alkoxy or halogen, or is heterocyclyl or heterocyclyl-lower alkyl containing 1 to 3 carbon atoms in the lower alkyl moiety, and each containing one or two aza atoms and having 5 or 6 ring members, or $R_4$ and $R_5$ together are also $C_3$-$C_4$ alkylene, $R_7$ hydrogen, and one of $A_1$ and $A_2$ is a radical of the formula

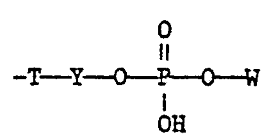

(II)

wherein T is NH or O, Y is lower alkylene which is unsubstituted or substituted or is a radical of the formula

$$Y_1{-}COO{-}Y_2 \quad (IIIa)$$

$$Y_1{-}OOC{-}Y_2 \quad (IIIb)$$

$$Y_1{-}CO{-}\underset{\substack{|\\R_8}}{N}{-}Y_2 \quad (IIIc)$$

or

$$Y_1{-}\underset{\substack{|\\R_8}}{N}CO{-}Y_2 \quad (IIId),$$

wherein each of $Y_1$ and $Y_2$ is lower alkylene which is unsubstituted or substituted and $R_8$ is hydrogen, W is a $C_{10}$-$C_{25}$ alkyl group carrying in the 2-position a hydroxyl group, an alkanoyloxy group, an amino group or an alkanoylamino group, and the other radical $A_1$ or $A_2$ is hydroxy, lower alkoxy, amino or lower alkylamino, or is aminocarbonyl-lower alkylamino which is unsubstituted or substituted in the lower alkyl moiety by hydroxy, carboxy or amino groups, and/or a salt thereof.

8. A process according to any one of claims 1, 6 and 7, which comprises preparing a compound of formula I, wherein Y is $C_1$-$C_4$ alkylene which is unsubstituted or substituted by $C_1$-$C_4$ alkyl and may be interrupted by carbonyloxy or carbonylimino, and/or a salt thereof.

9. A process according to claim 1, which comprises preparing a compound of formula I according to claim 1, wherein X is carbonyl, $R_1$ is lower $C_1$-$C_3$ alkyl or phenyl, $R_2$, $R_4$ and $R_6$ are hydrogen, $R_3$ is hydrogen or lower $C_1$-$C_3$ alkyl, $R_5$ is hydrogen, lower $C_1$-$C_3$ alkyl which is unsubstituted or substituted by hydroxy, methoxy, mercapto, methylmercapto or halogen, or is phenyl or phenylmethyl, each unsubstituted or substituted by hydroxy, methoxy or halogen, or is heterocyclyl or heterocyclylmethyl, each containing one or two aza atoms and having 5 ring members, or $R_4$ and $R_5$ together are also trimethylene, $R_7$ is hydrogen and one of $A_1$ and $A_2$ is a radical of the formula

$$-T{-}Y{-}O{-}\overset{\substack{O\\\|}}{\underset{\substack{|\\OH}}{P}}{-}O{-}W \quad (II)$$

wherein T is NH or O, Y is lower $C_2$-$C_3$ alkylene, or is a radical of the formula (IIIa) or (IIIc)

$$Y_1 - COO - Y_2 \quad (IIIa)$$

$$Y_1 - CO - \underset{\substack{|\\R_8}}{N} - Y_2 \quad (IIIc)$$

wherein $R_8$ is hydrogen, and each of $Y_1$ and $Y_2$ independently of the other is lower $C_1$-$C_3$ alkylene which is unsubstituted or substituted by hydroxy, lower alkoxy, mercapto or lower alkylmercapto, or is lower $C_1$-$C_3$ alkylene which is substituted by phenyl or phenyl-lower alkyl, which may themselves be unsubstituted or substituted by hydroxy, methoxy or halogen; or is lower $C_1$-$C_3$ alkylene which is substituted by heterocyclyl or heterocyclyl-lower alkyl containing 1 to 3 carbon atoms in the lower alkyl radical, and each containing one or two aza atoms and having 5 or 6 ring members, W is a $C_{10}$-$C_{25}$ alkyl group substituted in the 2-position by hydroxy, lower alkanoyloxy, amino or alkanoylamino, and the other radical $A_1$ or $A_2$ is hydroxy, lower alkoxy, amino, lower alkymamino or aminocarbonyl-lower alkylamino, and/or a salt thereof.

10. A process according to any one of claims 1 to 9, which comprises choosing the starting materials such that a compound of formula I is obtained, wherein in each asymmetric substitution at C-$R_3$ the D-configuration, at C-$R_5$ the L-configuration and at $C{-}\underset{\substack{|\\R_6}}{N}$ the D-configuration is present, and/or a salt thereof.

11. A process according to claim 1, which comprises preparing a N-acetyldesmethylmuramyl-L-alanyl-D-isoglutaminyl-L-alanine 2-(hexadecyoxyhydroxyphosphoryloxy)ethylamide, and/or a salt thereof.

12. A process according to claim 1, which comprises preparing a N-acetylmuramyl-L-alanyl-D-isoglutaminyl-L-alanine 2-(hexadecyoxyhydroxyphosphoryloxy)ethylamide, and/or a salt thereof.

13. A process according to claim 1, which comprises preparing a N-acetylmuramyl-L-alanyl-D-isoglutaminyl-L-alanine 2-(tetradecyloxyhydroxyphosphoryloxy)ethylamide, and/or a salt thereof.

14. A process according to claim 1, which comprises preparing a N-acetylmuramyl-L-alanyl-D-isoglutaminyl-L-alanine 2-[(3′R)-hydroxy-(2′S)-palmitoylamino-4′t-octadecenyloxyhydroxyphosphoryloxy]ethylamide, and/or a salt thereof.

15. A process according to claim 1, which comprises preparing a N-acetylmuramyl-L-α-aminobutyryl-D-isoglutaminyl-L-alanine 2-[(3′R)-hydroxy-(2′S)-palmitoylamino-4′t-octadecenyloxyhydroxyphosphoryloxy]ethylamide, and/or a salt thereof.

16. A process according to claim 1, which comprises preparing a N-acetylmuramyl-L-α-aminobutyryl-D-isoglutaminyl, L-alanine 2-(3′R)-hydroxy-(2′S)-palmitoylaminooctadecyloxyhydroxyphosphoryloxy]ethylamide, and/or a salt thereof.

17. A process according to claim 1, which comprises preparing a N-acetylmuramyl-L-alanyl-D-isoglutaminyl-L-alanine 2-(cholest-5-ene-3β-oxyhydroxyphosphoryloxy)ethylamide, and/or a salt thereof.

18. A process according to claim 1, which comprises preparing a N-benzoylmuramyl-L-alanyl-D-isoglutaminyl-L-alanine 2-(hexadecyloxyhydroxyphosphoryloxy)ethylamide, and/or a salt thereof.

19. A process according to claim 1, which comprises preparing a compound selected from the group consisting of N-acetylmuramyl-L-alanyl-D-isoglutamine 2-(hexadecyloxyhydroxyphosphoryloxy)ethylamide, N-acetyldesmethylmuramyl-L-alanyl-D-isoglutamine 2-(hexadexyloxyhydroxyphosphoryloxy)-ethylamide, N-acetylmuramyl-L-alanyl-D-isoglutaminyloxymethyl-carboxylic acid 2-(hexadecyloxyhydroxyphosphoryloxy)ethylamide, N-acetylmuramyl-L-alanyl-D-isoglutamine 2-[(3′R)-hydroxy-(2′S)-palmitoylamino-4′t-octadecenyloxyhydroxyphosphoryloxy]ethylamide, N-acetyldesmethylmuramyl-L-alanyl-D-isoglutamine 1-[(3′R)-hydroxy-(2′S)-palmitoylamino-4′t-octadecenyloxyhydroxyphosphoryloxy]ethylamide, N-acetyldesmethylmuramyl-L-alanyl-D-isoglutaminyl-L-alanine 2-[(3′R)-hydroxy-(2′S)-palmitoylamino-4′t-octadecenyloxyhydroxyphosphoryloxy]ethylamide, N-acetylmuramyl-L-alanyl-D-isoglutaminyloxymethylcarboxylic acid [(3′R)-hydroxy-(2′S)-palmitoylamino-4′t-octadecenyloxyhydroxyphosphoryloxy]-ethylamide, and/or a salt thereof.

20. A process according to claim 1, which comprises preparing an immunopotentiatingly active compound of formula I according to any one of claims 1 to 16, and/or a salt thereof.

21. A process according to any one of claims 1 to 20, which comprises removing the protecting groups by treatment with an acid or hydrogen, in the presence of a catalyst.

22. A process according to any one of claims 1 to 20, which comprises using, as compound containing one or more reactive esterified hydroxyl groups, a compound wherein the hydroxyl group or groups is or are esterified with a strong inorganic acid or a sulfonic acid.

23. A process according to any one of claims 1 to 20, which comprises using, as compound containing one or more reactive esterified hydroxyl groups, a compound wherein the hydroxyl group or groups is or are esterified with a hydrohalic acid.

24. A process according to any one of claims 1 to 20, which comprises using, as compound with one or more protecting hydroxyl groups, a compound wherein the hydroxyl group or groups is or are protected by acyl or aroyl radicals, by radicals derived from carbonic acid derivatives, by alkyl radicals branched in the α-position, by α-mono-, α-di- or α-triaryl-lower alkyl radicals or by acetal-forming radicals.

25. A process according to claim 24, which comprises using, as compound with one or more protecting hydroxyl groups, a compound wherein the hydroxyl group or groups is or are protected by lower alkanoyl, benzoyl, benzyloxycarbonyl, lower alkoxycarbonyl or tert-butyl radicals, or by benzyl, triphenylmethyl or tetrahydropyranyl radicals which are unsubstituted or substituted.

26. A process according to any one of claims 1 to 20, which comprises using, as compound with one or more protecting carboxyl groups, a compound wherein the carboxyl group or groups is or are protected by tert-butyl or benzyl radicals, or by triphenylmethyl or benzhydryl radicals which are unsubstituted or substituted by halogen or lower alkoxy.

27. A process according to any one of claims 1 to 20, which comprises using, as compound with one or more activated carboxylic acid groups, a compound wherein the carboxylic acid group or groups is or are present in the form of an anhydride, azide, activated amide or activated ester.

28. A process according to claim 27, wherein the acid anhydrides are those with carbonic acid lower alkyl esters, the acid amides are imidazolides or isooxazolides and the activated esters are cyanomethyl ester or carboxymethyl ester, acetylaminoethylthio ester, p-nitrophenyl ester or 2,4,5-trichlorophenyl ester, N-hydroxysuccinimide ester, N-hydroxyphthalimide ester or N-hydroxypiperidine ester, 8-hydroxyquinoline ester, methoxyethylthio ester, or esters obtained by reaction with carbodiimide with the addition of N-hydroxysuccinimide or a 1-hydroxybenzotriazole or 3-hydroxy-4-oxo-3,4-dihydrobenzo[d]-1,2,3-triazine.

29. A process for the preparation of a pharmaceutical composition, which comprises mixing a compound obtained according to any one of claims 1 to 28 with an antibiotic and a pharmaceutically acceptable carrier.

**Revendications** (pour les Etats contractants : BE, CH, DE, FR, GB, IT, LI, LU, NL, SE)

1. Phosphorylmuramylpeptides de formule générale

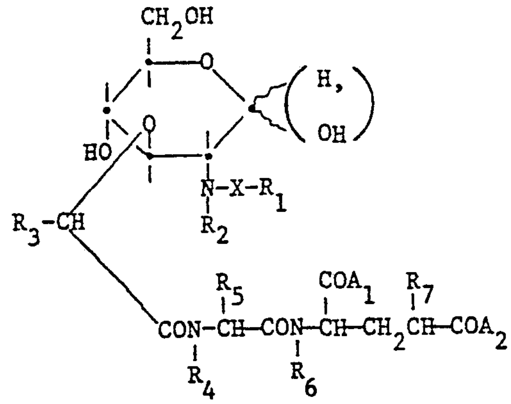

(I)

où X représente un carbonyle ou un carbonyloxy, $R_1$ un alcoyle ou un aryle éventuellement substitué, $R_2$, $R_4$ et $R_6$ un hydrogène ou un alcoyle inférieur, $R_3$ un hydrogène ou un alcoyle inférieur, $R_5$ un hydrogène, un alcoyle inférieur, un hydroxyalcoyle inférieur libre ou fonctionnellement modifié, un mercaptoalcoyle inférieur libre ou fonctionnellement modifié, un aminoalcoyle inférieur éventuellement substitué, un cycloalcoyle, un cycloalcoyle-alcoyle inférieur, un aryle ou un aralcoyle éventuellement substitué, un hétérocyclyle ou hétérocyclyl-alcoyle inférieur contenant de l'azote, ou $R_4$ et $R_5$ représentent également ensemble un alcoylène en $C_3$ à $C_4$, $R_7$ un hydrogène ou un carboxyle éventuellement estérifié ou amidé et l'un des radicaux $A_1$ et $A_2$ représente un radical de formule

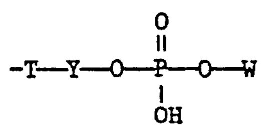

(II)

où T représente NH ou O, Y représente un groupe alcoylène éventuellement substitué, qui peut également être interrompu par un ou deux oxycarbonyle et/ou iminocarbonyle, et W représente un radical aliphatique ou un radical cycloalcoyle ou cycloalcényle avec à chaque fois plus de 6 atomes de carbone, et l'autre des radicaux $A_1$ et $A_2$ représente un hydroxy libre ou éthérifié, un amino, un alcoyle inférieur-amino ou un aminocarbonyl-alcoyle inférieur-amino éventuellement substitué dans le radical alcoyle inférieur, où le préfixe « inférieur » utilisé ci-dessus désigne un radical ayant jusqu'à 7 atomes de carbone compris, et leurs sels à l'exception des composés publiés dans la demande de brevet européen ayant le N° de publication 0 025 495, à savoir des muramylpeptides de formule

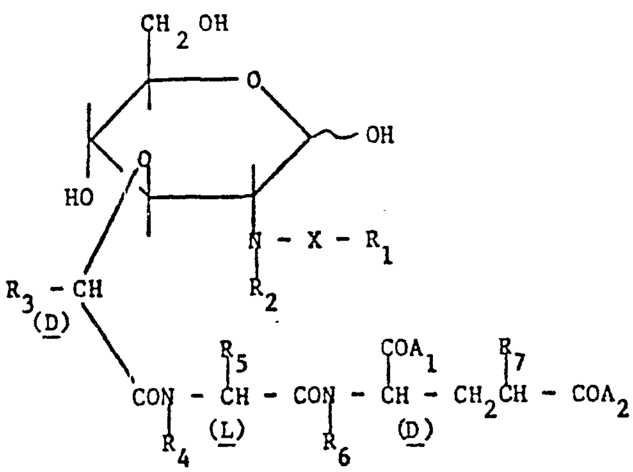

(Ia)

où X représente un carbonyle ou un carbonyloxy, $R_1$ un alcoyle ou un aryle éventuellement substitué, $R_2$, $R_4$ et $R_6$ un hydrogène ou un alcoyle inférieur, $R_3$ un hydrogène ou un alcoyle inférieur, $R_5$ un hydrogène, un alcoyle inférieur, un hydroxyalcoyle inférieur libre ou fonctionnellement modifié, un mercaptoalcoyle inférieur libre ou fonctionnellement modifié, un aminoalcoyle inférieur éventuellement substitué, un cycloalcoyle, un cycloalcoyle-alcoyle inférieur, un aryle ou un aralcoyle éventuellement substitué, un

hétérocyclyle ou hétérocyclyl-alcoyle inférieur contenant de l'azote, ou $R_4$ et $R_5$ représentent également ensemble un alcoylène en $C_3$ à $C_4$, $R_7$ représente un hydrogène ou un carboxyle éventuellement estérifié ou amidé et l'un des radicaux $A_1$ et $A_2$ représente un radical de formule

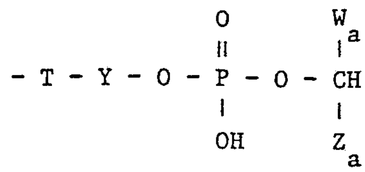

(IIa)

où T représente HN ou O, Y représente un groupe alcoylène éventuellement substitué, qui peut également être interrompu par un ou deux oxycarbonyle et/ou iminocarbonyle, $W_a$ représente un hydrogène et $Z_a$ représente un groupe 1,2-dihydroxyéthyle ou 2-hydroxyéthyle, où au moins un groupe hydroxy est estérifié avec un acide carboxylique aliphatique en $C_{12}$ à $C_{90}$ éventuellement non saturé ou éthérifié avec un alcool aliphatique en $C_{10}$ à $C_{22}$ éventuellement non saturé, où $W_a$ et $Z_a$ représentent chacun un groupe hydroxyméthyle estérifié avec un acide carboxylique aliphatique en $C_{12}$ à $C_{90}$ éventuellement non saturé ou éthérifié avec un alcool aliphatique en $C_{10}$ à $C_{22}$ éventuellement non saturé, et l'autre des radicaux $A_1$ et $A_2$ est un hydroxy libre ou éthérifié, un amino, un alcoyle inférieur-amino ou un carboxamidoalcoyle inférieur-amino, et de leurs sels, où le préfixe « inférieur » désigne des radicaux ayant jusqu'à 7 atomes de carbone compris, ainsi que des muramylpeptides de formule Ia représentée ci-dessus, où X représente un carbonyle ou un carbonyloxy, $R_1$ un alcoyle inférieur en $C_1$ à $C_3$ ou un phényle, $R_2$, $R_4$ et $R_6$ représentent un hydrogène, $R_3$ représente un hydrogène ou un alcoyle inférieur en $C_1$ à $C_3$, $R^5$ représente un hydrogène ou un alcoyle inférieur, $R^7$ représente un hydrogène, $A_1$ un amino, un alcoyle inférieur-amino, un hydroxy ou un alcoxy inférieur et $A_2$ un radical de formule IIa représentée ci-dessus, où T représente NH ou O, Y représente un alcoylène inférieur en $C_2$ à $C_3$ ou un radical de formule

$$-CH_2-CO-NH-CH_2-CH_2-$$

$W_a$ représente un hydrogène et $Z_a$ un groupe 1,2-dihydroxyéthyle ou 2-hydroxyéthyle, où un ou deux groupes hydroxy sont estérifiés avec des acides alcanecarboxyliques en $C_{16}$ à $C_{22}$ semblables ou différents éventuellement mono- ou di-insaturés ou éthérifiés avec un alcanol en $C_{12}$ à $C_{18}$ éventuellement mono- ou di-insaturé, ou bien où deux groupes hydroxy ou bien où deux groupes hydroxy vicinaux sont formellement cétalisés, avec formation d'un noyau dioxolane, avec une bis-alcoyle inférieur-cétone ou acétalisés avec un aldéhyde aliphatique non substitué ayant jusqu'à 20 atomes de carbone, ou bien où $W_a$ et $Z_a$ représentent chacun un groupe hydroxyméthyle, qui est estérifié avec un acide alcanecarboxylique éventuellement mono- ou di-insaturé ou éthérifié avec un alcanol en $C_{12}$ à $C_{18}$ éventuellement mono- ou di-insaturé, et de leurs sels, où le préfixe « inférieur » désigne des radicaux ayant jusqu'à 7 atomes de carbone compris, ainsi que du N-acétyl-muramyl-L-alanyl-D-isoglutaminyl-L-alanine-2-(1',2'-hexadécylidène-sn-glycéro-3'-hydroxy-phosphoryloxy)-éthylamide et du N-acétyl-desméthylmuramyl-L-alanyl-D-isoglutaminyl-L-alanine-2-(1',2'-hexadécylidène-sn-glycéro-3'-hydroxyphosphoryloxy)-éthylamide et de leurs sels.

2. Composés de formule I selon la revendication 1, où W représente un alcoyle ou un alcényle ayant jusqu'à 30 atomes de carbone, qui est non substitué ou substitué par un hydroxy, un alcoxy inférieur, un alcanoyloxy inférieur, un halogène, un amino, un alcanoylamino ou un oxo, et leurs sels.

3. Composés de formule I selon la revendication 1, où X représente un carbonyle, $R_1$ un alcoyle inférieur en $C_1$ à $C_7$ ou un phényle, $R_2$, $R_4$, $R_6$ et $R_7$ rerprésentent un hydrogène, $R_3$ un hydrogène ou un alcoyle inférieur, $R_5$ un hydrogène, un alcoyle inférieur, un hydroxyméthyle, un mercaptométhyle, un 1-hydroxyéthyle, un 2-méthylthioéthyle, un phénylméthyle, un p-hydroxy-phényl-méthyle, un 4-aminobutyle, un 4-imidazolyl-méthyle, un 3-indolylméthyle où $R_4$ et $R_5$ représentent également ensemble un triméthylène, où $A_1$ représente un hydroxy, un alcoxy inférieur, un amino, un alcoyle inférieur-amino ou un aminocarbonylalcoyle inférieur-amino et $A_2$ représente un radical de formule

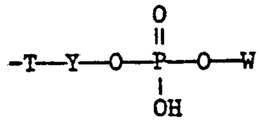

(II)

où T représente NH ou O et W un groupe alcoyle ou alcényle, qui est non substitué ou substitué par un hydroxy, un alcoxy inférieur, un alcanoyloxy inférieur, un amino, un alcoyle inférieur-amino ou un alcanoylamino ayant jusqu'à 25 atomes de carbone, ou un radical cycloalcoyle ou cycloalcényle en $C_{10}$ à $C_{30}$, Y représente un éthylène ou un radical de formules

$$Y_1 - COO - Y_2 \quad \text{(IIIa)}$$

ou

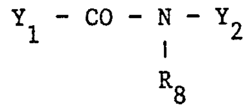

(IIIc)

où $R_8$ représente un hydrogène et $Y_1$ et $Y_2$ représentent chacun indépendamment l'un et l'autre un alcoylène inférieur en $C_1$ à $C_7$, qui est éventuellement substitué par un hydroxy, un alcoxy inférieur, un mercapto, un méthylthio, un phényle, un 4-imidazolyle ou un 3-indolyle, et leurs sels.

4. Composés de formule I selon la revendication 1, où X représente un carbonyle, $R_1$ un alcoyle inférieur en $C_1$ à $C_3$, $R_2$, $R_4$, $R_6$ et $R_7$ un hydrogène, $R_3$ un hydrogène ou un méthyle, $R_5$ un hydrogène ou un alcoyle inférieur, $A_1$ un amino et $A_2$ un radical de formule

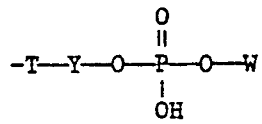

(II)

où T représente NH, W représente un groupe alcoyle ou alcényle en $C_{10}$ à $C_{25}$, qui est non substitué ou substitué par un hydroxy, un alcoxy inférieur, un alcanoyloxy inférieur, un amino, un alcoyle inférieur-amino ou un alcanoyl-amino ayant jusqu'à 25 atomes de carbone, ou un radical cholestéryle et Y représente un éthylène ou un radical de formule

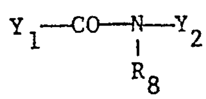

(IIIc)

où $R_8$ représente un hydrogène et $Y_1$ et $Y_2$ représentent chacun indépendamment l'un de l'autre un alcoyle inférieur, et leurs sels.

5. Composés selon les revendications 3 et 4, caractérisés en ce que les significations de $A_1$ et $A_2$ sont échangées, et leurs sels.

6. Composés de formule I selon la revendication 1, où X représente un carbonyle, $R_1$ un alcoyle ou un aryle éventuellement substitué, $R_2$, $R_3$, $R_4$ et $R_6$ représentent un hydrogène ou un alcoyle inférieur, $R_5$ représente un hydrogène, un alcoyle inférieur éventuellement substitué par un hydroxy, un alcoxy inférieur, un mercapto ou un alcoyle inférieur-mercapto ou un halogène, un cycloalcoyle ou un cycloalcoyl-alcoyle inférieur, où le radical cycloalcoyle contient de 4 à 6 atomes de carbone, un phényle ou un phényl-alcoyle inférieur éventuellement substitué, un hétérocyclyle ou hétérocyclyl-alcoyle inférieur contenant un ou deux atomes d'aza, ou bien où $R_4$ et $R_5$ représentent également ensemble un alcoylène en $C_3$ à $C_4$, $R_7$ représente un hydrogène et l'un des radicaux $A_1$ et $A_2$ représente un radical de formule

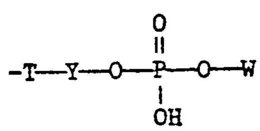

(II)

où T représente NH ou O, où Y représente un alcoylène éventuellement substitué, qui peut également être interrompu par un carbonyloxy ou un carbonylimino et W représente un groupe alcoyle éventuellement substitué par un hydroxy, un alcanoyloxy inférieur, un amino, un alcanoylamino ou un oxo ayant plus de 6 atomes de carbone, et l'autre des radicaux $A_1$ et $A_2$ est un hydroxy, un alcoxy inférieur, un amino, un alcoyle inférieur-amino ou un aminocarbonylalcoyle inférieur-amino éventuellement substitué dans le radical alcoyle inférieur par un hydroxy, un carboxy et/ou un amino, et leurs sels.

7. Composés de formule I selon la revendication 1, où X représente un carbonyle, $R_1$ représente un alcoyle inférieur éventuellement substitué par un hydroxy, un alcoxy inférieur ou un halogène ou un phényle éventuellement substitué par un hydroxy, un alcoxy inférieur, un alcoyle inférieur ou un halogène, $R_2$, $R_4$ et $R_6$ représentent un hydrogène, $R_3$ représente un hydrogène ou un alcoyle inférieur, $R_5$ représente un hydrogène, un alcoyle inférieur éventuellement substitué par un hydroxy, un alcoxy inférieur, un mercapto, un alcoyle inférieur-mercapto ou un halogène, un cycloalcoyle ou un cycloalcoyl-alcoyle inférieur, où le radical cycloalcoyle contient de 4 à 6 atomes de carbone et le radical alcoyle inférieur de 1 à 3 atomes de carbone, un phényle ou un phényl-alcoyle inférieur en $C_1$ à $C_3$ dans la fraction alcoyle inférieur éventuellement substitué par un hydroxy, un alcoxy inférieur ou un halogène, un hétérocyclyle ou hétérocyclyl-alcoyle inférieur contenant un ou deux atomes aza et ayant 5 à 6 chaînons

et de 1 à 3 atomes de carbone dans le radical alcoyle inférieur où $R_4$ et $R_5$ représentent également ensemble un alcoylène en $C_3$ à $C_4$, $R_7$ représente un hydrogène, et l'un des radicaux $A_1$ et $A_2$ représente un radical de formule

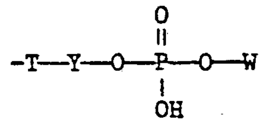 (II)

où T représente HN ou O, Y représente un alcoylène éventuellement substitué ou un radical d'une des formules

$Y_1$—COO—$Y_2$ (IIIa)

$Y_1$—OOC—$Y_2$ (IIIb)

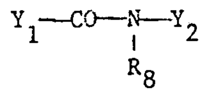 (IIIc)

ou

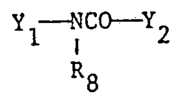 (IIId)

où $Y_1$ et $Y_2$ représentent chacun un alcoylène inférieur éventuellement substitué et $R_8$ représente un hydrogène, W représente un groupe alcoyle en $C_{10}$ à $C_{25}$, qui porte en position 2 un groupe hydroxy, un groupe alcanoyloxy, un amino ou un groupe alcanoylamino, et l'autre des radicaux $A_1$ et $A_2$ est un hydroxy, un alcoxy inférieur, un amino, un alcoyle inférieur-amino ou un aminocarbonyl-alcoxyle inférieur-amino éventuellement substitué par un hydroxy, un carboxy ou des groupes amino, et leurs sels.

8. Composés selon l'une des revendications 1, 6 et 7, où Y représente un alcoylène en $C_1$ à $C_4$ éventuellement substitué par un alcoyle en $C_1$ à $C_4$, et qui peut être interrompu par un carbonyloxy ou un carbonylimino.

9. Composés de formule I selon la revendication 1, où X représente un carbonyle, $R_1$ un alcoyle inférieur en $C_1$ à $C_3$ ou un phényle, $R_2$, $R_4$ et $R_6$ représentent un hydrogène, $R_3$ représente un hydrogène ou un alcoyle inférieur en $C_1$ à $C_3$, $C_5$ représente un hydrogène, un alcoyle inférieur en $C_1$ à $C_3$ éventuellement substitué par un hydroxy, un méthoxy, un mercapto, un méthylmercapto ou un halogène, ou phényle ou un phénylméthyle éventuellement substitué par un hydroxy, un méthoxy ou un halogène, un hétérocyclyle ou hétérocyclylméthyle contenant un ou deux atomes d'aza et ayant 5 chaînons, où $R_4$ et $R_5$ représentent également ensemble un triméthylène, $R_7$ représente un hydrogène et l'un des radicaux $A_1$ et $A_2$ représente un radical de formule

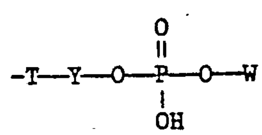 (II)

où T représente HN ou O, Y représente un alcoylène inférieur en $C_2$ à $C_3$ ou un radical de formules (IIIa) ou (IIIc)

$Y_1$ - COO - $Y_2$ (IIIa)

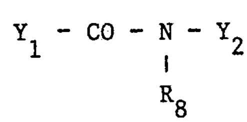 (IIIc)

où $R_8$ représente un hydrogène et $Y_1$ et $Y_2$ représentent chacun indépendamment l'un de l'autre un alcoylène inférieur en $C_1$ à $C_3$ éventuellement substitué par un hydroxy, un alcoxy inférieur, un mercapto ou un alcoyle inférieur-mercapto, ou un alcoylène inférieur en $C_1$ à $C_3$, qui est éventuellement substitué

par un phényle ou un phényl-alcoyle inférieur éventuellement substitué par un hydroxy, un méthoxy ou un halogène, ou un hétérocyclyle ou hétérocyclyl-alcoyle inférieur contenant un ou deux atomes d'aza et ayant de 5 à 6 chaînons et de 1 à 3 atomes de carbone dans le radical alcoyle inférieur, W représente un groupe alcoyle en $C_{10}$ à $C_{25}$, qui est substitué en position 2 par un hydroxy, un alcanoyloxy inférieur, un amino ou un alcanoylamino, et l'autre des radicaux $A_1$ et $A_2$ est un hydroxy, un alcoxy inférieur, un amino, un alcoyle inférieur-amino ou un aminocarbonylalcoyle inférieur-amino, et leurs sels.

10. Composés de formule I selon l'une des revendications 1-9, où chaque fois en cas de substitution asymétrique on a sur C-$R_3$ la configuration D, sur C-$R_5$ la configuration L et sur C-N la configuration D, et

$R_6$

leurs sels.

11. N-acétyl-desméthylmuramyl-L-alanyl-D-isoglutaminyl-L-alanine-2-(hexadécyloxy-hydroxyphosphoryloxy)-éthylamide et ses sels selon la revendication 1.

12. N-acétyl-muramyl-L-alanyl-D-isoglutaminyl-L-alanine-2-(hexadécyloxy-hydroxyphosphoryloxy)-éthylamide et ses sels selon la revendication 1.

13. N-acétyl-muramyl-L-alanyl-D-isoglutaminyl-L-alanine-2-(tétradécyloxy-hydroxyphosphoryloxy)-éthylamide et ses sels selon la revendication 1.

14. N-acétyl-muramyl-L-alanyl-D-isoglutaminyl-L-alanine-2-[(3'R)-hydroxy-(2'S)-palmitoylamino-4't-octadécényloxyhydroxyphosphoryloxy]-éthylamide et ses sels selon la revendication 1.

15. N-acétyl-muramyl-L-α-aminobutyryl-D-isoglutaminyl-L-alanine-2-[3'R)-hydroxy-(2'S)-palmitoylamino-4't-octadécényloxy-hydroxyphosphoryloxy]-éthylamide et ses sels selon la revendication 1.

16. N-acétyl-muramyl-L-α-aminobutyryl-D-isoglutaminyl-L-alanine-2-[(3'-R)-hydroxy-(2'S)-palmitoylamino-octadécyloxyhydroxyphosphoryloxy]-éthylamide et ses sels selon la revendication 1.

17. N-acétyl-muramyl-L-alanyl-D-isoglutaminyl-L-alanine-2-(cholest-5-èn-3β-oxy-hydroxyphosphoryloxy)-éthylamide et ses sels selon la revendication 1.

18. N-benzoyl-muramyl-L-alanyl-D-isoglutaminyl-L-alanine-2-(hexadécyloxy-hydroxyphosphoryloxy)-éthylamide et ses sels selon la revendication 1.

19. Composé choisi dans le groupe N-acétyl-muramyl-L-alanyl-D-isoglutamine-2-(hexadécyloxy-hydroxyphosphoryloxy)-éthylamide, N-acétyl-desméthylmuramyl-L-alanyl-D-isoglutamine-2-(hexadécyloxy-hydroxyphosphoryloxy)-éthylamide, 2-(hexadécyloxy-hydroxyphosphoryloxy)-éthylamide de l'acide N-acétyl-muramyl-L-alanyl-D-isoglutaminyl-oxyméthylcarboxylique, N-acétyl-muramyl-L-alanyl-D-isoglutamine-2-[(3'R)-hydroxy-(2'S)-palmitoylamino-4't-octadécényloxyhydroxyphosphoryloxy]-éthylamide, N-acétyl-desméthylmuramyl-L-alanyl-D-isoglutamine-2-[(3'R)-hydroxy-(2'S)-palmitoylamino-4't-octadécényloxyhydroxyphosphoryloxy]-éthylamide, N-acétyl-desméthylmuramyl-L-alanyl-D-isoglutaminyl-L-alanine-2-[(3'R)-hydroxy-(2'S)-palmitoylamino-4't-octadécényloxy-hydroxyphosphoryloxy]-éthylamide, [(3'R)-hydroxy-(2'S)-palmitoylamino-4't-octadécényloxy-hydroxyphosphoryloxy]-éthylamide de l'acide N-acétyl-muramyl-L-alanyl-D-isoglutaminyl-oxyméthylcarboxylique ou un sel de ces composés selon la revendication 1.

20. Les composés de formule I et leurs sels selon l'une des revendications 1 à 19 ayant une action immunopotentialisante.

21. Préparations utilisables en pharmacie contenant des composés de formule I selon l'une des revendications 1 à 19 avec un support pharmaceutiquement acceptable.

22. Application de composés de formule I selon l'une des revendications 1 à 19 à la préparation de préparations pharmaceutiques.

23. Composé selon l'une des revendications 1 à 19 aux fins d'application comme médicament.

24. Composé selon l'une des revendications 1 à 19 aux fins d'application comme agent immunostimulant.

25. Préparations pharmaceutiques qui contiennent au moins un composé selon l'une des revendications 1 à 19 et au moins un antibiotique avec un support pharmaceutiquement acceptable.

26. Muramylpeptide selon l'une des revendications 1 à 19 aux fins d'application dans un procédé d'accroissement de l'activité antibiotique des antibiotiques, caractérisé en ce qu'on administre le muramylpeptide et un antibiotique essentiellement ensemble dans le temps (au cours d'une journée).

27. Procédé de préparation des composés selon la revendication 1, caractérisé en ce que

a) on fait réagir un composé de formule

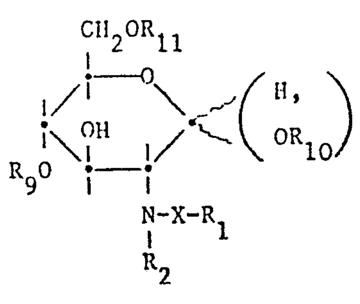

(V)

où X, $R_1$ et $R_2$ ont la signification donnée dans la revendication 1 et où les groupes hydroxy qui s'y trouvent éventuellement sont protégés par un groupe protecteur facilement séparable, $R_9$, $R_{10}$ et $R_{11}$ représentent chacun un groupe protecteur facilement séparable, ou un de ses composés métalliques, avec un composé de formule

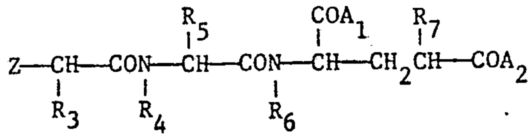

(VI)

où Z représente un groupe hydroxy estérifié réactif, $R_3$, $R_4$, $R_5$, $R_6$, $R_7$, $A_1$ et $A_2$ ont la signification donnée dans la revendication 1 et les groupes hydroxy qui s'y trouvent éventuellement sont protégés par un groupe protecteur facilement séparable, et on sépare les groupes protecteurs présents,

b) de façon connue on condense un composé de formule

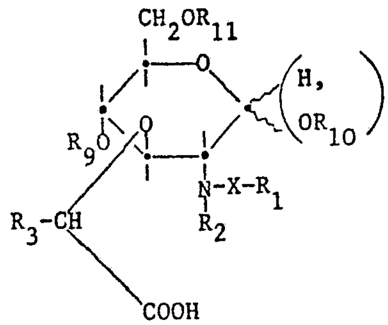

(VII)

où X, $R_1$, $R_2$ et $R_3$ ont la signification donnée dans la revendication 1 et $R_9$, $R_{10}$ et $R_{11}$ représentent un hydrogène ou un groupe protecteur facilement séparable, ou un de ses dérivés avec un composé de formule

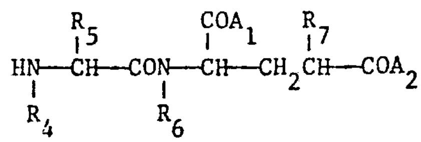

(VIII)

où $R_4$, $R_5$, $R_6$, $R_7$, $COA_1$ et $COA_2$ ont la signification donnée dans la revendication 1, avec la précision que les groupes carboxy et, si on le désire, les groupes hydroxy libres présents dans ces radicaux sont protégés par des groupes protecteurs facilement séparables, ou un de ses dérivés, et on sépare les groupes protecteurs présents,

c) on condense un composé de formule

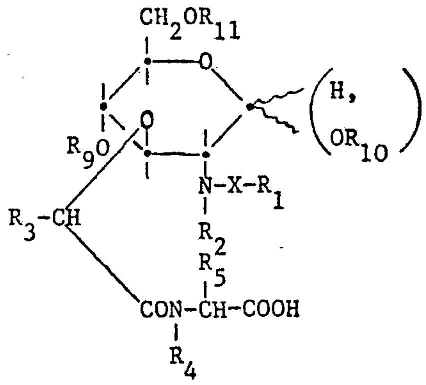

(IX)

où X, $R_1$, $R_2$, $R_3$, $R_4$ et $R_5$ ont la signification donnée dans la revendication 1, avec la précision que les groupes hydroxy qui y sont contenus sont éventuellement protégés par un groupe protecteur facilement séparable, et $R_9$, $R_{10}$ et $R_{11}$ représentent un hydrogène ou des groupes protecteurs facilement séparables, ou des dérivés de ce corps avec un composé de formule

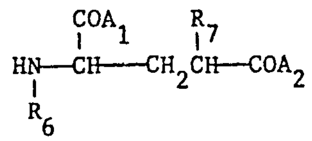

(X)

où $R_6$, $R_7$, $A_1$ et $A_2$ ont la signification donnée dans la revendication 1, avec la précision que les groupes carboxyle libres présents dans les radicaux $R_7$, —$COA_1$ et —$COA_2$ sont protégés par des groupes protecteurs facilement séparables, et on sépare les groupes protecteurs présents,

d) de façon connue on condense un composé de formule

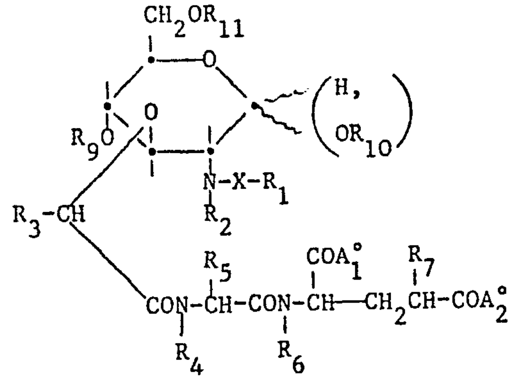

(XI)

où X, $R_1$, $R_2$, $R_3$, $R_4$, $R_5$, $R_6$ et $R_7$ ont la signification donnée dans la revendication 1, $R_9$, $R_{10}$ et $R_{11}$ représentent un hydrogène ou un groupe protecteur facilement séparable et l'un des radicaux $A_1°$ et $A_2°$ représente un groupe hydroxy activé et l'autre est un hydroxy éthérifié, un amino, un alcoyle inférieur-amino ou un aminocarbonylalcoyle inférieur-amino, avec un composé de formule

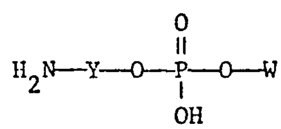

(XII)

où Y et W ont la signification donnée dans la revendication 1, et on sépare les groupes protecteurs éventuellement présents,

e) de façon connue on estérifie un composé de formule

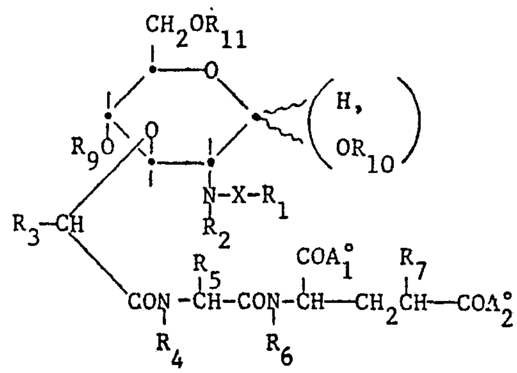

(XIa)

où X, $R_1$, $R_2$, $R_3$, $R_4$, $R_5$, $R_6$ et $R_7$ ont la signification donnée dans la revendication 1, $R_9$, $R_{10}$ et $R_{11}$ représentent un hydrogène ou un groupe protecteur facilement séparable et l'un des radicaux $A_1°$ et $A_2°$ représente un groupe hydroxy et l'autre est un hydroxy éthérifié, un amino, un alcoyle inférieur-amino ou un aminocarbonyl-alcoyle inférieur-amino, avec un composé de formule

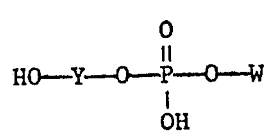

(XIIa)

où Y et W ont la signification donnée dans la revendication 1, où l'acide XIa ou l'alcool XXa est présent sous forme réactive, et on sépare les groupes protecteurs éventuellement présents,

f) dans un composé de formule

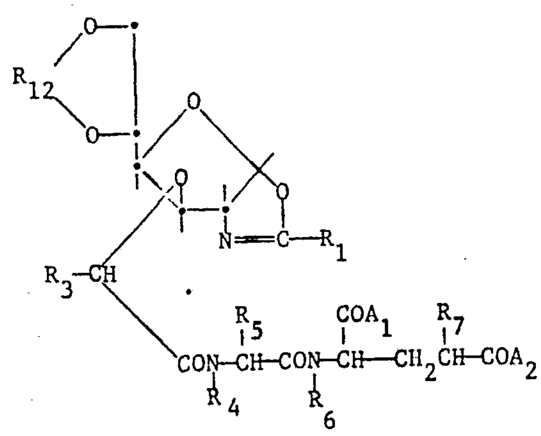

(XIII)

où $R_1$, $R_3$, $R_4$, $R_5$, $R_6$, $R_7$, $A_1$ et $A_2$ ont la signification donnée dans la revendication 1 et $R_{12}$ est un groupe alcoylidène ou cycloalcoylidène, on effectue un clivage acide du noyau oxazoline et du noyau dioxolane, et éventuellement on sépare les groupes protecteurs présents,

g) on condense un composé de formule

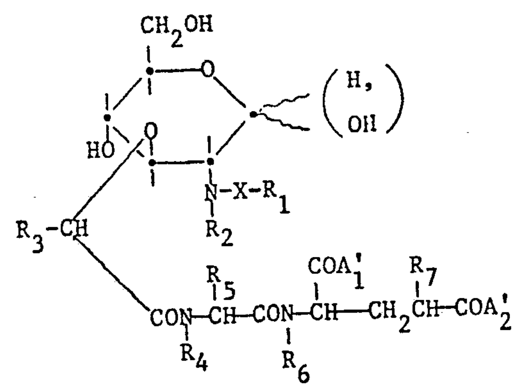

(XIV)

où l'un des radicaux $A_1'$ et $A_2'$ est un radical de formule

$$-T-Y_1-M_1 \qquad (XV)$$

et l'autre des radicaux $A_1'$ et $A_2'$ est un hydroxy éthérifié ou un amino, un alcoyle inférieur-amino ou un aminocarbonylalcoyle inférieur-amino, et un composé de formule

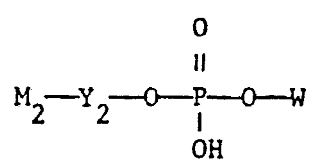

(XVI)

où X, $R_1$, $R_2$, $R_3$, $R_4$, $R_5$, $R_6$, $R_7$, T et W ont les significations données dans la revendication 1, $Y_1$ et $Y_2$ représentent chacun un alcoylène inférieur éventuellement substitué et les groupes hydroxy qui y sont présents sont éventuellement protégés par des groupes protecteurs facilement séparables et l'un des radicaux $M_1$ et $M_2$ représente un groupe amino libre ou un des dérivés activés et l'autre un groupe acide carboxylique ou un de ses dérivés activés, et on sépare les groupes protecteurs éventuellemnt présents

h) on estérifie de façon connue un composé de formule

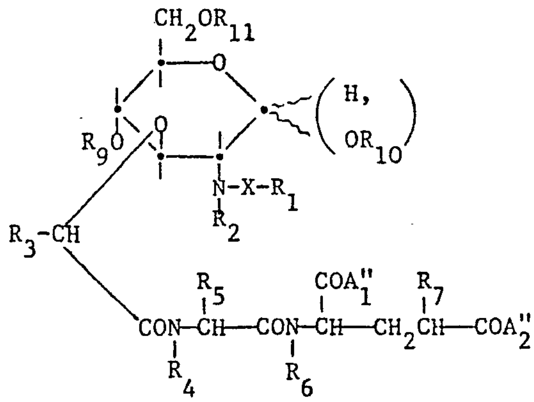

(XVII)

où l'un des radicaux $A_1''$ et $A_2''$ représente un radical de formule

$$—T—Y_1—M_3 \quad \text{(XVIII)}$$

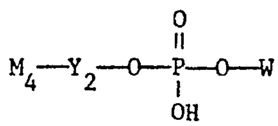

(XIX)

où X, $R_1$, $R_2$, $R_3$, $R_4$, $R_5$, $R_6$, $R_7$, T et W ont la signification donnée dans la revendication 1, $Y_1$ et $Y_2$ représentent chacun un alcoylène inférieur éventuellement substitué et les groupes hydroxy qui s'y trouvent éventuellement sont protégés par des groupes protecteurs facilement séparables, $R_9$, $R_{10}$ et $R_{11}$ représentent des groupes protecteurs facilement séparables, et l'autre des radicaux $A_1''$ et $A_2''$ est un hydroxy éthérifié, un amino, un alcoyle inférieur-amino ou un aminocarbonylalcoyle inférieur-amino, et l'un des radicaux $M_3$ et $M_4$ représente un groupe hydroxy libre et l'autre un groupe carboxyle libre, où le cas échéant l'un des deux radicaux $M_3$ et $M_4$ se présente sous forme réactive, et on sépare les groupes protecteurs éventuellement présents,

i) on fait réagir un composé de formule

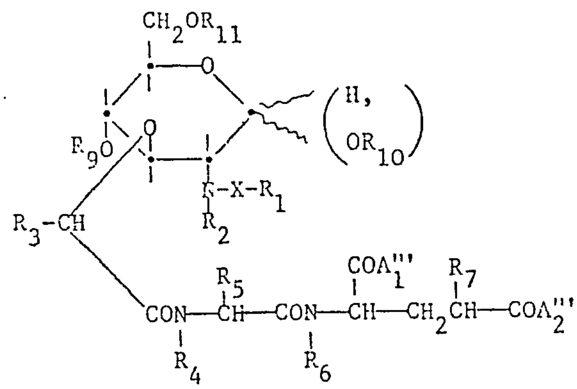

(XX)

où X, $R_1$, $R_2$, $R_3$, $R_4$, $R_5$, $R_6$ et $R_7$ ont les significations données dans la revendication 1 et où les groupes hydroxy qui y sont éventuellement présents sont protégés par un groupe protecteur facilement séparable, $R_9$, $R_{10}$ et $R_{11}$ représentent des groupes protecteurs facilement séparables et l'un des radicaux $A_1'''$ et $A_2'''$ représente —T—Y—OH, où Y et T ont les significations données dans la revendication 1, et l'autre des radicaux $A_1'''$ et $A_2'''$ représente un hydroxy libre ou éthérifié, un amino, un alcoyle inférieur-amino ou un aminocarbonylalcoyle inférieur-amino éventuellement substitué dans le radical alcoyle inférieur, avec un composé donnant le radical de formule XXI,

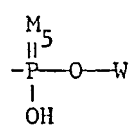

(XXI)

où $=M_5$ représente une paire d'électrons ou un oxo et W a la signification donnée dans la revendication

1, si $=M_5$ est une paire d'électrons on oxyde avec un oxydant faible, et on sépare les groupes protecteurs présents,

k) on fait réagir un composé de formule

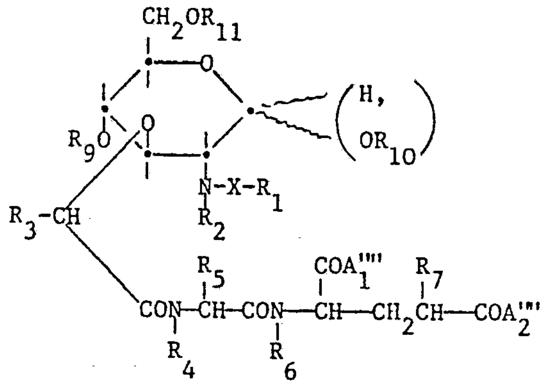

(XXIII)

où X, $R_1$, $R_2$, $R_3$, $R_4$, $R_5$, $R_6$ et $R_7$ ont les significations données dans la revendication 1 et les groupes hydroxy qui y sont éventuellement présents sont protégés avec un groupe protecteur facilement séparable, $R_9$, $R_{10}$ et $R_{11}$ représentent des groupes protecteurs facilement séparables et l'un des radicaux $A_1''''$ et $A_2''''$ représente

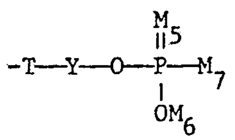

(XXIV)

et l'autre représente un hydroxy libre ou éthérifié, un amino, un alcoyle inférieur-amino ou un aminocarbonyl-alcoyle inférieur-amino, où T et Y ont la signification donnée dans la revendication 1, $=M_5$ représente une paire d'électrons ou un oxo, $M_6$ représente un hydrogène ou un groupe protecteur facilement séparable et $M_7$ représente un groupe hydroxy éventuellement présent sous forme réactive, avec un composé de formule

$$HO—W \qquad (XXV)$$

où W a la signification donnée dans la revendication 1, si $=M_5$ est une paire d'électrons on oxyde avec un oxydant faible, et on sépare les groupes protecteurs présents,

l) dans un composé de formule I où un ou plusieurs groupes fonctionnels sont protégés par des groupes protecteurs, on sépare ces groupes protecteurs, et, si on le désire, après avoir effectué l'un des procédés a-l) on transforme un composé de formule I obtenu en son sel.

28. Procédé selon la revendication 27, caractérisé en ce qu'on choisit les produits de départ de manière à obtenir un composé selon la revendication 10.

29. Les composés obtenus selon un procédé de la revendication 27 et leurs sels.

**Revendications** (pour l'Etat contractant AT)

1. Procédé de préparation de muramylpeptides de formule générale

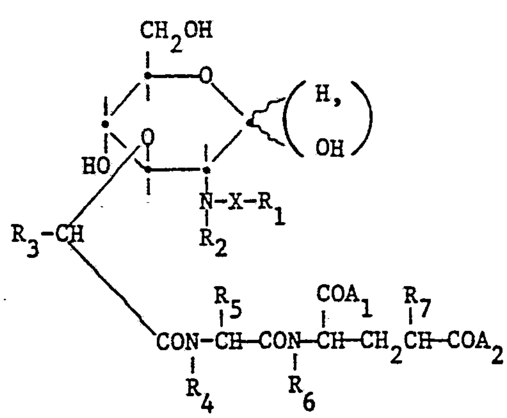

(I)

où X représente un carbonyle ou un carbonyloxy, $R_1$ un alcoyle ou un aryle éventuellement substitué, $R_2$, $R_4$ et $R_6$ un hydrogène ou un alcoyle inférieur, $R_3$ un hydrogène ou un alcoyle inférieur, $R_5$ un hydrogène, un alcoyle inférieur, un hydroxyalcoyle inférieur libre ou fonctionnellement modifié, un mercaptoalcoyle inférieur libre ou fonctionnellement modifié, un aminoalcoyle inférieur éventuellement substitué, un cycloalcoyle, un cycloalcoyle-alcoyle inférieur, un aryle ou un aralcoyle éventuellement substitué, un hétérocyclyle ou hétérocyclyl-alcoyle inférieur contenant de l'azote, où $R_4$ et $R_5$ représentent également ensemble un alcoylène en $C_3$ à $C_4$, $R_7$ un hydrogène ou un carboxyle éventuellement estérifié ou amidé et l'un des radicaux $A_1$ et $A_2$ représente un radical de formule

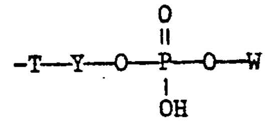

(II)

où T représente NH ou O, Y représente un groupe alcoylène éventuellement substitué, qui peut également être interrompu par un ou deux oxycarbonyle et/ou iminocarbonyle, et W représente un radical aliphatique ou un radical cycloalcoyle ou cycloalcényle avec à chaque fois plus de 6 atomes de carbone, et l'autre des radicaux $A_1$ et $A_2$ représente un hydroxy libre ou éthérifié, un amino, un alcoyle inférieur-amino ou un aminocarbonyl-alcoyle inférieur-amino éventuellement substitué dans le radical alcoyle inférieur, où le préfixe « inférieur » utilisé ci-dessus désigne un radical ayant jusqu'à 7 atomes de carbone compris, et leurs sels à l'exception des composés publiés dans la demande de brevet européen ayant le N° de publication 0 025 495, à savoir des muramylpeptides de formule

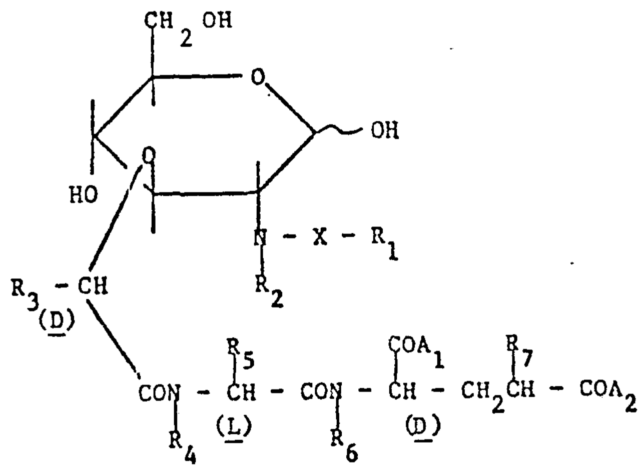

(Ia)

où X représente un carbonyle ou un carbonyloxy, $R_1$ un alcoyle ou un aryle éventuellement substitué, $R_2$, $R_4$ et $R_6$ un hydrogène ou un alcoyle inférieur, $R_3$ un hydrogène ou un alcoyle inférieur, $R_5$ un hydrogène, un alcoyle inférieur, un hydroxyalcoyle inférieur libre ou fonctionnellement modifié, un mercaptoalcoyle inférieur libre ou fonctionnellement modifié, un aminoalcoyle inférieur éventuellement substitué, un cycloalcoyle, un cycloalcoyle-alcoyle inférieur, un aryle ou un aralcoyle éventuellement substitué, un hétérocyclyle ou hétérocyclyl-alcoyle inférieur contenant de l'azote, où $R_4$ et $R_5$ représentent également ensemble un alcoylène en $C_3$ et $C_4$, $R_7$ représente un hydrogène ou un carboxyle éventuellement estérifié ou amidé et l'un des radicaux $A_1$ et $A_2$ représente un radical de formule

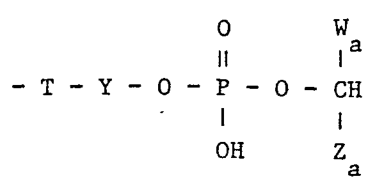

(IIa)

où T représente HN ou O, Y représente un groupe alcoylène éventuellement substitué, qui peut également être interrompu par un ou deux oxycarbonyle et/ou iminocarbonyle, $W_a$ représente un hydrogène et $Z_a$ représente un groupe 1,2-dihydroxyéthyle ou 2-hydroxyéthyle, où au moins un groupe hydroxy est estérifié avec un acide carboxylique aliphatique en $C_{12}$ à $C_{90}$ éventuellement non saturé ou éthérifié avec un alcool aliphatique en $C_{10}$ à $C_{22}$ éventuellement non saturé, où $W_a$ et $Z_a$ représentent chacun un groupe hydroxyméthyle estérifié avec un acide carboxylique aliphatique en $C_{12}$ à $C_{90}$ éventuellement non saturé ou éthérifié avec un alcool aliphatique en $C_{10}$ à $C_{22}$ éventuellement non saturé, et l'autre des radicaux $A_1$ et $A_2$ est un hydroxy libre ou éthérifié, un amino, un alcoyle inférieur-amino ou un

carboxamidoalcoyle inférieur-amino, et de leurs sels, où le préfixe « inférieur » désigne des radicaux ayant jusqu'à 7 atomes de carbone compris, ainsi que des muramylpeptides de formule la représentée ci-dessus, où X représente un carbonyle ou un carbonyloxy, $R_1$ un alcoyle inférieur en $C_1$ à $C_3$ ou un phényle, $R_2$, $R_4$ et $R_6$ représentent un hydrogène, $R^3$ représente un hydrogène ou un alcoyle inférieur en $C_1$ à $C_3$, $R^5$ représente un hydrogène ou un alcoyle inférieur, $R^7$ représente un hydrogène, $A_1$ un amino, un alcoyle inférieur-amino, un hydroxy ou un alcoxy inférieur et $A_2$ un radical de formule IIa représentée ci-dessus, où T représente NH ou O, Y représente un alcoylène inférieur en $C_2$ à $C_3$ ou un radical de formule

$$—CH_2—CO—NH—CH_2—CH_2—$$

$W_a$ représente un hydrogène et $Z_a$ un groupe 1,2-dihydroxyéthyle ou 2-hydroxyéthyle, où un ou deux groupes hydroxy sont estérifiés avec des acides alcanecarboxyliques en $C_{16}$ à $C_{22}$ semblables ou différents éventuellement mono- ou di-insaturés ou éthérifiés avec un alcanol en $C_{12}$ à $C_{18}$ éventuellement mono- ou di-insaturé, ou bien où deux groupes hydroxy ou bien où deux groupes hydroxy vicinaux sont formellement cétalisés, avec formation d'un noyau dioxolane, avec une bis-alcoyle inférieur-cétone ou acétalisés avec un aldéhyde aliphatique non substitué ayant jusqu'à 20 atomes de carbone, ou bien où $W_a$ et $Z_a$ représentent chacun un groupe hydroxyméthyle, qui est estérifié avec un acide alcanecarboxylique éventuellement mono- ou di-insaturé ou éthérifié avec un alcanol en $C_{12}$ à $C_{18}$ éventuellement mono- ou di-insaturé, et de leurs sels, où le préfixe « inférieur » désigne des radicaux ayant jusqu'à 7 atomes de carbone compris, ainsi que du N-acétyl-muramyl-L-alanyl-D-isoglutaminyl-L-alanine-2-(1',2'-hexadécylidène-sn-glycéro-3'-hydroxy-phosphoryloxy)éthylamide et du N-acétyl-desméthylmuramyl-L-alanyl-D-isoglutaminyl-L-alanine-2-(1',2'-hexadécylidène-sn-glycéro-3'-hydroxyphosphoryloxy)-éthylamide et de leurs sels, caractérisé en ce que

a) on fait réagir un composé de formule

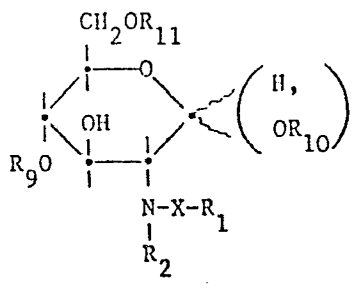

(V)

où X, $R_1$ et $R_2$ ont la signification donnée dans la revendication 1 et où les groupes hydroxy qui s'y trouvent éventuellement sont protégés par un groupe protecteur facilement séparable, $R_9$, $R_{10}$ et $R_{11}$ représentent chacun un groupe protecteur facilement séparable, ou un de ses composés métalliques, avec un composé de formule

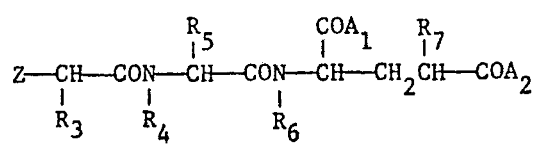

(VI)

où Z représente un groupe hydroxy estérifié réactif, $R_3$, $R_4$, $R_5$, $R_6$, $R_7$, $A_1$ et $A_2$ sont la signification donnée dans la revendication 1 et les groupes hydroxy qui s'y trouvent éventuellement sont protégés par un groupe protecteur facilement séparable, et on sépare les groupes protecteurs présents,

b) de façon connue on condense un composé de formule

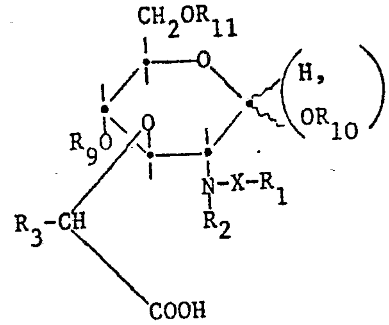

(VII)

où X, $R_1$, $R_2$ et $R_3$ ont la signification donnée dans la revendication 1 et $R_9$, $R_{10}$ et $R_{11}$ représentent un hydrogène ou un groupe protecteur facilement séparable, ou un de ses dérivés avec un composé de formule

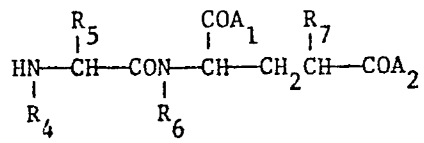

(VIII)

où $R_4$, $R_5$, $R_6$, $R_7$, $COA_1$ et $COA_2$ ont la signification donnée dans la revendication 1, avec la précision que les groupes carboxy et, si on le désire, les groupes hydroxy libres présents dans ces radicaux sont protégés par des groupes protecteurs facilement séparables, ou un de ses dérivés, et on sépare les groupes protecteurs présents,

c) on condense un composé de formule

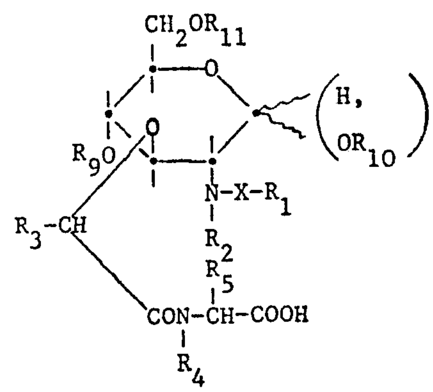

(IX)

où X, $R_1$, $R_2$, $R_3$, $R_4$ et $R_5$ ont la signification donnée dans la revendication 1, avec la précision que les groupes hydroxy qui y sont contenus sont éventuellement protégés par un groupe protecteur facilement séparable, et $R_9$, $R_{10}$ et $R_{11}$ représentent un hydrogène ou des groupes protecteurs facilement séparables, ou des dérivés de ce corps avec un composé de formule

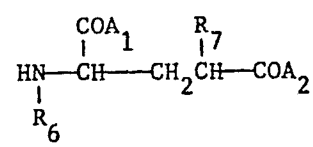

(X)

où $R_6$, $R_7$, $A_1$ et $A_2$ ont la signification donnée dans la revendication 1, avec la précision que les groupes carboxyle libres présents dans les radicaux $R_7$, $-COA_1$ et $-COA_2$ sont protégés par des groupes protecteurs facilement séparables, et on sépare les groupes protecteurs présents,

d) de façon connue on condense un composé de formule

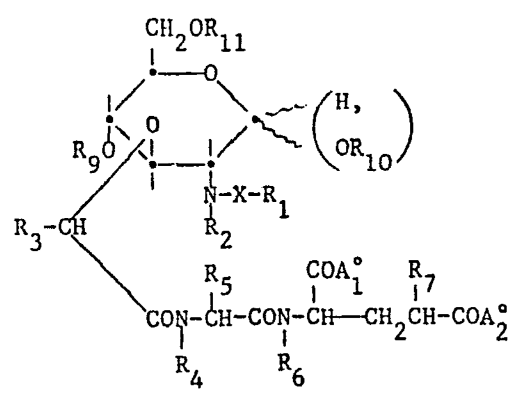

(XI)

où X, $R_1$, $R_2$, $R_3$, $R_4$, $R_5$, $R_6$ et $R_7$ ont la signification donnée dans la revendication 1, $R_9$, $R_{10}$ et $R_{11}$ représentent un hydrogène ou un groupe protecteur facilement séparable et l'un des radicaux $A_1°$ et $A_2°$

représente un groupe hydroxy activé et l'autre est un hydroxy éthérifié, un amino, un alcoyle inférieur-amino ou un amino-carbonylalcoyle inférieur-amino, avec un composé de formule

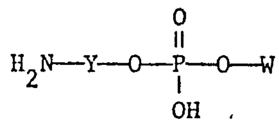

(XII)

où Y et W ont la signification donnée dans la revendication 1, et on sépare les groupes protecteurs éventuellement présents,

e) de façon connue on estérifie un composé de formule

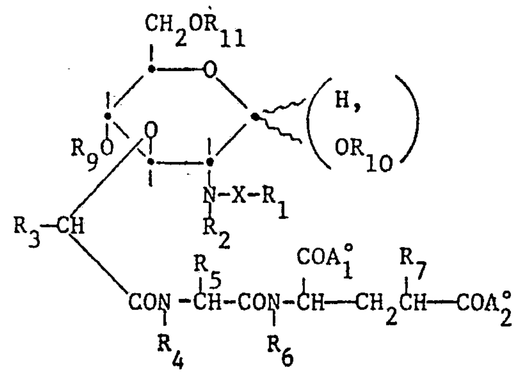

(XIa)

où X, $R_1$, $R_2$, $R_3$, $R_4$, $R_5$, $R_6$ et $R_7$ ont la signification donnée dans la revendication 1, $R_9$, $R_{10}$ et $R_{11}$ représentent un hydrogène ou un groupe protecteur facilement séparable et l'un des radicaux $A_1°$ et $A_2°$ représente un groupe hydroxy et l'autre est un hydroxy éthérifié, un amino, un alcoyle inférieur-amino ou un aminocarbonyl-alcoyle inférieur-amino, avec un composé de formule

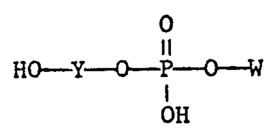

(XIIa)

où Y et W ont la signification donnée dans la revendication 1, où l'acide XIa ou l'alcool XXa est présent sous forme réactive, et on sépare les groupes protecteurs éventuellement présents,

f) dans un composé de formule

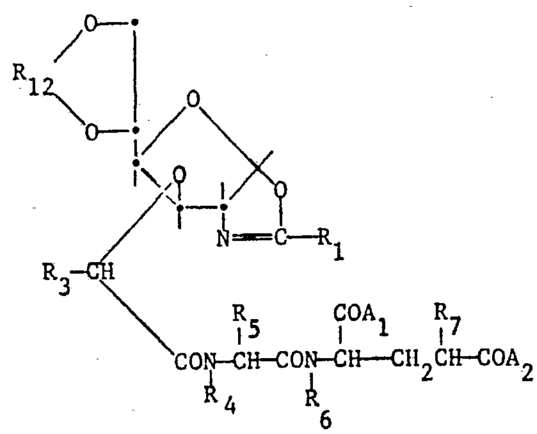

(XIII)

où $R_1$, $R_3$, $R_4$, $R_5$, $R_6$, $R_7$, $A_1$ et $A_2$ ont la signification donnée dans la revendication 1 et $R_{12}$ est un groupe alcoylidène ou cycloalcoylidène, on effectue un clivage acide du noyau oxazoline et du noyau dioxolane, et éventuellement on sépare les groupes protecteurs présents,

g) on condense un composé de formule

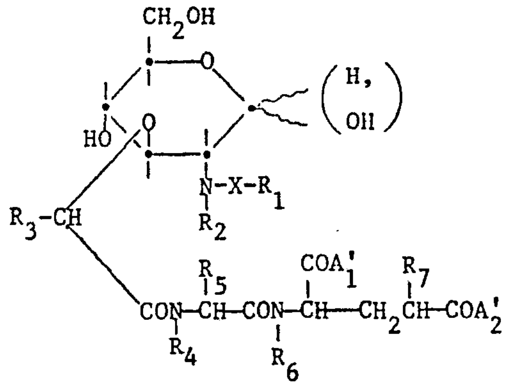

(XIV)

où l'un des radicaux $A_1'$ et $A_2'$ est un radical de formule

$$—T—Y_1—M_1 \qquad (XV)$$

et l'autre des radicaux $A_1'$ et $A_2'$ est un hydroxy éthérifié ou un amino, un alcoyle inférieur-amino ou un aminocarbonylalcoyle inférieur-amino, et un composé de formule

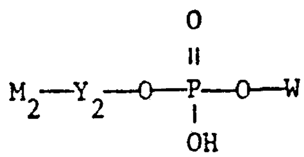

(XVI)

où X, $R_1$, $R_2$, $R_3$, $R_4$, $R_5$, $R_6$, $R_7$, T et W ont les significations données dans la revendication 1, $Y_1$ et $Y_2$ représentent chacun un alcoylène inférieur éventuellement substitué et les groupes hydroxy qui y sont présents sont éventuellement protégés par des groupes protecteurs facilement séparables et l'un des radicaux $M_1$ et $M_2$ représente un groupe amino libre ou un de ses dérivés activés et l'autre un groupe acide carboxylique ou un de ses dérivés activés, et on sépare les groupes protecteurs éventuellement présents,

h) on estérifie de façon connue un composé de formule

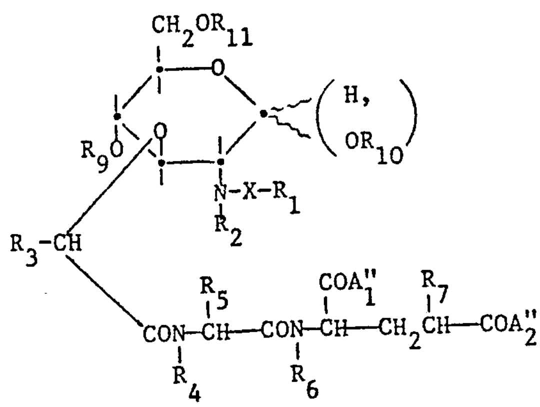

(XVII)

où l'un des radicaux $A_1''$ et $A_2''$ représente un radical de formule

$$—T—Y_1—M_3 \qquad (XVIII)$$

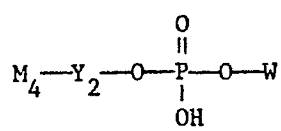

(XIX)

où X, $R_1$, $R_2$, $R_3$, $R_4$, $R_5$, $R_6$, $R_7$, T et W ont la signification donnée dans la revendication 1, $Y_1$ et $Y_2$ représentent chacun un alcoylène inférieur éventuellement substitué et les groupes hydroxy qui s'y trouvent éventuellement sont protégés par des groupes protecteurs facilement séparables, $R_9$, $R_{10}$ et $R_{11}$ représentent des groupes protecteurs facilement séparables, et l'autre des radicaux $A_1''$ et $A_2''$ est

un hydroxy éthérifié, un amino, un alcoyle inférieur-amino ou un aminocarbonylalcoyle inférieur-amino, et l'un des radicaux $M_3$ et $M_4$ représente un groupe hydroxy libre et l'autre un groupe carboxyle libre, où le cas échéant l'un des deux radicaux $M_3$ et $M_4$ se présente sous forme réactive, et on sépare les groupes protecteurs éventuellement présents,

i) on fait réagir un composé de formule

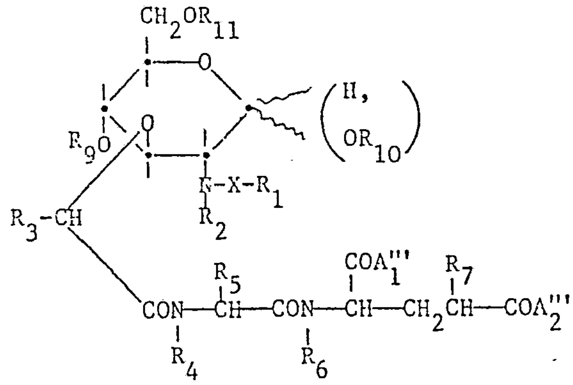

(XX)

où X, $R_1$, $R_2$, $R_3$, $R_4$, $R_5$, $R_6$ et $R_7$ ont les significations données dans la revendication 1 et où les groupes hydroxy qui y sont éventuellement présents sont protégés par un groupe protecteur facilement séparable, $R_9$, $R_{10}$ et $R_{11}$ représentent des groupes protecteurs facilement séparables et l'un des radicaux $A_1''$ et $A_2'''$ représente —T—Y—OH, où Y et T ont les significations données dans la revendication 1, et l'autre des radicaux $A_1'''$ et $A_2'''$ représente un hydroxy libre ou éthérifié, un amino, un alcoyle inférieur-amino ou un aminocarbonylalcoyle inférieur-amino éventuellement substitué dans le radical alcoyle inférieur, avec un composé donnant le radical de formule XXI,

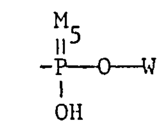

(XXI)

où $=M_5$ représente une paire d'électrons ou un oxo et W a la signification donnée dans la revendication 1, si $=M_5$ est une paire d'électrons on oxyde avec un oxydant faible, et on sépare les groupes protecteurs présents,

k) on fait réagir un composé de formule

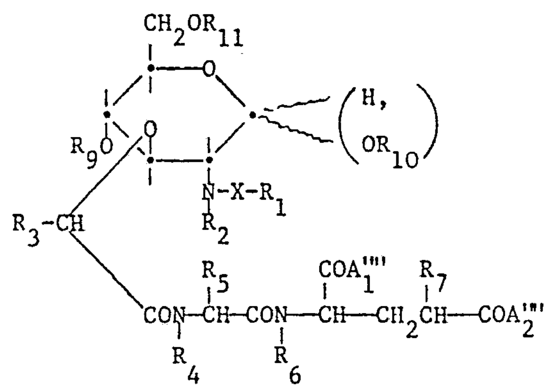

(XXIII)

où X, $R_1$, $R_2$, $R_3$, $R_4$, $R_5$, $R_6$ et $R_7$ ont les significations données dans la revendication 1 et les groupes hydroxy qui y sont éventuellement présents sont protégés avec un groupe protecteur facilement séparable, $R_9$, $R_{10}$ et $R_{11}$ représentent des groupes protecteurs facilement séparables et l'un des radicaux $A_1''''$ et $A_2''''$ représente

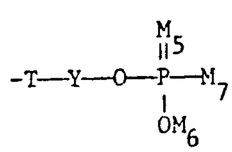

(XXIV)

et l'autre représente un hydroxy libre ou éthérifié, un amino, un alcoyle inférieur-amino ou un aminocarbonyl-alcoyle inférieur-amino, où T et Y ont la signification donnée dans la revendication 1, $=M_5$ représente une paire d'électrons ou un oxo, $M_6$ représente un hydrogène ou un groupe protecteur facilement séparable et $M_7$ représente un groupe hydroxy éventuellement présent sous forme réactive, avec un composé de formule

$$HO—W \qquad (XXV)$$

où W a la signification donnée dans la revendication 1, si $=M_5$ est une paire d'électrons on oxyde avec un oxydant faible, et on sépare les groupes protecteurs présents,

l) dans un composé de formule I où un ou plusieurs groupes fonctionnels sont protégés par des groupes protecteurs, on sépare ces groupes protecteurs, et, si on le désire, après avoir effectué l'un des procédés a-l) on transforme un composé de formule I obtenu en son sel.

2. Procédé selon la revendication 1, caractérisé en ce qu'on prépare des composés de formule I selon la revendication 1, où W représente un alcoyle ou un alcényle ayant jusqu'à 30 atomes de carbone, qui est non substitué ou substitué par un hydroxy, un alcoxy inférieur, un alcanoyloxy inférieur, un halogène, un amino, un alcanoylamino ou un oxo, et/ou leurs sels.

3. Procédé selon la revendication 1, caractérisé en ce qu'on prépare des composés de formule I selon la revendication 1, où X représente un carbonyle, $R_1$ un alcoyle inférieur en $C_1$ à $C_7$ ou un phényle, $R_2$, $R_4$, $R_6$ et $R_7$ représentent un hydrogène, $R_3$ un hydrogène ou un alcoyle inférieur, $R_5$ un hydrogène, un alcoyle inférieur, un hydroxyméthyle, un mercaptométhyle, un 1-hydroxyéthyle, un 2-méthylthioéthyle, un phénylméthyle, un p-hydroxy-phényl-méthyle, un 4-amino-butyle, un 4-imidazoyl-méthyle, un 3-indolyl-méthyle ou $R_4$ et $R_5$ représentent également ensemble un triméthylène, où $A_1$ représente un hydroxy, un alcoxy inférieur, un amino, un alcoyle inférieur-amino ou un amino-carbonylalcoyle inférieur-amino et $A_2$ représente un radical de formule

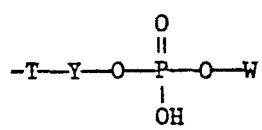
(II)

où T représente NH ou O et W un groupe alcoyle ou alcényle, qui est non substitué ou substitué par un hydroxy, un alcoxy inférieur, un alcanoyloxy inférieur, un amino, un alcoyle inférieur-amino ou un alcanoylamino ayant jusqu'à 25 atomes de carbone, ou un radical cycloalcoyle ou cycloalcényle en $C_{10}$ à $C_{30}$, Y représente un éthylène ou un radical de formules

$$Y_1 - COO - Y_2 \qquad (IIIa)$$

ou

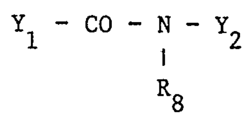
(IIIc)

où $R_8$ représente un hydrogène et $Y_1$ et $Y_2$ représentent indépendamment l'un de l'autre chacun un alcoylène inférieur en $C_1$ à $C_7$, qui est éventuellement substitué par un hydroxy, un alcoxy inférieur, un mercapto, un méthylthio, un phényle, un 4-imidazolyle ou un 3-indolyle, et/ou leurs sels.

4. Procédé selon la revendication 1, caractérisé en ce qu'on prépare des composés de formule I selon la revendication 1, où X représente un carbonyle, $R_1$ représente un alcoyle inférieur en $C_1$ à $C_3$, $R_2$, $R_4$, $R_6$ et $R_7$ représentent un hydrogène, $R_3$ représente un hydrogène ou un méthyle, $R_5$ représente un hydrogène ou un alcoyle inférieur, $A_1$ représente un amino et $A_2$ un radical de formule

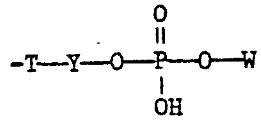
(II)

où T représente NH, W représente un groupe alcoyle ou alcényle en $C_{10}$ à $C_{25}$, qui est non substitué ou substitué par un hydroxy, un alcoxy inférieur, un alcanoyloxy inférieur, un amino, un alcoyle inférieur-amino ou un alcanoylamino ayant jusqu'à 25 atomes de carbone, ou un radical cholestéryle et Y représente un éthylène ou un radical de formule

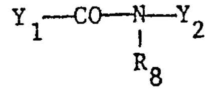
(IIIc)

où $R_8$ représente un hydrogène et $Y_1$ et $Y_2$ représentent chacun indépendamment l'un de l'autre un alcoylène inférieur, et/ou leurs sels.

5. Procédé selon la revendication 1, caractérisé en ce qu'on prépare des composés selon les revendications 3 et 4, où les significations de $A_1$ et $A_2$ sont échangées, et/ou leurs sels.

6. Procédé selon la revendication 1, caractérisé en ce qu'on prépare des composés de formule I selon la revendication 1 où X représente un carbonyle, $R_1$ un alcoyle ou un aryle éventuellement substitué, $R_2$, $R_3$, $R_4$ et $R_6$ représentent un hydrogène ou un alcoyle inférieur, $R_5$ représente un hydrogène, un alcoyle inférieur éventuellement substitué par un hydroxy, un alcoxy inférieur, un mercapto ou un alcoyle inférieur-mercapto ou un halogène, un cycloalcoyle ou un cycloalcoyl-alcoyle inférieur, où le radical cycloalcoyle contient de 4 à 6 atomes de carbone, un phényle ou un phényl-alcoyle inférieur éventuellement substitué, un hétérocyclyle ou un hétérocyclyl-alcoyle inférieur contenant un ou deux atomes d'aza, ou bien où $R_4$ et $R_5$ représentent également ensemble un alcoylène en $C_3$ à $C_4$, $R_7$ représente un hydrogène et l'un des radicaux $A_1$ et $A_2$ représente un radical de formule

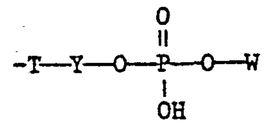

(II)

où T représente NH ou O, où Y représente un alcoylène éventuellement substitué, qui peut également être interrompu par un carbonyloxy ou un carbonylimino et W représente un groupe alcoyle éventuellement substitué par un hydroxy, un alcanoyloxy inférieur, un amino, un alcanoyl-amino ou un oxo ayant plus de 6 atomes de carbone, et l'autre des radicaux $A_1$ et $A_2$ est un hydroxy, un alcoxy inférieur, un amino, un alcoyle inférieur-amino ou un aminocarbonylalcoyle inférieur-amino éventuellement substitué dans le radical alcoyle inférieur par un hydroxy, un carboxy et/ou un amino, et leurs sels.

7. Procédé selon la revendication 1, caractérisé en ce qu'on prépare des composés de formule I selon la revendication 1, où X représente un carbonyle, $R_1$ représente un alcoyle inférieur éventuellement substitué par un hydroxy, un alcoxy inférieur ou un halogène ou un phényle éventuellement substitué par un hydroxy, un alcoxy inférieur, un alcoyle inférieur ou un halogène, $R_2$, $R_4$ et $R_6$ représentent un hydrogène, $R_3$ représente un hydrogène ou un alcoyle inférieur, $R_5$ représente un hydrogène, un alcoyle inférieur éventuellement substitué par un hydroxy, un alcoxy inférieur, un mercapto, un alcoyle inférieur-mercapto ou un halogène, un cycloalcoyle ou un cycloalcoyl-alcoyle inférieur, où le radical cycloalcoyle contient de 4 à 6 atomes de carbone et le radical alcoyle inférieur de 1 à 3 atomes de carbone, un phényle ou un phényl-alcoyle inférieur en $C_1$ à $C_3$ dans la fraction alcoyle inférieur éventuellement substitué par un hydroxy, un alcoxy inférieur ou un halogène, un hétérocyclyle ou hétérocyclyl-alcoyle inférieur contenant un ou deux atomes aza et ayant 5 à 6 chaînons et de 1 à 3 atomes de carbone dans le radical alcoyle inférieur où $R_4$ et $R_5$ représentent également ensemble un alcoylène en $C_3$ à $C_4$, $R_7$ représente un hydrogène, et l'un des radicaux $A_1$ et $A_2$ représente un radical de formule

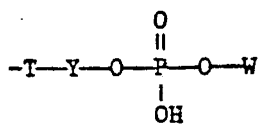

(II)

où T représente HN ou O, Y représente un alcoylène éventuellement substitué ou un radical d'une des formules

(IIIa)

$$Y_1—COO—Y_2$$

(IIIb)

$$Y_1—OOC—Y_2$$

(IIIc)

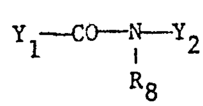

ou

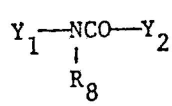

(IIId)

où $Y_1$ et $Y_2$ représentent chacun un alcoylène inférieur éventuellement substitué et $R_8$ représente un hydrogène, W représente un groupe alcoyle en $C_{10}$ à $C_{25}$, qui porte en position 2 un groupe hydroxy, un groupe alcanoyloxy, un amino ou un groupe alcanoylamino, et l'autre des radicaux $A_1$ et $A_2$ représente

un hydroxy, un alcoxy inférieur, un amino, un alcoyle inférieur-amino ou un aminocarbonylalcoyle inférieur-amino éventuellement substitué dans le radical alcoyle inférieur par un hydroxy, un carboxy ou des groupes amino, et/ou leurs sels.

8. Procédé selon l'une des revendications 1, 6 et 7, caractérisé en ce qu'on prépare des composés de formule I où Y représente un alcoylène en $C_1$ à $C_4$ éventuellement substitué par un alcoyle en $C_1$ à $C_4$, qui peut être interrompu par un carbonyloxy ou un carbonylimino, et/ou leurs sels.

9. Procédé selon la revendication 1, caractérisé en ce qu'on prépare des composés de formule I selon la revendication 1, où X représente un carbonyle, $R_1$ représente un alcoyle inférieur en $C_1$ à $C_3$ ou un phényle, $R_2$, $R_4$ et $R_6$ représentent un hydrogène, $R_3$ représente un hydrogène ou un alcoyle inférieur en $C_1$ à $C_3$, $R_5$ représente un hydrogène, un alcoyle inférieur en $C_1$ à $C_3$ éventuellement substitué par un hydroxy, un méthoxy, un mercapto, un méthylmercapto ou un halogène, un phényle ou un phénylméthyle éventuellement substitué par un hydroxy, un méthoxy ou un halogène, un hétérocyclyle ou un hétérocyclylméthyle contenant un ou deux atomes d'aza et ayant 5 chaînons, où $R_4$ et $R_5$ représentent également ensemble un triméthylène, $R_7$ représente un hydrogène et l'un des radicaux $A_1$ et $A_2$ représente un radical de formule

$$-T-Y-O-\overset{\overset{\displaystyle O}{\|}}{\underset{\underset{\displaystyle OH}{|}}{P}}-O-W \qquad (II)$$

où T représente HN ou O, Y représente un alcoylène inférieur en $C_2$ à $C_3$ ou un radical de formules (IIIa) ou (IIIc)

$$Y_1 - COO - Y_2 \qquad (IIIa)$$

$$Y_1 - CO - \underset{\underset{\displaystyle R_8}{|}}{N} - Y_2 \qquad (IIIc)$$

où $R_8$ représente un hydrogène et $Y_1$ et $Y_2$ représentent chacun indépendamment l'un de l'autre un alcoylène inférieur en $C_1$ à $C_3$ éventuellement substitué par un hydroxy, un alcoxy inférieur, un mercapto ou un alcoyle inférieur-mercapto, ou un alcoylène inférieur en $C_1$ à $C_3$ qui est substitué par un phényle ou un phénylalcoyle inférieur éventuellement substitué par un hydroxy, un méthoxy ou un halogène ou un hétérocyclyle ou un hétérocyclyl-alcoyle inférieur contenant un ou deux atomes d'aza et ayant 5 à 6 chaînons et de 1 à 3 atomes de carbone dans le radical alcoyle inférieur, W représente un groupe alcoyle en $C_{10}$ à $C_{25}$, qui est substitué en position 2 par un hydroxy, un alcanoyloxy inférieur, un amino ou un alcanoylamino, et l'autre des radicaux $A_1$ et $A_2$ est un hydroxy, un alcoxy inférieur, un amino, un alcoyle inférieur-amino ou un aminocarbonylalcoyle inférieur-amino, et/ou leurs sels.

10. Procédé selon l'une des revendications 1-9, caractérisé en ce qu'on choisit les produits de départ de manière à obtenir un composé de formule I où l'on obtient à chaque fois en cas de substitution asymétrique sur C-$R_3$ la configuration D, sur C-$R_5$ la configuration L et sur C-N-$R_6$ la configuration D, et/ou un sel de ce composé.

11. Procédé selon la revendication 1, caractérisé en ce qu'on prépare le N-acétyl-desméthylmuramyl-L-alanyl-D-isoglutaminyl-L-alanine-2-(hexadécyloxy-hydroxyphosphoryloxy)-éthylamide et/ou ses sels.

12. Procédé selon la revendication 1, caractérisé en ce qu'on prépare le N-acétyl-muramyl-L-alanyl-D-isoglutaminyl-L-alanine-2-(hexadécyloxy-hydroxyphosphoryloxy)-éthylamide et/ou ses sels.

13. Procédé selon la revendication 1, caractérisé en ce qu'on prépare le N-acétyl-muramyl-L-alanyl-D-isoglutaminyl-L-alanine-2-(tétradécyloxy-hydroxyphosphoryloxy)-éthylamide et/ou ses sels.

14. Procédé selon la revendication 1, caractérisé en ce qu'on prépare le N-acétyl-muramyl-L-alanyl-D-isoglutaminyl-L-alanine-2-[(3'R)-hydroxy-(2'S)-palmitoylamino-4't-octadécényloxy-hydroxyphosphoryloxy]-éthylamide et/ou ses sels.

15. Procédé selon la revendication 1, caractérisé en ce qu'on prépare le N-acétyl-muramyl-L-α-aminobutyryl-D-isoglutaminyl-L-alanine-2-[(3'R)-hydroxy-(2'S)-palmitoylamino-4't-octadécényloxy-hydroxyphosphoryloxy]-éthylamide et/ou ses sels.

16. Procédé selon la revendication 1, caractérisé en ce qu'on prépare le N-acétyl-muramyl-L-α-aminobutyryl-D-isoglutaminyl-L-alanine-2-[(3'R)-hydroxy-(2'S)-palmitoylamino-octadécyloxy-hydroxyphosphoryloxy]-éthylamide et/ou ses sels.

17. Procédé selon la revendication 1, caractérisé en ce qu'on prépare le N-acétyl-muramyl-L-alanyl-D-isoglutaminyl-L-alanine-2-(cholest-5-èn-3β-oxy-hydroxyphosphoryloxy)-éthylamide et/ou ses sels.

18. Procédé selon la revendication 1, caractérisé en ce qu'on prépare le N-benzoyl-muramyl-L-alanyl-D-isoglutaminyl-L-alanine-2-(hexadécyloxy-hydroxyphosphoryloxy)-éthylamide et/ou ses sels.

19. Procédé selon la revendication 1, caractérisé en ce qu'on prépare un composé choisi dans le

groupe N-acétyl-muramyl-L-alanyl-D-isoglutamine-2-(hexadécyloxy-hydroxyphosphoryloxy)-éthylamide, N-acétyl-desméthylmuramyl-L-alanyl-D-isoglutamine-2-(hexadécyloxy-hydroxyphosphoryloxy)-éthylamide, 2-(hexadécyloxy-hydroxyphosphoryloxy)-éthylamide de l'acide N-acétyl-muramyl-L-alanyl-D-isoglutaminyl-oxyméthylcarboxylique, N-acétyl-muramyl-L-alanyl-D-isoglutamine-2-[(3'R)-hydroxy-(2'S)-palmitoylamino-4't-octadécényloxy-hydroxyphosphoryloxy]-éthylamide, N-acétyl-desméthylmuramyl-L-alanyl-D-isoglutamine-2-[(3'R)-hydroxy-(2'S)-palmitoylamino-4't-octadecényloxy-hydroxy phosphoryloxy]-éthylamide, N-acétyl-desméthylmuramyl-L-alanyl-D-isoglutaminyl-L-alanine-2-[(3'R)-hydroxy-(2'S)-palmitoylamino-4't-octadécényloxy-hydroxyphosphoryloxy]-éthylamide, [(3'R)-hydroxy-(2'S)-palmitoylamino-4't-octadécényloxy-hydroxyphosphoryloxy]-éthylamide de l'acide N-acétyl-muramyl-L-alanyl-D-isoglutaminyl-oxyméthylcarboxylique ou un sel de ces composés.

20. Procédé selon la revendication 1, caractérisé en ce qu'on prépare des composés de formule I à action immunopotentialisante selon l'une des revendications 1 à 16 et/ou leurs sels.

21. Procédé selon l'une des revendications 1 à 20, caractérisé en ce que pour séparer les groupes protecteurs on traite avec un acide ou avec de l'hydrogène en présence d'un catalyseur.

22. Procédé selon l'une des revendications 1 à 20, caractérisé en ce qu'on utilise comme composés ayant un ou plusieurs groupes hydroxy estérifiés réactifs des composés où le(s) groupe(s) hydroxy est(sont) estérifié(s) par un acide inorganique fort ou un acide sulfonique.

23. Procédé selon l'une des revendications 1 à 20, caractérisé en ce qu'on utilise comme composés ayant un ou plusieurs groupes hydroxy estérifiés réactifs des composés où le(s) groupe(s) hydroxy est(sont) estérifié(s) avec un acide halohydrique.

24. Procédé selon l'une des revendications 1 à 20, caractérisé en ce qu'on utilise comme composés ayant un ou plusieurs groupes protecteurs d'hydroxy des composés où le(s) groupe(s) hydroxy est(sont) protégé(s) avec des radicaux acyle, aroyle, ou des radicaux provenant de dérivés de l'acide carbonique ou avec des radicaux alcoyle ramifiés en position α ou avec des radicaux α-mono-, -di- ou -tri-arylalcoyle inférieur ou avec des radicaux formateurs d'acétals.

25. Procédé selon la revendication 24, caractérisé en ce qu'on utilise comme composés ayant un ou plusieurs groupes protecteurs d'hydroxy des composés où le(s) groupe(s) hydroxy est(sont) protégé(s) avec des radicaux alcanoyle inférieur, benzoyle, benzyloxycarbonyle, alcoxy inférieur-carbonyle, tert.butyle ou avec des radicaux benzyle, triphénylméthyle ou tétrahydropyranyle éventuellement substitués.

26. Procédé selon l'une des revendications 1 à 20, caractérisé en ce qu'on utilise comme composés ayant un ou plusieurs groupes protecteurs de carboxy des composés où le(s) groupe(s) carboxy est(sont) protégé(s) par des radicaux tert.butyle, benzyle ou par des radicaux triphénylméthyle ou benzhydryle éventuellement substitués par des halogènes ou des alcoxy inférieurs.

27. Procédé selon l'une des revendications 1 à 20, caractérisé en ce qu'on utilise comme composés ayant un ou plusieurs groupes acide carboxylique activés des composés où le(s) groupe(s) acide carboxylique se présente(nt) sous forme d'anhydride, d'azide, d'amide activé ou d'ester activé.

28. Procédé selon la revendication 27, caractérisé en ce qu'on utilise comme anhydrides d'acide de tels corps avec des alcoyle inférieur-esters de l'acide carbonique, comme amides d'acide des imidazolides ou des isooxazolides et comme esters activés le cyano- ou le carboxyméthylester, l'acétylaminoéthylthioester, le p-nitro- ou 2,4,5-trichlorophénylester, le N-hydroxy-succinimide-, le N-hydroxy-phtalimide- ou le N-hydroxy-pipéridine-ester, le 8-hydroxy-quinoléinester, le méthoxyéthylthioester ou les esters obtenus par réaction avec le carbodiimide avec addition de N-hydroxy-succinimide ou d'un 1-hydroxybenzotriazole ou d'une 3-hydroxy-4-oxo-3,4-dihydro-benzo-[d]-1,2,3-triazine.

29. Procédé de préparation d'une préparation pharmaceutique, caractérisé en ce qu'on mélange un composé obtenu selon l'une des revendications 1 à 8 avec un antibiotique et un support pharmaceutiquement acceptable.